# EUROPEAN PATENT SPECIFICATION

(11) **EP 3 234 602 B1**
(45) Date of publication and mention of the grant of the patent: **25.08.2021**
(21) Application number: 15871277.8
(22) Date of filing: 21.12.2015
(51) Int. Cl.: C12N 5/07, C07H 21/04, C12Q 1/68

(54) **METHODS FOR IDENTIFYING MULTIPLE EPITOPES IN SELECTED SUB-POPULATIONS OF CELLS**
VERFAHREN ZUR IDENTIFIZIERUNG MEHRERER EPITOPE IN AUSGEWÄHLTEN SUBPOPULATIONEN VON ZELLEN
PROCÉDÉS D'IDENTIFICATION DE MULTIPLES ÉPITOPES DANS DES SOUS-POPULATIONS SÉLECTIONNÉES DE CELLULES

(30) Priority: 19.12.2014 US 201462094924 P; 19.12.2014 US 201462094919 P; 19.12.2014 US 201462094917 P
(43) Date of publication of application: 25.10.2017
(73) Proprietor: F. Hoffmann-La Roche AG, 4070 Basel (CH); Roche Diagnostics GmbH, 68305 Mannheim (DE)
(72) Inventor: NOLAN, Garry, P., Redwood City, CA 94062 (US)
(74) Representative: Avidity IP
(86) International application number: PCT/US2015/067147
(87) International publication number: WO 2016/100976

(56) References cited:
- WO-A2-2012/106385
- WO-A2-2014/026032
- US-A1- 2001 041 344
- US-A1- 2010 203 543
- US-A1- 2010 240 101
- US-A1- 2011 263 688
- H. C. FAN ET AL: "Combinatorial labeling of single cells for gene expression cytometry", SCIENCE, vol. 347, no. 6222, 5 February 2015 (2015-02-05), pages 1258367-1258367, XP055236295, ISSN: 0036-8075, DOI: 10.1126/science.1258367
- LAURING ET AL.: 'Exploring the Fitness Landscape of an RNA Virus by Using a Universal Barcode Microarray.' J VIROL. vol. 85, no. 8, 2011, pages 3780 - 91, XP055311579
- MACOSKO ET AL.: 'Highly Parallel Genome-wide Expression Profiling of Individual Cells Using Nanoliter Droplets.' CELL vol. 161, no. 5, 21 May 2015, pages 1202 - 14, XP029129143
- None

### SEQUENCE LISTING

<110> F. Hoffmann-La Roche AG et al
<120> METHODS FOR IDENTIFYING MULTIPLE EPITOPES IN SELECTED SUB-POPULATIONS OF CELLS
<130> P104015EP51
<140> 15 871277.8
   <141> 2015-12-21
<150> PCT/US2015/067147
   <151> 2015-12-21
<150> 62/094,917
   <151> 2014-12-19
<150> 62/094,919
   <151> 2014-12-19
<150> 62/094,924
   <151> 2014-12-19
<160> 135
<170> PatentIn version 3.5
<210> 1
   <211> 58
   <212> DNA
   <213> Artificial Sequence
<220>
   <223> Description of Artificial Sequence: Synthetic oligonucleotide
<220>
   <221> modified_base
   <222> (35)..(43)
   <223> a, c, t, g, unknown or other
<400> 1
   gtgactggag ttcagacgtg tgctcttccg atctnnnnnn nnncgtcaga cagggagc 58
<210> 2
   <211> 30
   <212> DNA
   <213> Artificial Sequence
<220>
   <223> Description of Artificial Sequence: Synthetic oligonucleotide
<220>
   <221> misc_feature
   <222> (1)..(30)
   <223> This sequence may encompass 4-30 nucleotides
<400> 2
   cccccccccc cccccccccc cccccccccc 30
<210> 3
   <211> 20
   <212> DNA
   <213> Artificial Sequence
<220>
   <223> Description of Artificial Sequence: Synthetic oligonucleotide
<220>
   <221> misc_feature
   <222> (1)..(20)
   <223> This sequence may encompass 6-20 nucleotides
<400> 3
   cccccccccc cccccccccc 20
<210> 4
   <211> 12
   <212> DNA
   <213> Artificial Sequence
<220>
   <223> Description of Artificial Sequence: Synthetic oligonucleotide
<220>
   <221> misc_feature
   <222> (1)..(12)
   <223> This sequence may encompass 6-12 nucleotides
<400> 4
   cccccccccc cc 12
<210> 5
   <211> 30
   <212> DNA
   <213> Artificial Sequence
<220>
   <223> Description of Artificial Sequence: Synthetic oligonucleotide
<220>
   <221> misc_feature
   <222> (1)..(30)
   <223> This sequence may encompass 0-30 nucleotides
<400> 5
   cccccccccc cccccccccc cccccccccc 30
<210> 6
   <211> 26
   <212> DNA
   <213> Artificial Sequence
<220>
   <223> Description of Artificial Sequence: Synthetic primer
<220>
   <221> modified_base
   <222> (16)..(26)
   <223> a, c, t, g, unknown or other
<400> 6
   gctccctgtc tgacgnnnnn nnnnnn 26
<210> 7
   <211> 21
   <212> DNA
   <213> Artificial Sequence
<220>
   <223> Description of Artificial Sequence: Synthetic primer
<220>
   <221> modified_base
   <222> (16)..(21)
   <223> a, c, t, g, unknown or other
<400> 7
   gctccctgtc tgacgnnnnn n 21
<210> 8
   <211> 157
   <212> DNA
   <213> Artificial Sequence
<220>
   <223> Description of Artificial Sequence: Synthetic polynucleotide
<220>
   <221> modified_base
   <222> (25)..(41)
   <223> a, c, t, g, unknown or other
<220>
   <221> modified_base
   <222> (68)..(74)
   <223> a, c, t, g, unknown or other
<220>
   <221> modified_base
   <222> (93)..(99)
   <223> a, c, t, g, unknown or other
<220>
   <221> modified_base
   <222> (118)..(124)
   <223> a, c, t, g, unknown or other
<220>
   <221> modified_base
   <222> (149)..(157)
   <223> a, c, t, g, unknown or other
<400> 8
   ccctacacga cgctcttccg atctnnnnnn nnnnnnnnnn ngctcaccaa gtcctagtgc 60
   gttacccnnn nnnncccatt ctcgtctttg ccnnnnnnnc cgttgtgggc gacttccnnn 120
   nnnnccaacc accgctccct gtctgacgnn nnnnnnn 157
<210> 9
   <211> 26
   <212> DNA
   <213> Artificial Sequence
<220>
   <223> Description of Artificial Sequence: Synthetic oligonucleotide
<400> 9
   gggtaacgca ctaggacttg gtgagc 26
<210> 10
   <211> 18
   <212> DNA
   <213> Artificial Sequence
<220>
   <223> Description of Artificial Sequence: Synthetic oligonucleotide
<400> 10
   ggcaaagacg agaatggg 18
<210> 11
   <211> 18
   <212> DNA
   <213> Artificial Sequence
<220>
   <223> Description of Artificial Sequence: Synthetic oligonucleotide
<400> 11
   ggaagtcgcc cacaacgg 18
<210> 12
   <211> 24
   <212> DNA
   <213> Artificial Sequence
<220>
   <223> Description of Artificial Sequence: Synthetic oligonucleotide
<400> 12
   cgtcagacag ggagcggtgg ttgg 24
<210> 13
   <211> 133
   <212> DNA
   <213> Artificial Sequence
<220>
   <223> Description of Artificial Sequence: Synthetic polynucleotide
<220>
   <221> modified_base
   <222> (25)..(41)
   <223> a, c, t, g, unknown or other
<220>
   <221> modified_base
   <222> (68)..(74)
   <223> a, c, t, g, unknown or other
<220>
   <221> modified_base
   <222> (93)..(99)
   <223> a, c, t, g, unknown or other
<220>
   <221> modified_base
   <222> (118)..(124)
   <223> a, c, t, g, unknown or other
<400> 13
   ccctacacga cgctcttccg atctnnnnnn nnnnnnnnnn ngctcaccaa gtcctagtgc 60
   gttacccnnn nnnncccatt ctcgtctttg ccnnnnnnnc cgttgtgggc gacttccnnn 120
   nnnnccaacc acc 133
<210> 14
   <211> 26
   <212> DNA
   <213> Artificial Sequence
<220>
   <223> Description of Artificial Sequence: Synthetic oligonucleotide
<400> 14
   gggtaacgca ctaggacttg gtgagc 26
<210> 15
   <211> 18
   <212> DNA
   <213> Artificial Sequence
<220>
   <223> Description of Artificial Sequence: Synthetic oligonucleotide
<400> 15
   ggcaaagacg agaatggg 18
<210> 16
   <211> 18
   <212> DNA
   <213> Artificial Sequence
<220>
   <223> Description of Artificial Sequence: Synthetic oligonucleotide
<400> 16
   ggaagtcgcc cacaacgg 18
<210> 17
   <211> 24
   <212> DNA
   <213> Artificial Sequence
<220>
   <223> Description of Artificial Sequence: Synthetic oligonucleotide
<400> 17
   cgtcagacag ggagcggtgg ttgg 24
<210> 18
   <211> 34
   <212> DNA
   <213> Artificial Sequence
<220>
   <223> Description of Artificial Sequence: Synthetic oligonucleotide
<400> 18
   tctagccttc tcgtgtgcag acttgaggtc agtg 34
<210> 19
   <211> 133
   <212> DNA
   <213> Artificial Sequence
<220>
   <223> Description of Artificial Sequence: Synthetic polynucleotide
<220>
   <221> modified_base
   <222> (25)..(41)
   <223> a, c, t, g, unknown or other
<220>
   <221> modified_base
   <222> (68)..(74)
   <223> a, c, t, g, unknown or other
<220>
   <221> modified_base
   <222> (93)..(99)
   <223> a, c, t, g, unknown or other
<220>
   <221> modified_base
   <222> (118)..(124)
   <223> a, c, t, g, unknown or other
<400> 19
   ccctacacga cgctcttccg atctnnnnnn nnnnnnnnnn ngctcaccaa gtcctagtgc    60
   gttacccnnn nnnncccatt ctcgtctttg ccnnnnnnnc cgttgtgggc gacttccnnn   120
   nnnnccaacc acc                                                       133
<210> 20
   <211> 26
   <212> DNA
   <213> Artificial Sequence
<220>
   <223> Description of Artificial Sequence: Synthetic oligonucleotide
<400> 20
   gggtaacgca ctaggacttg gtgagc 26
<210> 21
   <211> 18
   <212> DNA
   <213> Artificial Sequence
<220>
   <223> Description of Artificial Sequence: Synthetic oligonucleotide
<400> 21
   ggcaaagacg agaatggg 18
<210> 22
   <211> 18
   <212> DNA
   <213> Artificial Sequence
<220>
   <223> Description of Artificial Sequence: Synthetic oligonucleotide
<400> 22
   ggaagtcgcc cacaacgg 18
<210> 23
   <211> 24
   <212> DNA
   <213> Artificial Sequence
<220>
   <223> Description of Artificial Sequence: Synthetic oligonucleotide
<400> 23
   cgtcagacag ggagcggtgg ttgg 24
<210> 24
   <211> 34
   <212> DNA
   <213> Artificial Sequence
<220>
   <223> Description of Artificial Sequence: Synthetic oligonucleotide
<400> 24
   gtgactggag ttcagacgtg tgctcttccg atct 34
<210> 25
   <211> 52
   <212> DNA
   <213> Artificial Sequence
<220>
   <223> Description of Artificial Sequence: Synthetic primer
<400> 25
   gtgactggag ttcagacgtg tgctcttccg atcttcatct tgggagaggt ag 52
<210> 26
   <211> 48
   <212> DNA
   <213> Artificial Sequence
<220>
   <223> Description of Artificial Sequence: Synthetic oligonucleotide
<400> 26
   acaatacttt gacctgtgag gtgatgggac ctacctctcc caagatga 48
<210> 27
   <211> 82
   <212> DNA
   <213> Artificial Sequence
<220>
   <223> Description of Artificial Sequence: Synthetic oligonucleotide
<400> 27
   gtgactggag ttcagacgtg tgctcttccg atcttcatct tgggagaggt aggtcccatc 60
   acctcacagg tcaaagtatt gt 82
<210> 28
   <211> 35
   <212> DNA
   <213> Artificial Sequence
<220>
   <223> Description of Artificial Sequence: Synthetic oligonucleotide
<400> 28
   gctccctgtc tgacgacaat actttgacct gtgag 35
<210> 29
   <211> 82
   <212> DNA
   <213> Artificial Sequence
<220>
   <223> Description of Artificial Sequence: Synthetic oligonucleotide
<400> 29
   gtgactggag ttcagacgtg tgctcttccg atcttcatct tgggagaggt aggtcccatc 60
   acctcacagg tcaaagtatt gt 82
<210> 30
   <211> 35
   <212> DNA
   <213> Artificial Sequence
<220>
   <223> Description of Artificial Sequence: Synthetic oligonucleotide
<400> 30
   gctccctgtc tgacgacaat actttgacct gtgag 35
<211> 158
   <212> DNA
   <213> Artificial Sequence
<220>
   <223> Description of Artificial Sequence: Synthetic polynucleotide
<400> 31
<210> 32
   <211> 33
   <212> DNA
   <213> Artificial Sequence
<220>
   <223> Description of Artificial Sequence: Synthetic oligonucleotide
<400> 32
   acactctttc cctacacgac gctcttccga tct 33
<210> 33
   <211> 58
   <212> DNA
   <213> Artificial Sequence
<220>
   <223> Description of Artificial Sequence: Synthetic oligonucleotide
<400> 33
   aatgatacgg cgaccaccga gatctacact ctttccctac acgacgctct tccgatct 58
<210> 34
   <211> 129
   <212> DNA
   <213> Artificial Sequence
<220>
   <223> Description of Artificial Sequence: Synthetic polynucleotide
<220>
   <221> modified_base
   <222> (25)..(29)
   <223> a, c, t, g, unknown or other
<400> 34
   ccctacacga cgctcttccg atctnnnnna aacgctcacc aagtcctagt gcgttaccca 60
   aaccccattc tcgcgacttc caaacccaac caccgctccc tgtctgacga caatactttg 120
   acctgtgag 129
<210> 35
   <211> 158
   <212> DNA
   <213> Artificial Sequence
<220>
   <223> Description of Artificial Sequence: Synthetic polynucleotide
<400> 35
   gtgactggag ttcagacgtg tgctcttccg atcttcatct tgggagaggt aggtcccatc 60
   acctcacagg tcaaagtatt gtcgtcagac agggagcggt ggttggaaaa ggaagtcgcg 120
   agaatgggaa aagggtaacg cactaggact tggtgagc 158
<210> 36
   <211> 18
   <212> DNA
   <213> Artificial Sequence
<220>
   <223> Description of Artificial Sequence: Synthetic oligonucleotide
<220>
   <221> modified_base
   <222> (1)..(8)
   <223> a, c, t, g, unknown or other
<400> 36
   nnnnnnnntg caatacgg 18
<210> 37
   <211> 145
   <212> DNA
   <213> Artificial Sequence
<220>
   <223> Description of Artificial Sequence: Synthetic polynucleotide
<220>
   <221> modified_base
   <222> (11)..(18)
   <223> a, c, t, g, unknown or other
<220>
   <221> modified_base
   <222> (128)..(135)
   <223> a, c, t, g, unknown or other
<400> 37
   ccgtattgca nnnnnnnncc ctatcccttt tccgttatgc cgagttgcct tttcgctttc 60
   tggcgttcag gcgcaatgca ggcgcgttac ccttttccca ttctggcgac ttccttttcc 120
   aaccaccnnn nnnnncggtt agtgg 145
<210> 38
   <211> 21
   <212> DNA
   <213> Artificial Sequence
<220>
   <223> Description of Artificial Sequence: Synthetic oligonucleotide
<400> 38
   gcctgcattg cgcctgaacg c 21
<210> 39
   <211> 18
   <212> DNA
   <213> Artificial Sequence
<220>
   <223> Description of Artificial Sequence: Synthetic oligonucleotide
<220>
   <221> modified_base
   <222> (11)..(18)
   <223> a, c, t, g, unknown or other
<400> 39
   ccactaaccg nnnnnnnn 18
<210> 40
   <211> 34
   <212> DNA
   <213> Artificial Sequence
<220>
   <223> Description of Artificial Sequence: Synthetic oligonucleotide
<400> 40
   gtgactggag ttcagacgtg tgctcttccg atct 34
<210> 41
   <211> 15
   <212> DNA
   <213> Artificial Sequence
<220>
   <223> Description of Artificial Sequence: Synthetic oligonucleotide
<400> 41
   aaaaaaaaaa aaaaa 15
<210> 42
   <211> 72
   <212> DNA
   <213> Artificial Sequence
<220>
   <223> Description of Artificial Sequence: Synthetic oligonucleotide
<220>
   <221> modified_base
   <222> (1)..(8)
   <223> a, c, t, g, unknown or other
<400> 42
   nnnnnnnncc gtattgcacc ctatcccttt tccgttatgc cgagttgcct tttcgctttc 60
   tggcgttcag gc 72
<210> 43
   <211> 73
   <212> DNA
   <213> Artificial Sequence
<220>
   <223> Description of Artificial Sequence: Synthetic oligonucleotide
<220>
   <221> modified_base
   <222> (66)..(73)
   <223> a, c, t, g, unknown or other
<400> 43
   gcaatgcagg cgcgttaccc ttttcccatt ctggcgactt ccttttccaa ccacccggtt 60
   agtggnnnnn nnn 73
<210> 44
   <211> 28
   <212> DNA
   <213> Artificial Sequence
<220>
   <223> Description of Artificial Sequence: Synthetic oligonucleotide
<220>
   <221> modified_base
   <222> (11)..(17)
   <223> a, c, t, g, unknown or other
<400> 44
   cgcaagtccg nnnnnnncgt tacgtccg 28
<210> 45
   <211> 59
   <212> DNA
   <213> Artificial Sequence
<220>
   <223> Description of Artificial Sequence: Synthetic oligonucleotide
<220>
   <221> modified_base
   <222> (1)..(8)
   <223> a, c, t, g, unknown or other
<220>
   <221> modified_base
   <222> (19)..(59)
   <223> a, c, t, g, unknown or other
<220>
   <221> misc_feature
   <222> (26)..(59)
   <223> This region may encompass 2-34 nucleotides
<400> 45
   nnnnnnnntg caatacggnn nnnnnnnnnn nnnnnnnnnn nnnnnnnnnn nnnnnnnnn 59
<210> 46
   <211> 58
   <212> DNA
   <213> Artificial Sequence
<220>
   <223> Description of Artificial Sequence: Synthetic oligonucleotide
<220>
   <221> modified_base
   <222> (1)..(41)
   <223> a, c, t, g, unknown or other
<220>
   <221> misc_feature
   <222> (1)..(34)
   <223> This region may encompass 1-34 nucleotides
<220>
   <221> modified_base
   <222> (51)..(58)
   <223> a, c, t, g, unknown or other
<400> 46
   nnnnnnnnnn nnnnnnnnnn nnnnnnnnnn nnnnnnnnnn nccataaccg nnnnnnnn 58
<210> 47
   <211> 18
   <212> DNA
   <213> Artificial Sequence
<220>
   <223> Description of Artificial Sequence: Synthetic oligonucleotide
<220>
   <221> modified_base
   <222> (1)..(8)
   <223> a, c, t, g, unknown or other
<400> 47
   nnnnnnnntg caatacgg 18
<210> 48
   <211> 124
   <212> DNA
   <213> Artificial Sequence
<220>
   <223> Description of Artificial Sequence: Synthetic polynucleotide
<220>
   <221> modified_base
   <222> (11)..(18)
   <223> a, c, t, g, unknown or other
<220>
   <221> modified_base
   <222> (107)..(114)
   <223> a, c, t, g, unknown or other
<400> 48
   ccgtattgca nnnnnnnncc ctatcccttt tccgttatgc cgagttgcct tttcgctttc 60
   tggcgttacc cttttcccat tctggcgact tccttttcca accaccnnnn nnnncggtta 120
   gtgg 124
<210> 49
   <211> 18
   <212> DNA
   <213> Artificial Sequence
<220>
   <223> Description of Artificial Sequence: Synthetic oligonucleotide
<220>
   <221> modified_base
   <222> (11)..(18)
   <223> a, c, t, g, unknown or other
<400> 49
   ccactaaccg nnnnnnnn 18
<210> 50
   <211> 124
   <212> DNA
   <213> Artificial Sequence
<220>
   <223> Description of Artificial Sequence: Synthetic polynucleotide
<220>
   <221> modified_base
   <222> (1)..(8)
   <223> a, c, t, g, unknown or other
<220>
   <221> modified_base
   <222> (117)..(124)
   <223> a, c, t, g, unknown or other
<400> 50
   nnnnnnnncc gtattgcacc ctatcccttt tccgttatgc cgagttgcct tttcgctttc 60
   tggcgttacc cttttcccat tctggcgact tccttttcca accacccggt tagtggnnnn 120
   nnnn 124
<210> 51
   <211> 59
   <212> DNA
   <213> Artificial Sequence
<220>
   <223> Description of Artificial Sequence: Synthetic oligonucleotide
<220>
   <221> modified_base
   <222> (1)..(8)
   <223> a, c, t, g, unknown or other
<220>
   <221> modified_base
   <222> (19)..(59)
   <223> a, c, t, g, unknown or other
<220>
   <221> misc_feature
   <222> (26)..(59)
   <223> This region may encompass 2-34 nucleotides
<400> 51
   nnnnnnnntg caatacggnn nnnnnnnnnn nnnnnnnnnn nnnnnnnnnn nnnnnnnnn 59
<210> 52
   <211> 58
   <212> DNA
   <213> Artificial Sequence
<220>
   <223> Description of Artificial Sequence: Synthetic oligonucleotide
<220>
   <221> modified_base
   <222> (1)..(41)
   <223> a, c, t, g, unknown or other
<220>
   <221> misc_feature
   <222> (1)..(34)
   <223> This region may encompass 1-34 nucleotides
<220>
   <221> modified_base
   <222> (51)..(58)
   <223> a, c, t, g, unknown or other
<400> 52
   nnnnnnnnnn nnnnnnnnnn nnnnnnnnnn nnnnnnnnnn nccataaccg nnnnnnnn 58
<210> 53
   <211> 14
   <212> DNA
   <213> Artificial Sequence
<220>
   <223> Description of Artificial Sequence: Synthetic oligonucleotide
<400> 53
   aaaaaaaaaa aaaa 14
<210> 54
   <211> 115
   <212> DNA
   <213> Artificial Sequence
<220>
   <223> Description of Artificial Sequence: Synthetic polynucleotide
<400> 54
   tcaccaagtc ctagtcgagt tgccttttcg ctttctggcg ttaccctttt cccattctgg 60
   cgacttcctt ttccaaccac cagatcggaa gagcaaaaaa aagaactcca gtcac 115
<210> 55
   <211> 113
   <212> DNA
   <213> Artificial Sequence
<220>
   <223> Description of Artificial Sequence: Synthetic polynucleotide
<220>
   <221> modified_base
   <222> (86)..(89)
   <223> a, c, t, g, unknown or other
<400> 55
   ggtggttgga aaaggaagtc gccagaatgg gaaaagggta acgccagaaa gcgaaaaggc 60
   aactcgacta ggacttggtg aaaaannnna gatcggaaga gcgtcgtgta ggg 113
<210> 56
   <211> 34
   <212> DNA
   <213> Artificial Sequence
<220>
   <223> Description of Artificial Sequence: Synthetic primer
<400> 56
   gtgactggag ttcagacgtg tgctcttccg atct 34
<210> 57
   <211> 124
   <212> DNA
   <213> Artificial Sequence
<220>
   <223> Description of Artificial Sequence: Synthetic polynucleotide
<400> 57
   gctcaccaag tcctagtcga gttgcctttt cgctttctgg cgttaccctt ttcccattct 60
   ggcgacttcc ttttccaacc acctccctgt ctgacgtttt agatcggaag agcggttcag 120
   cagg 124
<210> 58
   <211> 161
   <212> DNA
   <213> Artificial Sequence
<220>
   <223> Description of Artificial Sequence: Synthetic polynucleotide
<220>
   <221> modified_base
   <222> (133)..(137)
   <223> a, c, t, g, unknown or other
<400> 58
   cctgctgaac cgctcttccg atctaaaaaa aacgtcagac agggaggtgg ttggaaaagg 60
   aagtcgccag aatgggaaaa gggtaacgcc agaaagcgaa aaggcaactc gactaggact 120
   tggtgagcaa aannnnntct agccttctcg cagcacatcc c 161
<210> 59
   <211> 58
   <212> DNA
   <213> Artificial Sequence
<220>
   <223> Description of Artificial Sequence: Synthetic primer
<400> 59
   aatgatacgg cgaccaccga gatctacact ctttccctac acgacgctct tccgatct 58
<210> 60
   <211> 150
   <212> DNA
   <213> Artificial Sequence
<220>
   <223> Description of Artificial Sequence: Synthetic polynucleotide
<220>
   <221> modified_base
   <222> (25)..(33)
   <223> a, c, t, g, unknown or other
<220>
   <221> modified_base
   <222> (60)..(63)
   <223> a, c, t, g, unknown or other
<220>
   <221> modified_base
   <222> (82)..(85)
   <223> a, c, t, g, unknown or other
<220>
   <221> modified_base
   <222> (110)..(117)
   <223> a, c, t, g, unknown or other
<400> 60
   ccctacacga cgctcttccg atctnnnnnn nnngctcacc aagtcctagt gcgttacccn 60
   nnncccattc tcgcgacttc cnnnnccaac caccgctccc tgtctgacgn nnnnnnnaga 120
   tcggaagagc ggttcagcag gaatgccgag 150
<210> 61
   <211> 76
   <212> DNA
   <213> Artificial Sequence
<220>
   <223> Description of Artificial Sequence: Synthetic oligonucleotide
<400> 61
   cgtcagacag ggagcggtgg ttggaaaagg aagtcgcgag aatgggaaaa gggtaacgca 60
   ctaggacttg gtgagc 76
<210> 62
   <211> 47
   <212> DNA
   <213> Artificial Sequence
<220>
   <223> Description of Artificial Sequence: Synthetic oligonucleotide
<400> 62
   aagacggcat acgagatcgg tttcctgctg aaccgctctt ccgatct 47
<210> 63
   <211> 96
   <212> DNA
   <213> Artificial Sequence
<220>
   <223> Description of Artificial Sequence: Synthetic oligonucleotide
<400> 63
   cctgctgaac cgctcttccg atctggtggt tggggaagtc gcgagaatgg ggggtaacgc 60
   actaggactt ggtgagccaa aaaaaaaaaa aaaaaa 96
<210> 64
   <211> 58
   <212> DNA
   <213> Artificial Sequence
<220>
   <223> Description of Artificial Sequence: Synthetic primer
<400> 64
   aatgatacgg cgaccaccga gatctacact ctttccctac acgacgctct tccgatct 58
<210> 65
   <211> 165
   <212> DNA
   <213> Artificial Sequence
<220>
   <223> Description of Artificial Sequence: Synthetic polynucleotide
<220>
   <221> modified_base
   <222> (25)..(33)
   <223> a, c, t, g, unknown or other
<220>
   <221> modified_base
   <222> (60)..(63)
   <223> a, c, t, g, unknown or other
<220>
   <221> modified_base
   <222> (82)..(85)
   <223> a, c, t, g, unknown or other
<220>
   <221> modified_base
   <222> (110)..(118)
   <223> a, c, t, g, unknown or other
<400> 65
   ccctacacga cgctcttccg atctnnnnnn nnngctcacc aagtcctagt gcgttacccn 60
   nnncccattc tcgcgacttc cnnnnccaac caccgctccc tgtctgacgn nnnnnnnnag 120
   atcggaagag cacacgtctg aactccagtc acgaactcca tcagc 165
<210> 66
   <211> 76
   <212> DNA
   <213> Artificial Sequence
<220>
   <223> Description of Artificial Sequence: Synthetic oligonucleotide
<400> 66
   cgtcagacag ggagcggtgg ttggaaaagg aagtcgcgag aatgggaaaa gggtaacgca 60
   ctaggacttg gtgagc 76
<210> 67
   <211> 24
   <212> DNA
   <213> Artificial Sequence
<220>
   <223> Description of Artificial Sequence: Synthetic oligonucleotide
<400> 67
   ttcagacgtg tgctcttccg atct 24
<210> 68
   <211> 27
   <212> DNA
   <213> Artificial Sequence
<220>
   <223> Description of Artificial Sequence: Synthetic oligonucleotide
<400> 68
   aataaagctg atggagttcg tgactgg 27
<210> 69
   <211> 43
   <212> DNA
   <213> Artificial Sequence
<220>
   <223> Description of Artificial Sequence: Synthetic oligonucleotide
<400> 69
   caagcagaag acggcatacg agatcgtgat gtgactggag ttc 43
<210> 70
   <211> 43
   <212> DNA
   <213> Artificial Sequence
<220>
   <223> Description of Artificial Sequence: Synthetic oligonucleotide
<400> 70
   caagcagaag acggcatacg agatacatcg gtgactggag ttc 43
<210> 71
   <211> 43
   <212> DNA
   <213> Artificial Sequence
<220>
   <223> Description of Artificial Sequence: Synthetic oligonucleotide
<400> 71
   caagcagaag acggcatacg agatgcctaa gtgactggag ttc 43
<210> 72
   <211> 43
   <212> DNA
   <213> Artificial Sequence
<220>
   <223> Description of Artificial Sequence: Synthetic oligonucleotide
<400> 72
   caagcagaag acggcatacg agattggtca gtgactggag ttc 43
<210> 73
   <211> 43
   <212> DNA
   <213> Artificial Sequence
<220>
   <223> Description of Artificial Sequence: Synthetic oligonucleotide
<400> 73
   caagcagaag acggcatacg agatcactgt gtgactggag ttc 43
<210> 74
   <211> 43
   <212> DNA
   <213> Artificial Sequence
<220>
   <223> Description of Artificial Sequence: Synthetic oligonucleotide
<400> 74
   caagcagaag acggcatacg agatattggc gtgactggag ttc 43
<210> 75
   <211> 43
   <212> DNA
   <213> Artificial Sequence
<220>
   <223> Description of Artificial Sequence: Synthetic oligonucleotide
<400> 75
   caagcagaag acggcatacg agatgatctg gtgactggag ttc 43
<210> 76
   <211> 43
   <212> DNA
   <213> Artificial Sequence
<220>
   <223> Description of Artificial Sequence: Synthetic oligonucleotide
<400> 76
   caagcagaag acggcatacg agattcaagt gtgactggag ttc 43
<210> 77
   <211> 43
   <212> DNA
   <213> Artificial Sequence
<220>
   <223> Description of Artificial Sequence: Synthetic oligonucleotide
<400> 77
   caagcagaag acggcatacg agatctgatc gtgactggag ttc 43
<210> 78
   <211> 43
   <212> DNA
   <213> Artificial Sequence
<220>
   <223> Description of Artificial Sequence: Synthetic oligonucleotide
<400> 78
   caagcagaag acggcatacg agataagcta gtgactggag ttc 43
<210> 79
   <211> 43
   <212> DNA
   <213> Artificial Sequence
<220>
   <223> Description of Artificial Sequence: Synthetic oligonucleotide
<400> 79
   caagcagaag acggcatacg agatgtagcc gtgactggag ttc 43
<210> 80
   <211> 43
   <212> DNA
   <213> Artificial Sequence
<220>
   <223> Description of Artificial Sequence: Synthetic oligonucleotide
<400> 80
   caagcagaag acggcatacg agattacaag gtgactggag ttc 43
<210> 81
   <211> 57
   <212> DNA
   <213> Artificial Sequence
<220>
   <223> Description of Artificial Sequence: Synthetic oligonucleotide
<220>
   <221> modified_base
   <222> (35)..(42)
   <223> a, c, t, g, unknown or other
<400> 81
   gtgactggag ttcagacgtg tgctcttccg atctnnnnnn nncgtcagac agggagc 57
<210> 82
   <211> 58
   <212> DNA
   <213> Artificial Sequence
<220>
   <223> Description of Artificial Sequence: Synthetic primer
<400> 82
   aatgatacgg cgaccaccga gatctacact ctttccctac acgacgctct tccgatct 58
<210> 83
   <211> 147
   <212> DNA
   <213> Artificial Sequence
<220>
   <223> Description of Artificial Sequence: Synthetic polynucleotide
<220>
   <221> modified_base
   <222> (25)..(33)
   <223> a, c, t, g, unknown or other
<220>
   <221> modified_base
   <222> (60)..(63)
   <223> a, c, t, g, unknown or other
<220>
   <221> modified_base
   <222> (82)..(85)
   <223> a, c, t, g, unknown or other
<220>
   <221> modified_base
   <222> (136)..(147)
   <223> a, c, t, g, unknown or other
<400> 83
   ccctacacga cgctcttccg atctnnnnnn nnngctcacc aagtcctagt gcgttacccn 60
<210> 84
   <211> 76
   <212> DNA
   <213> Artificial Sequence
<220>
   <223> Description of Artificial Sequence: Synthetic oligonucleotide
<400> 84
   cgtcagacag ggagcggtgg ttggaaaagg aagtcgcgag aatgggaaaa gggtaacgca 60
   ctaggacttg gtgagc 76
<210> 85
   <211> 34
   <212> DNA
   <213> Artificial Sequence
<220>
   <223> Description of Artificial Sequence: Synthetic primer
<400> 85
   gtgactggag ttcagacgtg tgctcttccg atct 34
<210> 86
   <211> 58
   <212> DNA
   <213> Artificial Sequence
<220>
   <223> Description of Artificial Sequence: Synthetic primer
<400> 86
   aatgatacgg cgaccaccga gatctacact ctttccctac acgacgctct tccgatct 58
<210> 87
   <211> 132
   <212> DNA
   <213> Artificial Sequence
<220>
   <223> Description of Artificial Sequence: Synthetic polynucleotide
<220>
   <221> modified_base
   <222> (25)..(33)
   <223> a, c, t, g, unknown or other
<220>
   <221> modified_base
   <222> (60)..(63)
   <223> a, c, t, g, unknown or other
<220>
   <221> modified_base
   <222> (82)..(85)
   <223> a, c, t, g, unknown or other
<220>
   <221> modified_base
   <222> (110)..(132)
   <223> a, c, t, g, unknown or other
<400> 87
   ccctacacga cgctcttccg atctnnnnnn nnngctcacc aagtcctagt gcgttacccn 60
   nnncccattc tcgcgacttc cnnnnccaac caccgctccc tgtctgacgn nnnnnnnnnn 120
   nnnnnnnnnn nn 132
<210> 88
   <211> 76
   <212> DNA
   <213> Artificial Sequence
<220>
   <223> Description of Artificial Sequence: Synthetic oligonucleotide
<400> 88
   cgtcagacag ggagcggtgg ttggaaaagg aagtcgcgag aatgggaaaa gggtaacgca 60
   ctaggacttg gtgagc 76
<210> 89
   <211> 34
   <212> DNA
   <213> Artificial Sequence
<220>
   <223> Description of Artificial Sequence: Synthetic primer
<400> 89
   gtgactggag ttcagacgtg tgctcttccg atct 34
<210> 90
   <211> 150
   <212> DNA
   <213> Artificial Sequence
<220>
   <223> Description of Artificial Sequence: Synthetic polynucleotide
<220>
   <221> modified_base
   <222> (25)..(33)
   <223> a, c, t, g, unknown or other
<220>
   <221> modified_base
   <222> (60)..(63)
   <223> a, c, t, g, unknown or other
<220>
   <221> modified_base
   <222> (82)..(85)
   <223> a, c, t, g, unknown or other
<220>
   <221> modified_base
   <222> (110)..(117)
   <223> a, c, t, g, unknown or other
<400> 90
   ccctacacga cgctcttccg atctnnnnnn nnngctcacc aagtcctagt gcgttacccn 60
   nnncccattc tcgcgacttc cnnnnccaac caccgctccc tgtctgacgn nnnnnnnaga 120
   tcggaagagc ggttcagcag gaatgccgag 150
<210> 91
   <211> 76
   <212> DNA
   <213> Artificial Sequence
<220>
   <223> Description of Artificial Sequence: Synthetic oligonucleotide
<400> 91
   cgtcagacag ggagcggtgg ttggaaaagg aagtcgcgag aatgggaaaa gggtaacgca 60
   ctaggacttg gtgagc 76
<210> 92
   <211> 134
   <212> DNA
   <213> Artificial Sequence
<220>
   <223> Description of Artificial Sequence: Synthetic polynucleotide
<220>
   <221> modified_base
   <222> (117)..(123)
   <223> a, c, t, g, unknown or other
<400> 92
   ccctatccct tttccgttat gccgagttgc cttttcgctt tctggcgtta cccttttccc 60
   attctggcga cttccttttc caaccaccgc gttcaggcgg ccattcggtt ttttttnnnn 120
   nnncgttacg tccg 134
<210> 93
   <211> 30
   <212> DNA
   <213> Artificial Sequence
<220>
   <223> Description of Artificial Sequence: Synthetic oligonucleotide
<400> 93
   ccgaatggcc gcctgaacgc aaaaaaaaaa 30
<210> 94
   <211> 21
   <212> DNA
   <213> Artificial Sequence
<220>
   <223> Description of Artificial Sequence: Synthetic oligonucleotide
<400> 94
   cggacgtaac gaaaaaaaaa a 21
<210> 95
   <211> 64
   <212> DNA
   <213> Artificial Sequence
<220>
   <223> Description of Artificial Sequence: Synthetic oligonucleotide
<400> 95
   aaaaaaaaaa ccctatccct tttccgttat gccgagttgc cttttcgctt tctggcgttc 60
   aggc 64
<210> 96
   <211> 36
   <212> DNA
   <213> Artificial Sequence
<220>
   <223> Description of Artificial Sequence: Synthetic oligonucleotide
<220>
   <221> modified_base
   <222> (12)..(18)
   <223> a, c, t, g, unknown or other
<400> 96
   gcctgcattg cnnnnnnntt ttttttgcct gaacgc 36
<210> 97
   <211> 65
   <212> DNA
   <213> Artificial Sequence
<220>
   <223> Description of Artificial Sequence: Synthetic oligonucleotide
<400> 97
   aaaaaaaaaa gcaatgcagg cgcgttaccc ttttcccatt ctggcgactt ccttttccaa 60
   ccacc 65
<210> 98
   <211> 107
   <212> DNA
   <213> Artificial Sequence
<220>
   <223> Description of Artificial Sequence: Synthetic polynucleotide
<220>
   <221> modified_base
   <222> (1)..(5)
   <223> a, c, t, g, unknown or other
<220>
   <221> modified_base
   <222> (76)..(83)
   <223> a, c, t, g, unknown or other
<400> 98
   nnnnnaaaag tgacttcgct aggaaaagga agcgtagaaa agttcggtag caaaagaacg 60
   gctccctgtc tgacgnnnnn nnnagatcgg aagagcggtt cagcagg 107
<210> 99
   <211> 61
   <212> DNA
   <213> Artificial Sequence
<220>
   <223> Description of Artificial Sequence: Synthetic oligonucleotide
<400> 99
   caagcagaag acggcatacg agatcggtct cggcattcct gctgaaccgc tcttccgatc 60
   t 61
<210> 100
   <211> 50
   <212> DNA
   <213> Artificial Sequence
<220>
   <223> Description of Artificial Sequence: Synthetic oligonucleotide
<220>
   <221> modified_base
   <222> (19)..(26)
   <223> a, c, t, g, unknown or other
<400> 100
   gctcctccct gtctgacgnn nnnnnnagat cggaagagcg gttcagcagg 50
<210> 101
   <211> 45
   <212> DNA
   <213> Artificial Sequence
<220>
   <223> Description of Artificial Sequence: Synthetic oligonucleotide
<400> 101
   cctgctgaac cgctcttccg atctaaaaaa aacgtcagac aggga 45
<210> 102
   <211> 25
   <212> DNA
   <213> Artificial Sequence
<220>
   <223> Description of Artificial Sequence: Synthetic oligonucleotide
<220>
   <221> modified_base
   <222> (11)..(11)
   <223> Any photocleavable nucleotide
<400> 102
   ggagccgttc ngtagcaaaa gaacg 25
<210> 103
   <211> 119
   <212> DNA
   <213> Artificial Sequence
<220>
   <223> Description of Artificial Sequence: Synthetic polynucleotide
<220>
   <221> modified_base
   <222> (55)..(55)
   <223> Any photocleavable nucleotide
<220>
   <221> modified_base
   <222> (88)..(95)
   <223> a, c, t, g, unknown or other
<400> 103
   cctgctgaac gctcttccga tctaaaaaaa acgtcagaca gggaggagcc gttcngtagc 60 aaaagaacgg ctcctccctg tctgacgnnn nnnnnagatc ggaagagcgg ttcagcagg 119
<210> 104
   <211> 64
   <212> DNA
   <213> Artificial Sequence
<220>
   <223> Description of Artificial Sequence: Synthetic oligonucleotide
<220>
   <221> modified_base
   <222> (33)..(40)
   <223> a, c, t, g, unknown or other
<400> 104
   gtagcaaaag aacggctcct ccctgtctga cgnnnnnnnn agatcggaag agcggttcag 60
   cagg 64
<210> 105
   <211> 55
   <212> DNA
   <213> Artificial Sequence
<220>
   <223> Description of Artificial Sequence: Synthetic oligonucleotide
<400> 105
   cctgctgaac cgctcttccg atctaaaaaa aacgtcagac agggaggagc cgttc 55
<210> 106
   <211> 25
   <212> DNA
   <213> Artificial Sequence
<220>
   <223> Description of Artificial Sequence: Synthetic oligonucleotide
<220>
   <221> modified_base
   <222> (11)..(11)
   <223> Any photocleavable nucleotide
<400> 106
   gctaccgaac ncgtagaaaa gttcg 25
<210> 107
   <211> 78
   <212> DNA
   <213> Artificial Sequence
<220>
   <223> Description of Artificial Sequence: Synthetic oligonucleotide
<220>
   <221> modified_base
   <222> (47)..(54)
   <223> a, c, t, g, unknown or other
<400> 107
   cgtagaaaag ttcggtagca aaagaacggc tcctccctgt ctgacgnnnn nnnnagatcg 60
   gaagagcggt tcagcagg 78
<210> 108
   <211> 65
   <212> DNA
   <213> Artificial Sequence
<220>
   <223> Description of Artificial Sequence: Synthetic oligonucleotide
<400> 108
   cctgctgaac cgctcttccg atctaaaaaa aacgtcagac agggaggagc cgttcgctac 60
   cgaac 65
<210> 109
   <211> 25
   <212> DNA
   <213> Artificial Sequence
<220>
   <223> Description of Artificial Sequence: Synthetic oligonucleotide
<220>
   <221> modified_base
   <222> (11)..(11)
   <223> Any photocleavable nucleotide
<400> 109
   ctacgcttcc nctaggaaaa ggaag 25
<210> 110
   <211> 92
   <212> DNA
   <213> Artificial Sequence
<220>
   <223> Description of Artificial Sequence: Synthetic oligonucleotide
<220>
   <221> modified_base
   <222> (61)..(68)
   <223> a, c, t, g, unknown or other
<400> 110
   ctaggaaaag gaagcgtaga aaagttcggt agcaaaagaa cggctcctcc ctgtctgacg 60
   nnnnnnnnag atcggaagag cggttcagca gg 92
<210> 111
   <211> 75
   <212> DNA
   <213> Artificial Sequence
<220>
   <223> Description of Artificial Sequence: Synthetic oligonucleotide
<400> 111
   cctgctgaac cgctcttccg atctaaaaaa aacgtcagac agggaggagc cgttcgctac 60
   cgaacctacg cttcc 75
<210> 112
   <211> 57
   <212> DNA
   <213> Artificial Sequence
<220>
   <223> Description of Artificial Sequence: Synthetic oligonucleotide
<220>
   <221> modified_base
   <222> (15)..(15)
   <223> Any photocleavable nucleotide
<220>
   <221> modified_base
   <222> (40)..(44)
   <223> a, c, t, g, unknown or other
<400> 112
   cctagcgaag tcacncccta cacgacgctc ttccgatctn nnnnaaaagt gacttcg 57
<210> 113
   <211> 134
   <212> DNA
   <213> Artificial Sequence
<220>
   <223> Description of Artificial Sequence: Synthetic polynucleotide
<220>
   <221> modified_base
   <222> (25)..(29)
   <223> a, c, t, g, unknown or other
<220>
   <221> modified_base
   <222> (103)..(110)
   <223> a, c, t, g, unknown or other
<400> 113
   ccctacacga cgctcttccg atctnnnnna aaagtgactt cgctaggaaa aggaagcgta      60
   gaaaagttcg gtagcaaaag aacggctcct ccctgtctga cgnnnnnnnn agatcggaag     120
   agcggttcag cagg                                                       134
<210> 114
   <211> 89
   <212> DNA
   <213> Artificial Sequence
<220>
   <223> Description of Artificial Sequence: Synthetic oligonucleotide
<400> 114
   cctgctgaac cgctcttccg atctaaaaaa aacgtcagac agggaggagc cgttcgctac      60
   cgaacctacg cttcccctag cgaagtcac                                        89
<210> 115
   <211> 110
   <212> DNA
   <213> Artificial Sequence
<220>
   <223> Description of Artificial Sequence: Synthetic polynucleotide
<220>
   <221> modified_base
   <222> (1)..(5)
   <223> a, c, t, g, unknown or other
<220>
   <221> modified_base
   <222> (79)..(86)
   <223> a, c, t, g, unknown or other
<400> 115
   nnnnnaaaag tgacttcgct aggaaaagga agcgtagaaa agttcggtag caaaagaacg 60
   gctcctccct gtctgacgnn nnnnnnagat cggaagagcg gttcagcagg 110
<210> 116
   <211> 50
   <212> DNA
   <213> Artificial Sequence
<220>
   <223> Description of Artificial Sequence: Synthetic oligonucleotide
<220>
   <221> modified_base
   <222> (19) .. (26)
   <223> a, c, t, g, unknown or other
<400> 116
   gctcctccct gtctgacgnn nnnnnnagat cggaagagcg gttcagcagg 50
<210> 117
   <211> 45
   <212> DNA
   <213> Artificial Sequence
<220>
   <223> Description of Artificial Sequence: Synthetic oligonucleotide
<400> 117
   cctgctgaac cgctcttccg atctaaaaaa aacgtcagac aggga 45
<210> 118
   <211> 57
   <212> DNA
   <213> Artificial Sequence
<220>
   <223> Description of Artificial Sequence: Synthetic oligonucleotide
<220>
   <221> modified_base
   <222> (15)..(15)
   <223> Any photocleavable nucleotide
<220>
   <221> modified_base
   <222> (40)..(44)
   <223> a, c, t, g, unknown or other
<400> 118
   cctagcgaag tcacncccta cacgacgctc ttccgatctn nnnnacgggt gacttcg 57
<210> 119
   <211> 25
   <212> DNA
   <213> Artificial Sequence
<220>
   <223> Description of Artificial Sequence: Synthetic oligonucleotide
<220>
   <221> modified_base
   <222> (11)..(11)
   <223> Any photocleavable nucleotide
<400> 119
   ctacgcttcc nctaggacct ggaag 25
<210> 120
   <211> 25
   <212> DNA
   <213> Artificial Sequence
<220>
   <223> Description of Artificial Sequence: Synthetic oligonucleotide
<220>
   <221> modified_base
   <222> (11)..(11)
   <223> Any photocleavable nucleotide
<400> 120
   gctaccgaac ncgtagaagc gttcg 25
<210> 121
   <211> 25
   <212> DNA
   <213> Artificial Sequence
<220>
   <223> Description of Artificial Sequence: Synthetic oligonucleotide
<220>
   <221> modified_base
   <222> (11)..(11)
   <223> Any photocleavable nucleotide
<400> 121
   ggagccgttc ngtagcaacg gaacg 25
<210> 122
   <211> 110
   <212> DNA
   <213> Artificial Sequence
<220>
   <223> Description of Artificial Sequence: Synthetic polynucleotide
<220>
   <221> modified_base
   <222> (1)..(5)
   <223> a, c, t, g, unknown or other
<220>
   <221> modified_base
   <222> (79)..(86)
   <223> a, c, t, g, unknown or other
<400> 122
   nnnnnaaaag tgacttcgct aggaaaagga agcgtagaaa agttcggtag caaaagaacg 60
   gctcctccct gtctgacgnn nnnnnnagat cggaagagcg gttcagcagg 110
<210> 123
   <211> 50
   <212> DNA
   <213> Artificial Sequence
<220>
   <223> Description of Artificial Sequence: Synthetic oligonucleotide
<220>
   <221> modified_base
   <222> (19)..(26)
   <223> a, c, t, g, unknown or other
<400> 123
   gctcctccct gtctgacgnn nnnnnnagat cggaagagcg gttcagcagg 50
<210> 124
   <211> 45
   <212> DNA
   <213> Artificial Sequence
<220>
   <223> Description of Artificial Sequence: Synthetic oligonucleotide
<400> 124
   cctgctgaac cgctcttccg atctaaaaaa aacgtcagac aggga 45
<210> 125
   <211> 57
   <212> DNA
   <213> Artificial Sequence
<220>
   <223> Description of Artificial Sequence: Synthetic oligonucleotide
<220>
   <221> modified_base
   <222> (15)..(15)
   <223> Any photocleavable nucleotide
<220>
   <221> modified_base
   <222> (40)..(44)
   <223> a, c, t, g, unknown or other
<400> 125
   cctagcgaag tcacncccta cacgacgctc ttccgatctn nnnnaaaagt gacttcg 57
<210> 126
   <211> 25
   <212> DNA
   <213> Artificial Sequence
<220>
   <223> Description of Artificial Sequence: Synthetic oligonucleotide
<220>
   <221> modified_base
   <222> (11)..(11)
   <223> Any photocleavable nucleotide
<400> 126
   gctaccgaac ncgtagaaaa gttcg 25
<210> 127
   <211> 25
   <212> DNA
   <213> Artificial Sequence
<220>
   <223> Description of Artificial Sequence: Synthetic oligonucleotide
<220>
   <221> modified_base
   <222> (11)..(11)
   <223> Any photocleavable nucleotide
<400> 127
   gctaccgaac ncgtagaaaa gttcg 25
<210> 128
   <211> 25
   <212> DNA
   <213> Artificial Sequence
<220>
   <223> Description of Artificial Sequence: Synthetic oligonucleotide
<220>
   <221> modified_base
   <222> (11)..(11)
   <223> Any photocleavable nucleotide
<400> 128
   ggagccgttc ngtagcaaaa gaacg 25
<210> 129
   <211> 110
   <212> DNA
   <213> Artificial Sequence
<220>
   <223> Description of Artificial Sequence: Synthetic polynucleotide
<220>
   <221> modified_base
   <222> (1)..(5)
   <223> a, c, t, g, unknown or other
<220>
   <221> modified_base
   <222> (79)..(86)
   <223> a, c, t, g, unknown or other
<400> 129
   nnnnnaaaag tgacttcgct aggaaaagga agcgtagaaa agttcggtag caaaagaacg 60
   gctcctccct gtctgacgnn nnnnnnagat cggaagagcg gttcagcagg 110
<210> 130
   <211> 50
   <212> DNA
   <213> Artificial Sequence
<220>
   <223> Description of Artificial Sequence: Synthetic oligonucleotide
<220>
   <221> modified_base
   <222> (19)..(26)
   <223> a, c, t, g, unknown or other
<400> 130
   gctcctccct gtctgacgnn nnnnnnagat cggaagagcg gttcagcagg 50
<210> 131
   <211> 45
   <212> DNA
   <213> Artificial Sequence
<220>
   <223> Description of Artificial Sequence: Synthetic oligonucleotide
<400> 131
   cctgctgaac cgctcttccg atctaaaaaa aacgtcagac aggga 45
<210> 132
   <211> 57
   <212> DNA
   <213> Artificial Sequence
<220>
   <223> Description of Artificial Sequence: Synthetic oligonucleotide
<220>
   <221> modified_base
   <222> (15)..(15)
   <223> Any photocleavable nucleotide
<220>
   <221> modified_base
   <222> (40)..(44)
   <223> a, c, t, g, unknown or other
<400> 132
   cctagcgaag tcacncccta cacgacgctc ttccgatctn nnnnacgggt gacttcg 57
<210> 133
   <211> 25
   <212> DNA
   <213> Artificial Sequence
<220>
   <223> Description of Artificial Sequence: Synthetic oligonucleotide
<220>
   <221> modified_base
   <222> (11)..(11)
   <223> Any photocleavable nucleotide
<400> 133
   gctaccgaac ncgtagaagc gttcg 25
<210> 134
   <211> 25
   <212> DNA
   <213> Artificial Sequence
<220>
   <223> Description of Artificial Sequence: Synthetic oligonucleotide
<220>
   <221> modified_base
   <222> (11)..(11)
   <223> Any photocleavable nucleotide
<400> 134
   gctaccgaac ncgtagaagc gttcg 25
<210> 135
   <211> 25
   <212> DNA
   <213> Artificial Sequence
<220>
   <223> Description of Artificial Sequence: Synthetic oligonucleotide
<220>
   <221> modified_base
   <222> (11)..(11)
   <223> Any photocleavable nucleotide
<400> 135
   ggagccgttc ngtagcaacg gaacg 25

## Description

### BACKGROUND OF THE INVENTION

Although all cells in the human body contain the same genetic material, the same sets of genes are not active in all of those cells. Alterations in gene expression patterns can have profound effects on biological function. Understanding the dynamics of production and regulation of gene products (proteins) and their interactions will be essential in understanding, for example, the mechanisms underlying genetic and/or environmentally induced health disorders, and may provide the foundation for discovery of new diagnostic and therapeutic targets. Therefore, techniques for monitoring gene expression profiles and detecting specific variants of whole proteins (*e.g.*, splice variants, point mutations, post-translational modifications, and environmentally- or therapeutically-induced modifications) in individual cells, and for quantifying their levels over time, may aid in the development of new diagnostics and therapeutic procedures. Moreover, it is becoming increasingly important to perform these analyses not for just one, but for multiple target molecules simultaneously in single cells.

The methods available to date often require significant amounts of biological sample and/or will not provide cell-specific information. Additionally, performing multiplexed measurements is often particularly demanding due to the challenges inherent in the analysis of complex samples. Thus, there exists a need for methods capable of accurate and sensitive detection, identification, and quantification of target molecules in the individual cells of a complex cell population. Furthermore, it is often desirable to perform these analyses for individual cells of a selected subpopulation of cells within a sample, and to retain cell-specific information regarding the presence of one or more target molecules.

WO2012/106385 discloses methods for detection of target molecules, including multiplexed target detection. WO2014/026032 discloses methods for detection of targets, including by use of tags that bind the target and represent the target identity.

### SUMMARY OF THE INVENTION

The present disclosure describes methods, compositions, and kits for the detection of a plurality of target nucleic acid sequences in single cells. The disclosed methods are suitable for detection of nucleic acid target molecules in general, and are especially suited for detection of mRNA target molecules in single cells present in a mixture of cells, where the individual cell origination information is retained.

The invention provides a method for detecting one or more target molecules in a sub-population of cells, the method comprising:
a) contacting a sample comprising a complex mixture of cells with two unique binding agents, wherein the two unique binding agents are proximity oligonucleotide probes designed to hybridize to complementary segments of a target mRNA that are in close proximity to each other, and wherein at least one of the two oligonucleotide probes is attached to an oligonucleotide epitope specific barcode that represents the identities of the target molecules;
b) linking the proximity oligonucleotide probes together using bridge oligonucleotides;
c) sequentially attaching two or more oligonucleotide assayable polymer subunits to the epitope specific barcodes using a split-pool combinatorial synthesis approach to create unique cell origination barcodes that represent the identities of individual cells to which the unique proximity oligonucleotide probes have bound;
d) selectively amplifying and sequencing the epitope specific barcode and cell origination barcodes associated with the proximity oligonucleotide probes to identify a sub-population of cells expressing the target RNA.

Disclosed herein is a method for identifying a target nucleic acid molecule in a single cell, the method comprising: a) providing a first oligonucleotide proximity probe comprising an epitope specific barcode sequence and a first target recognition sequence that is capable of hybridizing to a first segment of the target nucleic acid sequence; b) providing a second oligonucleotide proximity probe comprising a second target recognition sequence that is capable of hybridizing to a second segment of the target nucleic acid sequence, wherein the first and second segments of the target nucleic acid sequence are different and are separated from each other by a specified number of nucleotides, N; and c) providing a bridge oligonucleotide that comprises two probe recognition sequences, wherein the first probe recognition sequence is capable of hybridizing to a segment of the first oligonucleotide proximity probe, and the second probe recognition sequence is capable of hybridizing to a segment of the second oligonucleotide proximity probe, thereby creating a target specific probe complex that includes the epitope specific barcode.

In some embodiments, the first and second proximity probes and bridge oligonucleotide are covalently joined using a ligase or polymerase reaction. In some embodiments, the method further comprises attaching two or more assayable polymer subunits to the target specific probe complex in an ordered manner to create a unique cell origination barcode that represents the identity of the single cell. In some embodiments, the two or more assayable polymer subunits are attached to the target specific probe complex in successive rounds of split-pool synthesis. In some embodiments, the attaching comprises hybridization to an oligonucleotide template molecule, wherein one end of the template molecule is complementary to the target specific probe complex, and wherein the assayable polymer subunits and target specific probe complex are covalently joined after hybridization using a ligase reaction. In some embodiments, the oligonucleotide template molecule comprises a stop code sequence positioned between the sections of the template molecule sequence to which the assayable polymer subunits hybridize, thereby inhibiting amplification of the oligonucleotide template molecule during amplification reactions. In some embodiments, the stop code sequence comprises a poly-dT sequence. In some embodiments, the stop code sequence comprises a poly-T sequence. In some embodiments, the stop code sequence comprises a three carbon linker. In some embodiments, at least one of the first or second oligonucleotide proximity probes further comprises one or more primer sequences. In some embodiments, the cell origination barcode further comprises one or more primer sequences. In some embodiments, at least one of the primer sequences is an amplification primer sequence. In some embodiments, the disclosed method further comprises amplifying and sequencing all or a portion of the complete set of cell origination barcodes and their associated epitope specific barcodes. In some embodiments, at least one of the primer sequences is a sequencing primer sequence. In some embodiments, the target nucleic acid molecule is a DNA molecule. In some embodiments, the target nucleic acid molecule is an RNA molecule. In some embodiments, the RNA molecule is an mRNA molecule. In some embodiments, the oligonucleotide proximity probes are DNA molecules. In some embodiments, the oligonucleotide proximity probes are 10 to 200 nucleotides in length. In some embodiments, the target recognition sequences are 5 to 50 nucleotides in length. In some embodiments, the epitope specific barcode is 5 to 50 nucleotides in length. In some embodiments, N is between 1 and 20. In some embodiments, N is between 20 and 40. In some embodiments, N is between 40 and 100. In some embodiments, the bridge oligonucleotide is a DNA molecule. In some embodiments, the bridge molecule's probe recognition sequences are 5 to 50 nucleotides in length. In some embodiments, the assayable polymer subunits comprise nucleic acid sequences. In some embodiments, the method is multiplexed. In some embodiments, the method further comprises attachment of an additional primer to an end of the cell origination barcode. In some embodiments, one of the oligonucleotide proximity probes further comprises the bridge oligonucleotide. In some embodiments, the bridge oligonucleotide functions as the template molecule for attachment of one or more assayable polymer subunits. In some embodiments, two or more template molecules are used to assembly the cell origination barcode.

Also disclosed herein is a method for detection of a target mRNA sequence, the method comprising: (a) lysing a cell sample to release mRNA; (b) contacting the lysed cell sample with a plurality of beads, wherein a bead comprises a plurality of tethered oligonucleotide sequences capable of hybridizing to the released mRNA molecules; (c) annealing a first oligonucleotide proximity probe with the hybridized mRNA molecules on the plurality of beads, wherein the first oligonucleotide proximity probe comprises an epitope specific barcode sequence and a first target recognition sequence that is capable of hybridizing to a first segment of the target nucleic acid sequence; (d) annealing a second oligonucleotide proximity probe with the hybridized mRNA molecules on the plurality of beads, wherein the second oligonucleotide proximity probe comprises a second target recognition sequence that is capable of hybridizing to a second segment of the target nucleic acid sequence, and wherein the first and second segments of the target nucleic acid sequence are different and are separated from each other by a specified number of nucleotides, N; (e) annealing a bridge oligonucleotide with the hybridized oligonucleotide proximity probes on the plurality of beads, wherein the bridge oligonucleotide comprises two probe recognition sequences, wherein the first probe recognition sequence is capable of hybridizing to a segment of the first oligonucleotide proximity probe, and the second probe recognition sequence is capable of hybridizing to a segment of the second oligonucleotide proximity probe, thereby creating a target specific probe complex that includes the epitope specific barcode; and (f) ligating the annealed oligonucleotide proximity probes and bridge oligonucleotide to create a covalently joined target specific probe complex.

In some embodiments, the plurality of tethered oligonucleotide sequences further comprise one or more primer sequences. In some embodiments, the plurality of tethered oligonucleotide sequences comprise poly-dT target recognition sequences. In some embodiments, the method further comprises amplification of the target specific probe complex comprising the epitope specific barcode using one or more target specific primers. In some embodiments, the method further comprises sequencing the amplification product to detect or quantify the presence of one or more mRNA sequences.

Disclosed herein is a composition comprising: (a) a first oligonucleotide proximity probe comprising an epitope specific barcode and a first target recognition sequence, wherein the first target recognition sequence is capable of hybridizing to a first segment of a target nucleic acid molecule scqucncc; (b) a second oligonucleotide proximity probe comprising a second target recognition sequence, wherein the second target recognition sequence is capable of hybridizing to a second segment of the target nucleic acid molecule sequence; and (c) a bridge oligonucleotide that comprises first and second probe recognition sequences, wherein the first probe recognition sequence is hybridized to a segment of the first oligonucleotide proximity probe, and the second probe recognition sequence is hybridized to a segment of the second oligonucleotide proximity probe.

The composition may further comprise the target nucleic acid molecule. The composition may further comprise a cell origination barcode comprising at least one assayable polymer subunit, wherein the at least one assayable polymer subunit is attached to one of the oligonucleotide proximity probes. In some embodiments, the first and second target recognition sequences are each at least 80% complementary to the respective first and second target sequence segments over a range of 10-30 base pairs. In some embodiments, the first and second probe recognition sequences of the bridge oligonucleotide are each at least 80% complementary to the corresponding segments of the first and second oligonucleotide proximity probes over a range of 10-30 base pairs. In some embodiments, the first and second oligonucleotide proximity probes and the bridge oligonucleotide are covalently connected. In some embodiments, the composition futher comprises one or more primers. In some embodiments, at least one of the primers is an amplification primer. In some embodiments, at least one of the primers is a sequencing primer.

Also disclosed herein is a kit comprising: (a) a first oligonucleotide proximity probe comprising an epitope specific barcode and a first target recognition sequence that is capable of hybridizing to a first segment of a target nucleic acid sequence; (b) a second oligonucleotide proximity probe comprising a second target recognition sequence that is capable of hybridizing to a second segment of the target nucleic acid sequence; and (c) a bridge oligonucleotide that comprises two probe recognition sequences, wherein the first probe recognition sequence is capable of hybridizing to a segment of the first oligonucleotide proximity probe, and the second probe recognition sequence is capable of hybridizing to a segment of the second oligonucleotide proximity probe; wherein the kit provides means for the detection and quantitation of target nucleic acid molecules in individual cells or mixtures of cells.

The kit may further comprise a plurality of assayable polymer subunits for split-pool synthesis of cell origination barcodes. The kit may further comprise reagents for enzymatic or chemical coupling of assayable polymer subunits. At least one of the oligonucleotide proximity probes may further comprise one or more primers. In some embodiments, the cell origination barcode synthesized from the plurality of assayable polymer subunits further comprises one or more primers. In some embodiments, at least one of the primers is an amplification primer. In some embodiments, at least one of the primers is a sequencing primer.

The present disclosure also describes methods, compositions, and kits for the detection of a plurality of target molecules in single cells within selected sub-populations of cells in biological samples comprising complex mixtures of cells.

Disclosed herein are methods, compositions, and kits for identifying a sub-population within a mixed population of cells, the method comprising: (a) contacting the mixed population of cells with a unique binding agent, wherein the unique binding agent is designed to bind to a target molecule present in the sub-population, and wherein the unique binding agent is attached to an epitope specific barcode that represents the identity of the target molecule; (b) sequentially attaching two or more assayable polymer subunits to the epitope specific barcode to create unique cell origination barcodes that represent the identities of individual cells to which the unique binding agent has bound; and (c) decoding the epitope specific barcode and cell origination barcodes, thereby identifying the sub-population within the mixed population of cells.

In some embodiments, the epitope specific barcode and assayable polymer subunits comprise oligonucleotide sequences. In some embodiments, the two or more assayable polymer subunits are attached to the epitope specific barcode using a split-pool combinatorial synthesis approach. In some embodiments, each occurrence of the epitope specific barcode and the two or more assayable polymer subunits of an associated cell origination barcode are linked to form a conjugate that can be amplified and sequenced. In some embodiments, the disclosed methods further comprise amplification of epitope specific barcode - cell origination barcode conjugates. In some embodiments, the decoding step comprises sequencing all or a portion of the amplified epitope specific barcode - cell origination barcode conjugates. In some embodiments, a ratio of the number of cell origination barcodes associated with the sub-population to the total number of cells in the mixed population provides a measure of the fraction of cells within the mixed population that contain the target molecule. In some embodiments, two or more unique binding agents are used to identify the subpopulation. In some embodiments, two or more unique binding agents are used to identify two or more subpopulations. In some embodiments, at least one unique binding agent is designed to bind to a target molecule selected from the group consisting of DNA, histones, housekeeping proteins and proteins in general. In some embodiments, decoding the epitope specific barcode - cell origination barcode conjugates associated with at least one unique binding agent is used to identify the sub-population comprising dead cells, cell fragments, cell clusters, or combinations thereof. In some embodiments, the sub-population is identified on the basis of the amount of DNA, the amount of protein, or the ratio of DNA to protein detected in a barcoded entity. In some embodiments, the disclosed methods further comprise decoding the epitope specific barcode - cell origination barcode conjugates associated with at least a second unique binding agent to identify at least a second target molecule present in individual cells of the mixed population of cells, while excluding cell origination barcodes for the sub-population comprising dead cells, cell fragments, cell clusters, or combinations thereof from further analysis. In some embodiments, the at least one unique binding agent is an antibody or antibody fragment directed towards a target molecule selected from the group consisting of DNA, histones, and housekeeping proteins. In some embodiments, the at least one unique binding agent is a DNA intercalating molecule selected from the group consisting of berberine, ethidium bromide, proflavine, daunomycin, dactinomycin, doxorubicin, daunorubicin, and thalidomide. In some embodiments, the at least one unique binding agent comprises an amine-reactive probe selected from the group consisting of succinimidyl esters, sulfosuccinimidyl esters, tetrafluorophenyl esters, sulfodichlorophenol esters, isothiocyanates, and sulfonyl chlorides.

Also disclosed herein are methods, compositions, and kits for detecting one or more target molecules in a sub-population of cells, the method comprising: (a) contacting a sample comprising a complex mixture of cells with two or more unique binding agents, wherein the two or more unique binding agents are designed to bind to different target molecules, and wherein the two or more unique binding agents are attached to epitope specific barcodes that represent the identities of the target molecules; (b) sequentially attaching two or more assayable polymer subunits to the epitope specific barcodes to create unique cell origination barcodes that represent the identities of individual cells to which one or more unique binding agents have bound; (c) selectively amplifying and sequencing the epitope specific barcode and cell origination barcodes associated with at least a first unique binding agent to identify a sub-population of cells; and (d) selectively amplifying and sequencing the epitope specific barcode(s) associated with at least a second unique binding agent that are attached to cell origination barcodes matching those identified in step (c) to detect the presence of at least a second target molecule in individual cells of the specified sub-population of cells.

The disclosed methods, compositions, and kits, the epitope specific barcodes and assayable polymer subunits may comprise oligonucleotide scqucnccs. In some embodiments, the two or more assayable polymer subunits are attached to the epitope specific barcodes using a split-pool combinatorial synthesis approach. In some embodiments, each occurrence of an epitope specific barcode and the two or more assayable polymer subunits of an associated cell origination barcode are linked to form a conjugate that can be amplified and sequenced. In some embodiments, at least one unique binding agent comprises a nucleic acid sequence that is capable of hybridizing to an intracellular nucleic acid sequence. In some embodiments, the at least one unique binding agent comprises a nucleic acid sequence that is capable of hybridizing to a viral genome nucleic acid sequence. In some embodiments, the at least one unique binding agent comprises a nucleic acid sequence that is capable of hybridizing to an HIV viral genome nucleic acid sequence. In some embodiments, at least one unique binding agent comprises an antibody or antibody fragment directed towards a cell surface marker. In some embodiments, the selective amplification of step (d) comprises performing two or more successive rounds of multiplexed, nested amplification reactions. In some embodiments, each successive round of amplification utilizes a set of primers designed to hybridize to the assayable polymer subunits located at a different position in the sequence of two or more assayable polymer subunit positions that constitute the cell origination barcodes identified in step (c). In some embodiments, the set of primers utilized in the first round of amplification hybridize to the assayable polymer subunits located at a position in the cell origination barcode that is farthest from the epitope specific barcode, and wherein each successive round of amplification utilizes a set of primers that hybridize to the assayable polymer subunits located at a position one step closer to the epitope specific barcode.

Disclosed herein are methods, compositions, and kits for detecting one or more target protein molecules in a selected sub-population of cells, the method comprising: (a) contacting a sample comprising a complex mixture of cells with a non-specific binding agent comprising an amine-reactive probe for non-specific labeling of proteins, wherein the non-specific binding agent is attached to a non-specific protein barcode; (b) contacting the sample with a first unique binding agent designed to bind to a target molecule present in the sub-population, and wherein the first unique binding agent is attached to an epitope specific barcode that represents the identity of the target molecule; (c) sequentially attaching two or more assayable polymer subunits to the non-specific and epitope specific barcodes to create unique cell origination barcodes that represent the identities of individual cells; (d) immuno-precipitating one or more target protein molecules from a portion of the sample in which cells have been lysed using a set of beads, wherein each bead comprises an immobilized antibody that binds one of the target protein molecules and an immobilized primer comprising an antibody specific barcode, and wherein the immobilized primer is capable of hybridizing to one end of the non-specific barcode - cell origination barcode complexes associated with the target protein molecule for that antibody; (e) performing a primer extension reaction to create antibody specific barcode - non-specific barcode - cell origination barcode complexes; (f) amplifying and sequencing the collection of antibody specific barcode - non-specific barcode - cell origination barcode complexes; and (g) determining whether the one or more target protein molecules of interest are present in the sub-population of individual cells defined by at least the first unique binding agent by comparing the list of cell origination barcodes for the one or more target protein molecules with the list of cell origination barcodes associated with the at least first unique binding agent.

In some embodiments, the non-specific barcode, epitope specific barcode, and assayable polymer subunits comprise oligonucleotide sequences. In some embodiments, each occurrence of the non-specific barcode or epitope specific barcode and the two or more assayable polymer subunits of an associated cell origination barcode are linked to form a conjugate that can be amplified and sequenced. In some embodiments, the amine-reactive probe is selected from the group including succinimidyl esters, sulfosuccinimidyl esters, tetrafluorophenyl esters, sulfodichlorophenol esters, isothiocyanates, and sulfonyl chlorides. In some embodiments, the first unique binding agent comprises an antibody, antibody fragment, or nucleic acid sequence. In some embodiments, the non-specific binding agent comprising an amine-reactive probe is replaced with a non-specific binding agent comprising a poly-dT oligonucleotide probe sequence for non-specific hybridization with mRNA molecules, and wherein the non-specific binding agent is attached to a non-specific mRNA barcode. In some embodiments, the methods, compositions, and kits disclosed herein further comprise replacing the beads of step (d) with beads comprising immobilized probe sequences, one unique probe sequence per bead, that are complementary to all or part of one or more mRNA molecules of interest and an immobilized primer comprising an mRNA specific barcode, and wherein the immobilized primer is capable of hybridizing to one end of the non-specific barcode - cell origination barcode complexes associated with the target mRNA molecule for that immobilized probe sequence. The methods, compositions, and kits disclosed herein may further comprise determining in step (g) whether the one or more target mRNA molecules of interest are present in the sub-population of cells defined by at least the first unique binding agent by comparing the list of cell origination barcodes for the one or more target mRNA molecules with the list of cell origination barcodes associated with the at least first unique binding agent.

Also disclosed herein are methods compositions, and kits for excluding a subpopulation of cells from further analysis when detecting target molecules in individual cells in a sample comprising a mixture of cells, the method comprising: (a) contacting the mixture of cells with a first set of unique binding agents, wherein each unique binding agent is designed to bind to a target molecule, and wherein each unique binding agent is attached to an epitope specific barcode that is the same for all of the unique binding agents in the set; (b) contacting the mixture of cells with a second set of unique binding agents, wherein each unique binding agent of the second set is designed to bind to a target molecule that is different from those of the first set of unique binding agents, and wherein each unique binding agent of the second set is attached to a unique epitope specific barcode that represents the identity of the target molecule; (c) sequentially attaching two or more assayable polymer subunits to create a set of unique cell origination barcodes that represent the identities of individual cells to which at least one unique binding agent has bound; (d) selectively amplifying and sequencing the epitope specific barcode and cell origination barcodes associated with the first set of unique binding agents to identify a sub-population; (e) selectively amplifying and sequencing the epitope specific barcodes and cell origination barcodes associated with the second set of unique binding agents; and (f) determining whether the target molecules for the second set of unique binding agents are present in the remaining individual cells of the sample after excluding the cells identified in step (d) by comparing the lists of cell origination barcodes generated in steps (d) and (e).

In some embodiments, the selective amplification of step (e) comprises performing two or more successive rounds of multiplexed, nested amplification. In some embodiments, each successive round of amplification utilizes a set of primers designed to hybridize to the assayable polymer subunits located at a different position in the sequence of two or more assayable polymer subunit positions that constitute the cell origination barcodes identified in step (d). In some embodiments, the set of primers utilized in the first round of amplification hybridize to the assayable polymer subunits located at a position in the cell origination barcode that is farthest from the epitope specific barcode, and wherein each successive round of amplification utilizes a set of primers that hybridize to the assayable polymer subunits located at a position one step closer to the epitope specific barcode. In some embodiments, the cell origination barcodes comprise four assayable polymer subunits. In some embodiments, the epitope specific barcodes and cell origination barcodes further comprise amplification primers. In some embodiments, the epitope specific barcodes and cell origination barcodes further comprise sequencing primers. In some embodiments, the epitope specific barcodes and cell origination barcodes further comprise Illumina sequencing primers. In some embodiments, the assayable polymer subunits comprise oligonucleotide sequences of about 15 to 35 nucleotides in length. In some embodiments, the assayable polymer subunits comprise oligonucleotide sequences having a variable coding region of between 3 and 10 nucleotides in length, flanked on either end by annealing regions of 6 to 12 nucleotides. In some embodiments, the assayable polymer subunits comprise oligonucleotide sequences having a variable 7 nucleotide coding region flanked on either end by annealing regions of 9 nucleotides in length. In some embodiments, the 7 nucleotide coding region is designed to provide error detection and correction capability. In some embodiments, the disclosed methods, compositions, and kits further comprise quantifying the amount of one or more target molecules present in individual cells in a sample comprising a mixture of cells.

Also disclosed herein is a composition comprising: (a) an amplified oligonucleotide barcode product that encodes the identity of a target molecule present in a single cell, wherein the oligonucleotide barcode comprises: (i) an epitope specific barcode sequence; and (ii) a cell origination barcode sequence.

The composition may further comprise one or more amplification primers. The composition may further comprise one or more sequencing primers. The one or more sequencing primers may be Illumina sequencing primers.

Also disclosed herein is a composition comprising: (a) a unique binding agent that is capable of binding or hybridizing to a target molecule of interest; and (b) a covalently attached oligonucleotide sequence, wherein the oligonucleotide sequence comprises an epitope specific barcode and one or more linker sequences, and wherein the linker sequences are capable of hybridizing with or covalently attaching to additional oligonucleotides.

In some embodiments, the unique binding agent is an antibody or antibody fragment. In some embodiments, the unique binding agent is an oligonucleotide probe designed to hybridize to a target sequence of interest.

Also disclosed herein is a composition comprising: (a) an assayable polymer subunit, wherein the assayable polymer subunit is an oligonucleotide comprising: (i) a variable coding region; and (ii) one or more linker sequences, wherein the linker sequences are capable of hybridizing with or covalently attaching to additional oligonucleotides.

Also disclosed herein is a kit comprising: (a) one or more unique binding agents further comprising epitope specific barcodes; (b) a set of assayable polymer subunits for combinatorial synthesis of cell origination barcodes; (c) enzymatic or chemical coupling reagents; and (d) a set of PCR amplification primers that includes primers designed to hybridize to each individual assayable polymer subunit of the set of assayable polymer subunits; wherein the kit provides instructions and methods for the detection and quantitation of target molecules in individual cells for samples comprising a mixture of cells.

In some embodiments, the one or more unique binding agents comprise antibodies or antibody fragments. In some embodiments, the one or more unique binding agents comprise nucleic acid sequences which are capable of hybridizing to viral genome nucleic acid sequences. In some embodiments, the one or more unique binding agents comprise nucleic acid sequences which are capable of hybridizing to HIV viral genome nucleic acid sequences.

Also disclosed is a non-transitory computer readable medium storing a program that provides analysis capabilities for decoding and grouping sequencing data for sets of epitope specific barcode - cell origination barcode conjugates. The program may further provide data visualization tools.

The present disclosure also describes methods, compositions, and kits for detecting target RNA sequences, comprising: (a) contacting a sample comprising a plurality of RNA sequences with an oligonucleotide probe , wherein the oligonucleotide probe comprises a target recognition region capable of hybridizing to a target RNA sequence and a target specific barcode; (b) attaching one or more assayable polymer subunits to the oligonucleotide probe in a sequential fashion to create a cell origination barcode; (c) performing a reverse transcription reaction to create a molecular complex comprising the cell origination barcode, target specific barcode, the target recognition region, and a cDNA copy of all or a portion of the target RNA sequence; (d) performing an amplification reaction to amplify the molecular complex; and (e) sequencing the amplified molecule complex.

In some embodiments, the target RNA sequence is an mRNA sequence. In some embodiments, the target recognition region comprises a sequence that is complementary to a portion of the target RNA sequence. In some embodiments, the target recognition region comprises a poly-dT sequence. In some embodiments, the molecular complex further comprises one or more primers. In some embodiments, one or more of the primers are amplification primers. In some embodiments, one or more of the primers are sequencing primers. In some embodiments, the amplification reaction is a PCR reaction. In some embodiments, the PCR reaction utilizes at least one amplification primer that comprises both a recognition region that is complementary to the cDNA copy of all or a portion of the target RNA sequence and a sequencing primer. In some embodiments, the methods further comprise addition of a poly-dG tail to the 3' end of the cDNA copy following the reverse transcription reaction, and wherein the PCR reaction utilizes at least one amplification primer that comprises both a poly-dC recognition region and a sequencing primer. In some embodiments, the PCR reaction utilizes at least one amplification primer that comprises both a semi-random recognition sequence that is complementary to the cDNA copy of all or a portion of the target RNA sequence and a sequencing primer. In some embodiments, the semi-random recognition sequence is designed to maximize the probability of the priming event occurring within 32 to 128 nucleotides from the point in the cDNA molecule corresponding to the 3' end of the target RNA sequence. In some embodiments, the design of the semi-random recognition sequence is based on the cDNA sequence, and takes the general form of NNNXXX, where NNN is any random set of three nucleotides, and XXX is a specific set of three nucleotides chosen to complement the cDNA sequence at a position of about 64 nucleotides from the point in the cDNA molecule corresponding to the 3' end of the target RNA sequence. In some embodiments, the disclosed methods further comprise addition of one or more blocking oligonucleotide sequences prior to performing the PCR amplification, wherein the blocking oligonucleotides are complementary to mRNA sequences corresponding to housekeeping genes or other unwanted target RNA sequences and thereby prevent the formation of unwanted amplification products. In some embodiments, the sample comprises a single cell.

Also disclosed herein is a semi-random primer for use in amplification of target oligonucleotide sequences, the primer comprising an oligonucleotide sequence of the form of (M)ᵢ(X)ⱼ(N)ₖ, wherein (M)ᵢ and (N)ₖ are any random oligonucleotide nucleotide sequences of length i and k respectively, and wherein (X)ⱼ is a specific oligonucleotide sequence of length j chosen to complement the target oligonucleotide sequence at a specified position relative to the 3' end of the target oligonucleotide sequence.

In some embodiments, (X)ⱼ is chosen so that when utilized in an amplification reaction, the semi-random primer hybridizes with the target oligonucleotide sequence at the specified position and the amplification reaction yields product that is approximately Z nucleotides in length. In some embodiments, Z is between 50 and 1000. In some embodiments, Z is between 100 and 300. In some embodiments, Z is 250. In some embodiments, i has a value ranging from 0 to 6. In some embodiments, j has a value ranging from 3 to 6. In some embodiments, k has a value ranging from 0 to 6.

### BRIEF DESCRIPTION OF THE DRAWINGS

A better understanding of the features and advantages of the present invention will be obtained by reference to the following detailed description that sets forth illustrative embodiments, in which the principles of the invention are utilized, and the accompanying drawings of which:
FIG. 1 shows a schematic illustration of one embodiment of a molecular complex comprising a unique binding agent (UBA), epitope specific barcode (ESB), and a series of assayable polymer subunits (APS) assembled to create a cell origination barcode (COB).

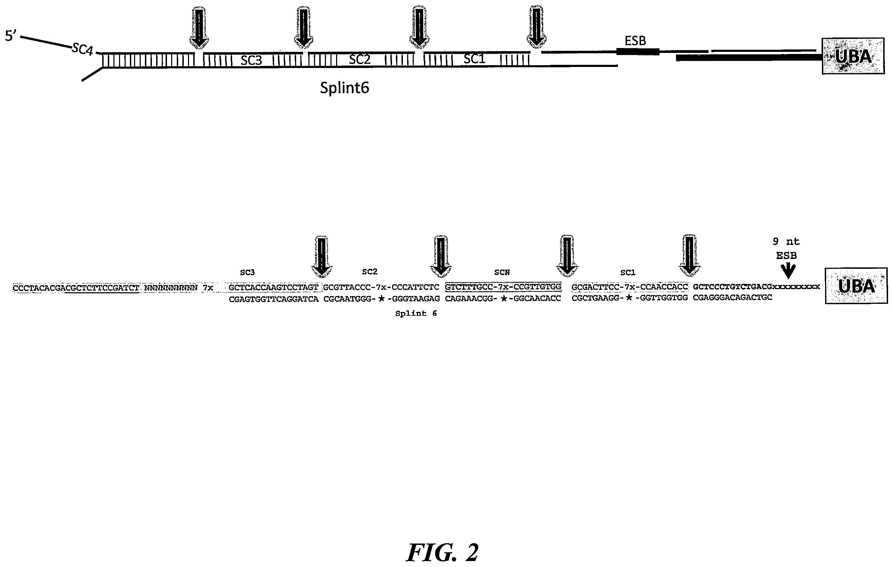

FIG. 2 shows a schematic illustration of one embodiment of a molecular complex comprising a unique binding agent (UBA), an epitope specific barcode (ESB) comprising a 9 nucleotide code (lower figure), and a cell origination barcode (COB) comprising 4 APS codes (SC1 - SC4). In this non-limiting example, the ESB is attached to the UBA by means of annealing to a complementary region of an oligonucleotide linker covalently attached to the UBA (upper figure). Each APS code comprises a 7 nucleotide code (lower figure) flanked on either end by annealing sequences that are complementary to a splint oligonucleotide (Splint 6), which is itself annealed to a complementary region of the ESB. Following the combinatorial assembly of the COB by means of annealing to the splint oligonucleotide, the APS subunits are ligated (at positions indicated by arrows) to form a single, covalently molecular complex comprising the ESB and COB.
FIG. 3 shows a graphical representation of the molecular components of the epitope specific barcodes and cell origination barcodes of the disclosed methods and compositions, and their assembly to form molecular barcoding complexes.
FIG. 4 shows a schematic illustration of the UBA-ESB components in one embodiment of the disclosed methods and compositions, and their use in detection of multiple epitopes in individual cells. In some embodiments, the UBA-ESB complexes further comprise a common linker (CL) used for assembly of cell origination barcodes.
FIG. 5 illustrates the first round of a split-pool synthetic route to creating unique cell origination barcodes. After treating with a cell sample with a plurality of UBA-ESB complexes, the sample is divided into a series of aliquots and a first APS is coupled to the bound UBA-ESB complexes within individual cells, wherein each sample aliquot is treated with a different APS.

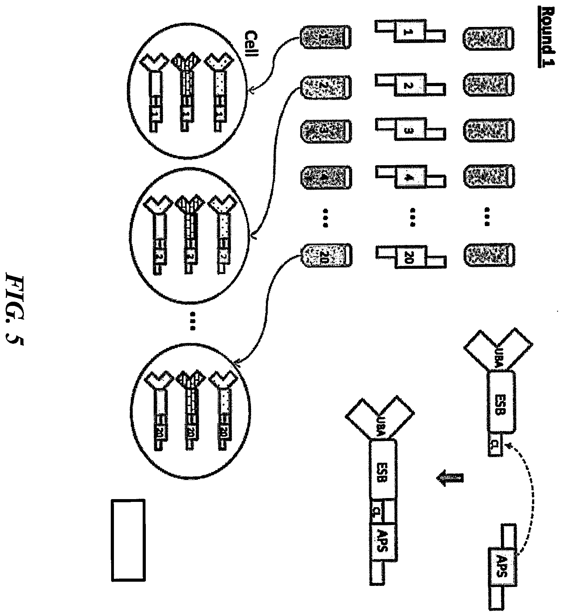

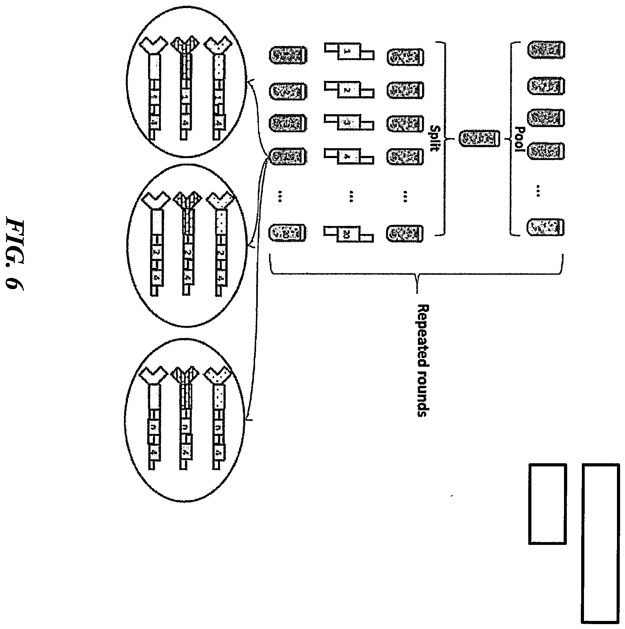

FIG. 6 illustrates subsequent rounds of a split-pool synthetic route to creating unique cell origination barcodes. Following the first APS coupling round illustrated in FIG. 5, the sample aliquots arc pooled, mixed, and redistributed into a new series of aliquots. A second APS unit is then coupled to the bound UBA-ESB-APS complexes generated in the first coupling step, wherein again each sample aliquot is treated with a different APS. Performance of repeated rounds of sample aliquoting, coupling reactions, and pooling of the cell sample split-pool results in the synthesis of a set of substantially unique cell origination barcodes.
FIG. 7 illustrates one example of a proximity probe set for detection and barcoding of target mRNA molecules, the probe set comprising a pair of oligonucleotide proximity probes **15** and **19,** each of which includes a sequence region that is complementary to the target mRNA sequence, and which may be joined using a bridge oligonucleotide **20,** and which may further comprise one or more primer sequences, epitope specific barcode regions, and common linker regions for use in creating unique cell origination barcodes using the compositions and methods of the present disclosure.
FIG. 8 illustrates another example of a proximity probe set for targeting specific mRNA molecules.
FIG. 9 illustrates an example of the process used for labeling each occurrence of a target mRNA molecule within a cell with a unique cell origination barcode. In this example, the UBA comprises a sequence-specific oligonucleotide probe that hybridizes to CD4 mRNA.
FIG. 10 illustrates another example of a proximity probe set for detecting and barcoding target mRNA molecules that utilizes two splint molecules and a bridge oligonucleotide in addition to the two proximity probe sequences.
FIG. 11 illustrates non-limiting examples of the oligonucleotide sequences used in creating the proximity probe set illustrated in FIG. 10.
FIG. 12 illustrates another non-limiting example of a proximity probe set for detecting and barcoding target mRNA molecules, which utilizes a single, combined splint-bridge oligonucleotide to join the two proximity probes.
FIG. 13 illustrates non-limiting examples of the oligonucleotide sequences used in creating the proximity probe set illustrated in FIG. 12.
FIG. 14 illustrates another non-limiting example of a proximity probe set for detecting and barcoding target mRNA molecules, which utilizes a single, combined splint-bridge oligonucleotide to join the two proximity probes.
FIG. 15 illustrates a non-limiting example of a splint oligonucleotide used for assembly of APS comprising coding regions SC1 - SC4 into a unique cell origination barcode. The lower figure indicates one example of an oligonucleotide for barcoding a UBA comprising an antibody or antibody fragment.
FIG. 16 illustrates a non-limiting example of a splint oligonucleotide molecule used for assembly of APS comprising coding regions SC1 - SC3 into a unique cell origination barcode. In some examples of the disclosed methods and compositions, the UBA may be an antibody. In other examples, the UBA may comprise an oligonucleotide probe sequence, e.g. an oligonucleotide probe that is specific for an RNA or mRNA sequence. The assembled cell origination barcode may further comprise one or more amplification primer and/or sequencing primer sequences.
FIG. 17 illustrates a non-limiting example of a splint oligonucleotide molecule used for assembly of APS comprising coding regions SC1 - SC3 into a unique cell origination barcode that may include PCR amplification and sequencing primers.
FIG. 18 illustrates a non-limiting example of a method for barcoding mRNA molecules using a generic poly-T primer sequence.
FIG. 19 illustrates a non-limiting example of a method for barcoding mRNA molecules using a target mRNA sequence-specific primer.
FIG. 20 illustrates a non-limiting example of a proximity probe set and splint oligonucleotide molecule used for assembly of APS comprising coding regions SC1 - SC3 into a unique cell origination barcode for specific mRNA target molecules (or oligonucleotide-tagged antibodies).
FIG. 21 illustrates a non-limiting example of extending the length (i.e. the number of sub-code regions) of a COB by hybridizing a second splint molecule (Splint SP-V5) to the 5' end of the growing COB assembled using a first splint molecule (Splint SP-V4).
FIG. 22 illustrates non-limiting examples of proximity probe sets (including pairs of target specific probes which may further comprise "bridge" sequences, and one or more "splint" oligonucleotide molecules) used for assembly of APS to create unique cell origination barcodes for target mRNA molecules, where the number of complementary sequence recognition events and the proximity requirements thereof combine to provide for increased target detection specificity.
FIG. 23 illustrates additional non-limiting examples of proximity probe sets (including pairs of target specific probes which may further comprise bridge sequences, and one or more splint oligonucleotide molecules) used for assembly of APS to create unique cell origination barcodes for target mRNA molecules, where the number of complementary sequence recognition events and the proximity requirements thereof combine to provide for increased target detection specificity.
FIG. 24 illustrates one embodiment for using the disclosed compositions and barcoding methods to identify a sub-population of rare cells, e.g. cells containing HIV viral nucleic acid sequences, within a sample comprising a mixed population of cells, and to detect a specific set of target molecules, e.g. protein X, in individual cells of the identified sub-population.
FIG. 25 illustrates one embodiment for using the disclosed compositions and methods for resampling a barcoded population of rare cells, e.g. cells containing HIV viral nucleic acid sequences, to detect a specific set of target molecules, e.g. protein X, on an individual cell basis, wherein the target molecule(s) of interest were unknown at the time that the barcoding was performed.
FIG. 26 illustrates methods for attaching self-circularizing oligonucleotide barcodes to antibodies or antibody fragments, where the oligonucleotide is attached to the protein molecule via a linker (left) or via annealing to a connection oligonucleotide (right). Oligonucleotide-tagged antibodies enable detection of low abundance proteins through the use of immuno-PCR reactions, wherein the oligonucleotide barcodes corresponding to bound antibodies are amplified and detected by means of quantitative PCR or sequencing.
FIG. 27 illustrates a non-limiting example of a process for barcoding each occurrence of a bound antibody-EST (epitope specific tag) complex with a unique cell origination barcode using a hairpin oligonucleotide structure containing a photo-cleavable bond.
FIG. 28 illustrates non-limiting examples of the hairpin-forming oligonucleotide sequences used in the COB assembly process illustrated in FIG. 27.

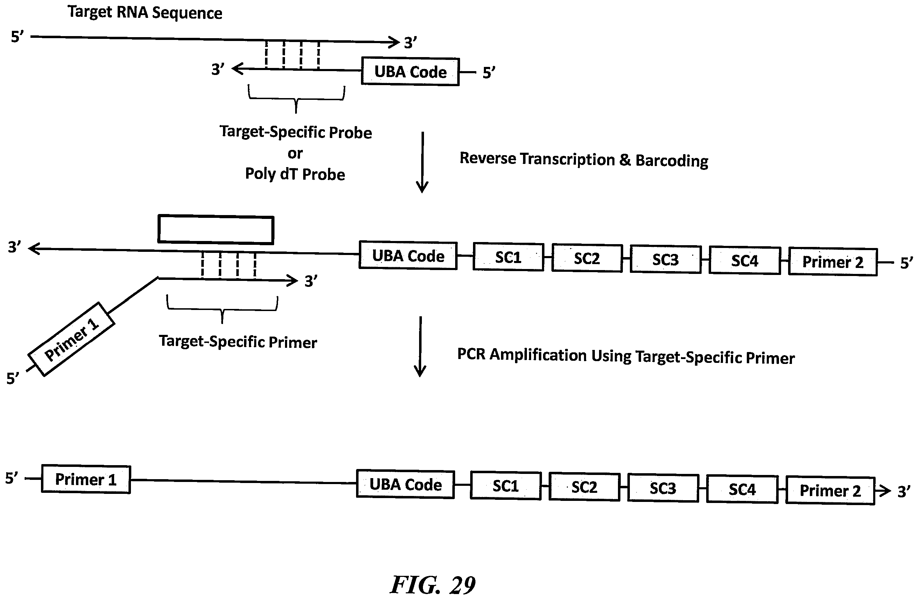

FIG. 29 illustrates one embodiment of the disclosed methods for identifying individual cells that contain one or more target RNA molecules of interest. A UBA comprising either a target-specific oligonucleotide probe sequence or a poly-dT oligonucleotide probe sequence is attached to a UBA code sequence (i.e. an ESB). Following hybridization with target RNA sequences in fixed, permeabilized cells, reverse transcription and barcoding reactions are performed to create a UBA-ESB-COB conjugate that includes a unique cell specific barcode for identifying the cell of origin. In some embodiments of the disclosed methods, a target specific primer that also comprises a sequencing primer is used to selectively amplify the cell origination barcodes associated with one or more target RNA molecules of interest, thereby creating amplification products that may be sequenced to decode the identity of the RNA target and the identities of individual cells in which the target RNA molecule was detected.

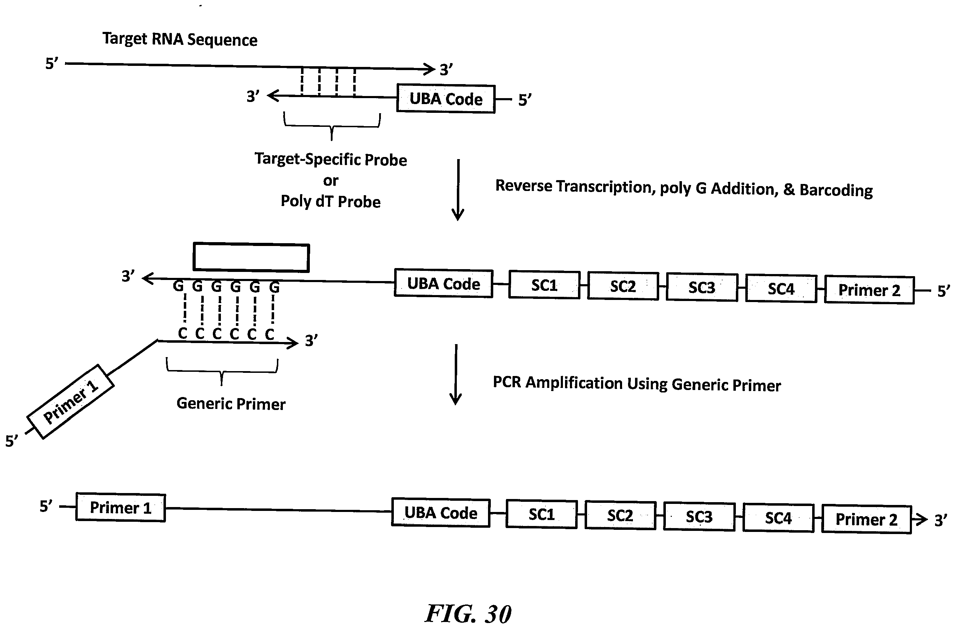

FIG. 30 illustrates an alternative embodiment of the disclosed methods for identifying individual cells that contain one or more target RNA molecules of interest. A UBA comprising either a target-specific oligonucleotide probe sequence or a poly-dT oligonucleotide probe sequence is attached to a UBA code sequence (i.e. an ESB). Following hybridization with target RNA sequences in fixed, permeabilized cells, reverse transcription, poly-dG addition, and barcoding reactions are performed to create a UBA-ESB-COB conjugate that includes a unique cell specific barcode for identifying the cell of origin. In this non-limiting embodiment, a poly-dC primer that optionally also comprises a sequencing primer is used to amplify all of the UBA-ESB-COB conjugates. Target specific primers further comprising sequencing primers may then subsequently be used to selectively amplify the cell origination barcodes associated with one or more target RNA molecules of interest, thereby creating amplification products that may be sequenced to decode the identity of the RNA target and the identities of individual cells in which the target RNA molecule was detected.

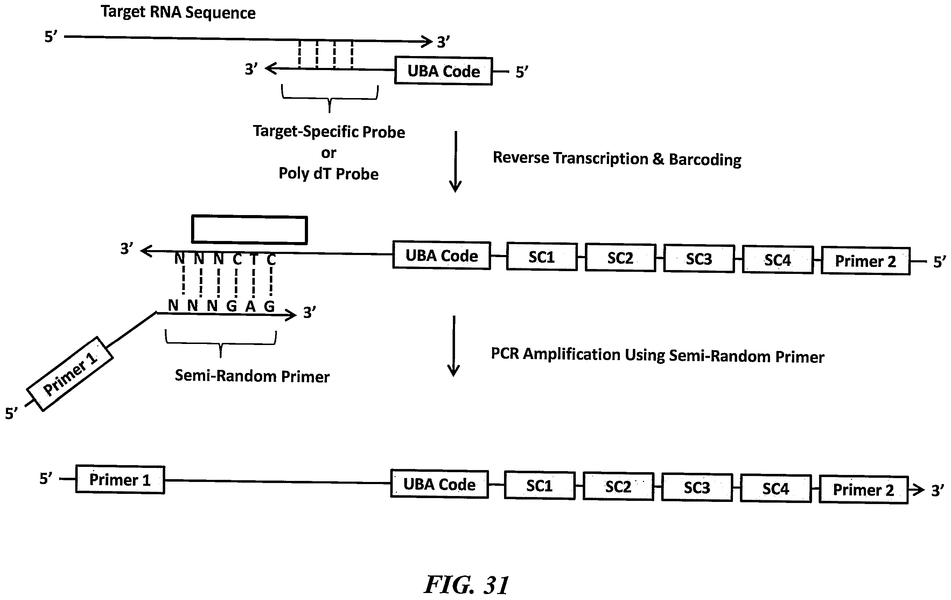

FIG. 31 illustrates yet another embodiment of the disclosed methods for identifying individual cells that contain one or more target RNA molecules of interest. A UBA comprising either a target-specific oligonucleotide probe sequence or a poly-dT oligonucleotide probe sequence is attached to a UBA code sequence (i.e. an ESB). Following hybridization with target RNA sequences in fixed, permeabilized cells, reverse transcription and barcoding reactions are performed to create a UBA-ESB-COB conjugate that includes a unique cell specific barcode for identifying the cell of origin. In some embodiments of the disclosed methods, a semi-random primer (e.g. having the sequence NNNGAG) that also comprises a sequencing primer is used to selectively amplify the cell origination barcodes associated with one or more target RNA molecules of interest, thereby creating amplification products that may be sequenced to decode the identity of the RNA target and the identities of individual cells in which the target RNA molecule was detected. The non-random portion of the semi-random primer is chosen to maximize the probability that the primer will be complementary to and hybridize with the UBA-ESB-COB conjugate at a position that is between 32 and 128 nucleotides from the position of the poly-dT or target specific sequence that was used to probe the target RNA, thereby ensuring that the amplified product is of an appropriate length to optimize the efficient use of the sequencing capacity of modern high-throughput sequencing systems.

### DETAILED DESCRIPTION

The present disclosure is an extension of the methods, compositions, and kits described previously in published patent applications WO2012/106385 and WO2014/026032. In particular, the present disclosure describes methods, compositions, and kits for the detection of a plurality of target nucleic acid sequences in single cells, and more specifically, detection of a plurality of target mRNA sequences in single cells, using proximity probes designed to minimize non-specific hybridization and amplification of background signal, thereby improving detection sensitivity and specificity. In some embodiments, the disclosed methods are applied to the detection of a plurality of target mRNA sequences within selected sub-populations of cells in biological samples comprising complex mixtures of cells. In particular, the present disclosure describes methods, compositions, and kits for the detection of a plurality of target molecules in single cells within selected sub-populations of cells in biological samples comprising complex mixtures of cells.

The present disclosure provides improvements over the previously disclosed techniques in that the disclosed methods provide means for (i) identifying dead cells, cell fragments, or cell clusters within a cell population and eliminating them from further analysis, thereby improving the quality of cell-specific data collected for a complex mixture of cells, and (ii) identifying rare cells or selected subpopulations of cells within the complex mixture of cells, based on the presence of specific intracellular or extracellular markers, and restricting the subsequent analysis to the selected set of cells, thereby improving the specificity of the data collected.

Multiplexed testing at the single cell level is a key advantage of the disclosed methods, and provides a number of potential benefits including improved understanding of the physiological processes within individual cells, reduced sample quantity requirements (proportional to the number of multiplexed measurements), improved testing accuracy (through the elimination of sample handling and measurement errors associated with replicate testing), and significant savings in terms of labor and cost.

### I. Definitions

As used herein, the phrase "unique binding agent" (UBA) refers to one of a variety of detection reagents for use in the disclosed methods. Each UBA is capable of binding or hybridizing to a single species of target molecule. It is this specificity of binding or hybridization that enables detection of the target molecule in a given individual cell.

As used herein, the term "epitope" is used in a more general sense to refer to the target molecule (including, but not limited to, proteins, peptides, DNA, RNA, mRNA, oligonucleotides, lipids, carbohydrates, and small molecules) or portion of a target molecule that is recognized by a unique binding agent. In common with the published patent applications referenced above, the terms "epitope" and "target molecule" are used interchangeably herein to refer to the molecule of interest (or a portion thereof) that is being detected and/or quantified by the methods described herein.

As used herein, the phrase "epitope specific barcode" (ESB) refers to a unique code that is associated with a specific epitope or target molecule. In some embodiments, the ESB is a molecule or assembly of molecules capable of encoding the identity of a target molecule. Examples of suitable ESB molecules or molecular assemblies include, but are not limited to, peptide sequences, oligonucleotide sequences, strings of covalently or non-covalently linked but distinguishable nanoparticles, and the like.

As used herein, the phrase "assayable polymer subunit" (APS) refers to a molecular building block comprising a distinct packet of information, wherein the molecular building blocks are capable of being linked in an ordered fashion to create cell origination barcodes. Examples of suitable assayable polymer subunits include, but are not limited to, amino acids, peptides, oligonucleotides, nanoparticles, and the like.

As used herein, the phrase "cell origination barcode" (COB) refers to an ordered assembly of assayable polymer subunits that creates a molecule or molecular assembly that encodes the identity of an individual cell. Examples of suitable COB molecules or molecular assemblies include, but are not limited to, peptide sequences, oligonucleotide sequences, strings of covalently or non-covalently linked but distinguishable nanoparticles, and the like.

As used herein, the phrase "common linker" (CL) refers to a linker moiety that may be directly or indirectly attached to UBA, ESB, or APS subunits for use in assembling molecular barcodes.

As used herein, the term "splint" refers to a template molecule used in the assembly of APS to form cell origination barcodes. In some embodiments, splint (or template, or annealing primer) molecules are oligonucleotides.

As used herein, the term "sub-code" (SC) refers to a unique coding region and/or a detectable molecule contained within an APS, wherein the serial combination of two or more APS create an individual COB having a detectable code or signal that distinguishes it from all other COB.

As used herein, the phrase "stop code" refers to a segment of a splint or template molecule that is designed to prevent replication or amplification of the splint or template molecule.

As used herein, the phrase "combinatorial synthesis" refers to synthetic methods that make it possible to synthesize large numbers of compounds (tens to thousands to hundreds of thousands, or more) in a single process comprising a minimal number of chemical coupling steps.

As used herein, the phrase "split-pool synthesis" refers to one example of a combinatorial synthesis process in which a reaction mixture is divided into several different aliquots prior to performing a coupling reaction, and wherein each aliquot receives a different monomer or component to be coupled. Following the coupling reaction, the aliquots are combined (pooled), mixed, and divided (split) into a new set of aliquots prior to performing the next round of coupling.

As used herein, the phrase "proximity probe" refers to each of a pair of probe molecules that are capable of hybridizing to different segments of the same target molecule. In some embodiments, proximity probes may be pairs of oligonucleotide probes capable of hybridizing to different segments of the same target oligonucleotide molecule. In some embodiments, the different segments of the target oligonucleotide recognized by the probes are in close proximity to each other.

As used herein, the phrase "bridge molecule" (or "bridge") refers to a connector molecule that is capable of binding or hybridizing to two corresponding proximity probes only when the latter are bound to, or hybridized with, their respective target molecule. In some embodiments, the bridge molecule is an oligonucleotide that is capable of simultaneously hybridizing to each of a pair of oligonucleotide proximity probes.

### II. Overview of Assay Methodology

The methods, compositions, and kits of the present disclosure provide means for the detection of a plurality of target molecules in single cells (including of selected sub-populations of cells) using a set of novel detection and barcoding reagents. In some embodiments of the disclosed methods, detection of a plurality of target molecules in single cells from selected sub-populations of cells is enabled. In general, the approach comprises the use of unique binding agents (UBA) to detect target molecules of interest, epitope specific barcodes (ESB) to encode the identities of the target molecules recognized by the UBA, and assayable polymer subunits (APS) to create unique cell origination barcodes (COB) that identify individual cclls, thereby allowing one to define a selected sub-population of cells within a sample comprising a complex mixture of cells on the basis of a specified set of biomarkers, and subsequently correlate the detection of one or more target molecules with individual cells in the selected sub-population of cells.

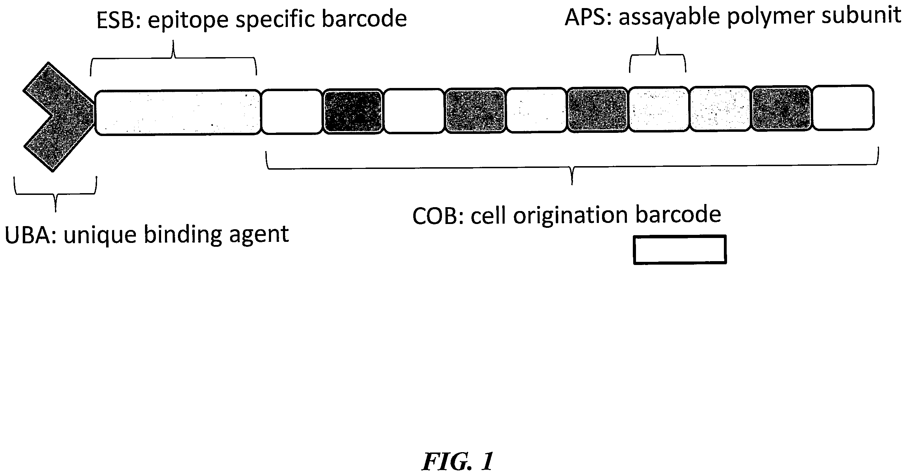

Unique binding agents comprise the detection reagents for use in the disclosed methods. Each UBA is specific for a single target molecule species, and provides the binding or hybridization specificity that enables detection of the target molecule in a given individual cell. In many embodiments of the disclosed methods, cell samples are incubated with one or more UBA (either prior to or following fixation and/or permeabilization of the cells), and non-bound UBA are subsequently rinsed away. Those UBA bound to target molecules on or within the cells of the sample may then subsequently be identified using epitope specific barcodes (ESB). Each ESB comprises a unique code that is associated with the UBA for a specific target molecule (FIG. 1). In other embodiments of the disclosed methods, the ESB are attached (either directly or indirectly) to the UBA prior to performing the assay. In some embodiments, the ESB are attached to the UBA following incubation of the sample with the UBA, e.g. as part of the assay procedure.

In addition to the ESB used to identify specific target molecules, the disclosed methods, compositions, and kits provide components for creating cell origination barcodes that provide a means for assigning detected target molecules to specific individual cells. Each individual COB comprises a unique code that is associated with a specific cell of origin. Thus the collection of UBA for an individual cell, as identified by their associated ESB, will share a common COB that is different from the COB for all other cells in the sample.

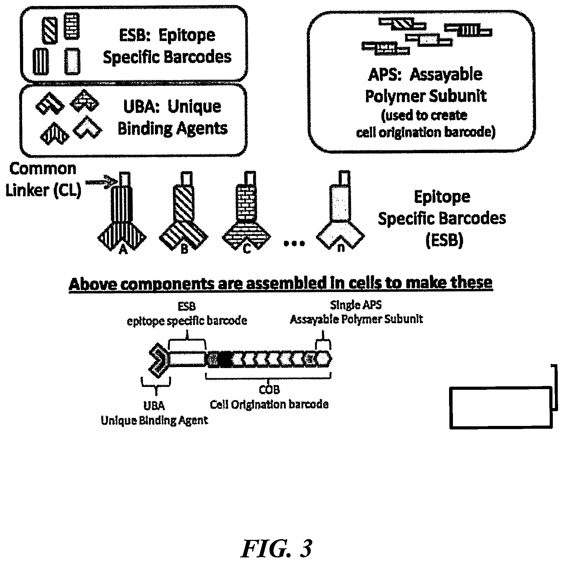

In some embodiments, the COB are composed of two or more assayable polymer subunits attached to the bound UBA-ESB complex (FIG. 1), wherein the set of APS further comprise a set of sub-codes (SC) (FIG. 2). Each SC comprises a unique coding region and/or a detectable molecule, wherein the serial combination of two or more APS create an individual COB having a detectable code or signal that distinguishes it from all other COB in the complete set of COB. Certain aspects of the present disclosure relate to the combinatorial synthesis of COB by linking a series of APS together via a split-pool synthesis approach (FIGS. 3-6), wherein the total number of unique COB that can be synthesized using a specified set of APS and a defined number of split-pool coupling rounds is significantly greater than the number of individual cells in the sample, thereby ensuring that the probability of any two individual cells having the same COB is extremely low.

Decoding of the ESB-COB complexes to identify the target molecules present in individual cells of the sample can be performed using a variety of techniques, as described in the published patent applications referenced above. In some embodiments, the ESB-COB complexes are decoded by amplification and sequencing. Accordingly, certain aspects of the present disclosure provide methods for barcoding cells using a plurality of UBA-ESB complexes and a set of APS, wherein each APS comprises a unique SC, and wherein the COB for each UBA-ESB-COB complex is the same for a given cell and distinct from those for all other cells, and wherein the amplification and sequencing of the complete set of ESB-COB complexes allows one to catalogue the complete set of target molecules associated with each individual cell in the sample or in a selected sub-population of cells. In some embodiments of the disclosed methods, compositions, and kits, selective amplification of UBA-ESB-COB complexes of interest is enabled through the design and use of target-specific or semi-random amplification primers that produce amplified product comprising only those sequences of interest and of an appropriate length to optimize the efficient use of the sequencing capacity of modern high-throughput sequencing systems.

### III. Compositions

### A. Unique Binding Agents (UBA)

UBAs are molecules or molecular assemblies that are designed to bind to or hybridize with at least one target molecule or portions thereof, and can, under appropriate conditions, form a molecular complex comprising the UBA and the target molecule. Examples of target molecules include, but are not limited to, proteins, peptides, nucleic acids, DNA, RNA, mRNA, lipids, carbohydrates, small organic molecules, drug molecules, organic monomers, and ions. For convenience, most of the methods, compositions, and kits described herein are explained within the context of UBA that bind to a target protein or a target mRNA. However, these methods, compositions, and kits can also be applied to other target molecules.

In some embodiments, UBA comprise at least one recognition element that allows them to bind to or interact with at least one target molecule, at least one part of at least one target molecule, at least one target molecule surrogate, at least part of a target molecule surrogate, or combinations thereof. UBA typically bind to or interact with target molecules in a sequence-specific manner, a conformation-specific manner, or a combination of both. Examples of suitable molecular recognition interactions include, but not limited to, antibody-antigen binding, receptor-ligand binding, aptamer-target binding, enzyme-substrate recognition, oligonucleotide probe - target sequence hybridization, and the like. Accordingly, suitable recognition elements for use in constructing UBA include, but are not limited to, antibodies, receptors, enzymes, peptoids, aptamers, peptide aptamers, nucleic acid aptamers, oligonucleotide probe sequences, and the like.

In some embodiments, UBA comprise at least one common linker (CL) element that allows them to attach to or hybridize with an ESB that encodes for the identity of the target molecule and/or a COB that encodes for the identity of a specific individual cell. The common linker may be directly or indirectly attached to the UBA. In some embodiments, the common linker element may be an oligonucleotide molecule. In some embodiments, the common linker element may be an oligonucleotide sequence that is covalently attached to the UBA, while in some embodiments it may be non-covalently attached to the UBA.

In some embodiments, UBA further comprise a capture region which may be used for isolation of the UBA and/or immobilization of the UBA on a surface. In some embodiments, the capture region may be an affinity tag, a bead, a slide, or an array. In some embodiments, the capture region is the associated ESB, for example, the ESB can be a detectable bead such as a bead with a unique spectral signature (e.g. a bead that incorporates specific fluorophores emitting in the visible, near-infrared, or infrared).

In some embodiments, the UBA comprise antibodies as recognition elements (FIG. 2). As used herein, the term "antibody" is used in a broad sense to include not only an intact antibody molecule, including but not limited to immunoglobulin A, immunoglobulin G, and immunoglobulin M, but also any immuno-reactive component(s) of an antibody molecule that specifically bind to at least one epitope. Such immuno-reactive components include, but are not limited to, Fab fragments, Fab' fragments, Fab'₂ fragments, single chain antibody fragments (scFv), miniantibodies, diabodies, crosslinked antibody fragments, Affibody™ molecules, cyclotide molecules, and the like. Immuno-reactive products derived using antibody engineering or protein engineering techniques are also expressly included within the meaning of the term "antibody" as used herein. Detailed descriptions of antibody and/or protein engineering, including relevant protocols, can be found in, for example, J. Maynard and G. Georgiou, Ann. Rev. Biomed. Eng. 2:339 76 (2000); Antibody Engineering, R. Kontermann and S. Dubel, eds., Springer Lab Manual, Springer Verlag (2001); U.S. Pat. No. 5,831,012; and Antibody Engineering Protocols, S. Paul, Humana Press (1995).

Those skilled in the art will appreciate that antibodies can be obtained from a variety of sources, including but not limited to polyclonal antibodies, monoclonal antibodies, monospecific antibodies, recombinantly expressed antibodies, humanized antibodies, plantibodies, and the like; and can be obtained from a variety of animal species, including rabbit, mouse, goat, rat, human, horse, bovine, guinea pig, chickcn, sheep, donkey, human, and the like. A wide variety of antibodies are commercially available from a variety of vendors, and custom-made antibodies can be obtained from a number of contract labs. Detailed descriptions of antibodies, including relevant protocols for production and use, can be found in, among other places, Current Protocols in Immunology, Coligan et al., eds., John Wiley & Sons (1999, including updates through August 2003); The Electronic Notebook: Basic Methods in Antibody Production and Characterization, G. Howard and D. Bethel, eds., CRC Press (2000); Monoclonal Antibodies: Principles and Practice, 3d Ed., J. Goding, Academic Press (1996); Using Antibodies, E. Harlow and D. Lane, Cold Spring Harbor Lab Press (1999); and Monoclonal Antibodies: A Practical Approach, P. Shepherd and C. Dean, Oxford University Press (2000).

In some embodiments, the antibodies described herein are attached to a nucleic acid, e.g., a common linker oligonucleotide or an ESB comprising an oligonucleotide sequence. One non-limiting example of an oligonucleotide sequence that comprises both a linker and an ESB is: where the NNNNNNNNN sequence is a 9 nucleotide code that is specific for the attached antibody. Methods for attaching nucleic acids to antibodies are well known in the art, and any suitable approach is encompassed within the presently disclosed methods, compositions, and kits. For example, in some embodiments antibodies may be attached to nucleic acid molecules using the methods described in Gullberg, et al. (2004), PNAS 101(22):228420-8424, and Boozer, et al. (2004), Analytical Chemistry 76(23):6967-6972. In some embodiments, antibodies may be attached to nucleic acid molecules by random coupling to free amines. In some embodiments, the antibodies may be attached to nucleic acid molecules by random coupling to free amines using a 10-to-1 ratio of nucleic acid to antibody. In some embodiments, antibodies may be attached to nucleic acid molecules using the methods described in Kozlov, et al. (2004), Biopolymers 5: 73 (5):621-630. In some embodiments, antibodies may be attached to nucleic acid molecules using hydrazine chemistry. In some embodiments, antibodies may be attached to nucleic acid molecules using "tadpoles" as described in Nolan (2005), Nature Methods 2:11 - 12. In general, antibodies may be attached to nucleic acid molecules using any suitable method known in the art for generating engineered antibodies, including the methods described herein.

In some embodiments of the disclosed methods, compositions, and kits, the UBA comprise nucleic acid sequences as recognition elements. Nucleic acid recognition elements may include target-specific recognition sequences, or generic target recognition sequences. Examples of suitable target recognition sequences include, but are not limited to, a poly-dT probe sequence for use in hybridization with mRNA molecules in general; an antisense DNA probe sequence for hybridization with a specific target mRNA, an oligonucleotide sequence designed to hybridize to an HIV viral sequence, and the like. The nucleic acid sequence is preferably at least 15 nucleotides in length, and more preferably is at least 20 nucleotides in length. In some embodiments, the target-specific recognition sequence is about 10 to 500, 20 to 400, 30 to 300, 40 to 200, or 50 to 100 nucleotides in length. In other embodiments, the target-specific sequence is about 30 to 70, 40 to 80, 50 to 90, or 60 to 100, 30 to 120, 40 to 140, or 50 to 150 nucleotides in length.

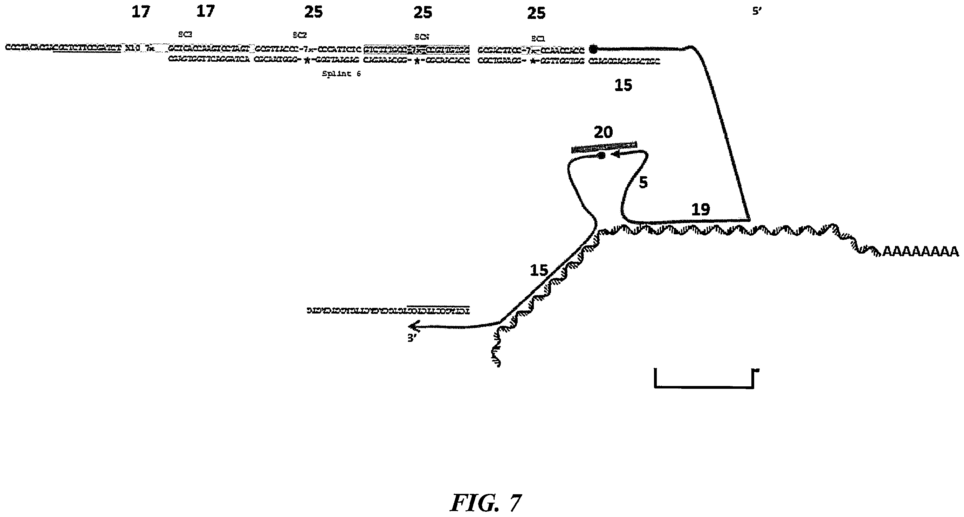

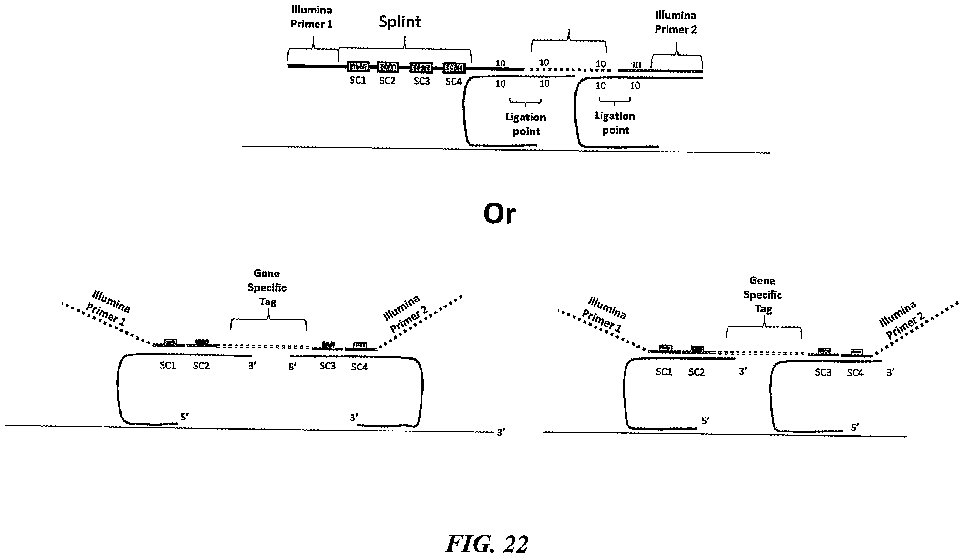

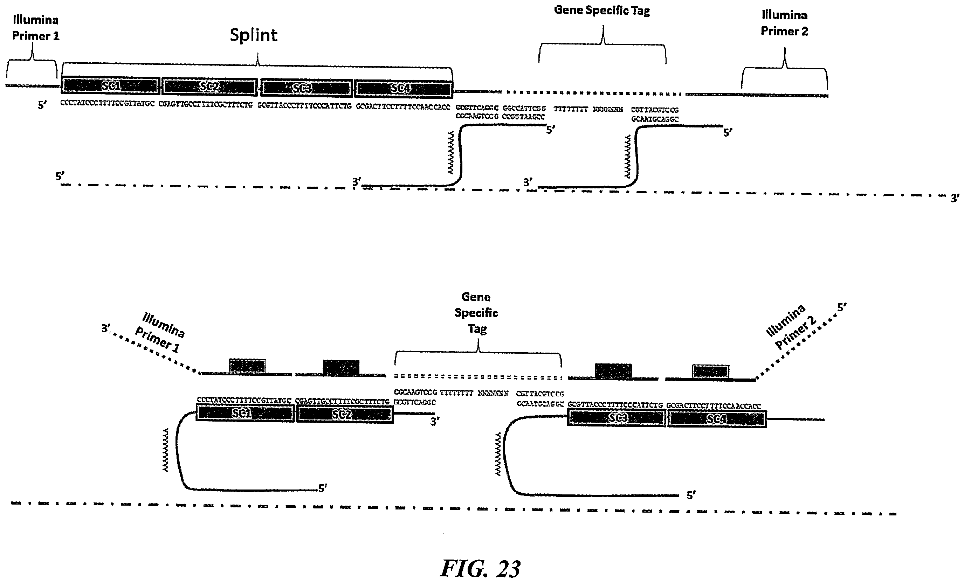

In some embodiments of the disclosed methods, compositions, and kits, the UBA comprise sets of oligonucleotide probes, e.g. a pair of proximity probes along with a bridge oligonucleotide sequence, which are designed to hybridize to a target nucleic acid molecule of interest, e.g. an mRNA molecule, with higher specificity than can be achieved using a single oligonucleotide recognition sequence. Examples of proximity oligonucleotide probe sets of the present disclosure that use a bridge molecule (e.g. a bridge oligonucleotide molecule) are illustrated in FIGS. 7, 8, 10, and 12. Additional examples of oligonucleotide probe sets of the present disclosure are illustrated in FIGS. 22 and 23. In some embodiments of the disclosed methods and compositions, the bridge molecule may be incorporated into a splint molecule used for assembly of the COB, and may incorporate one or more primer sequences as well.

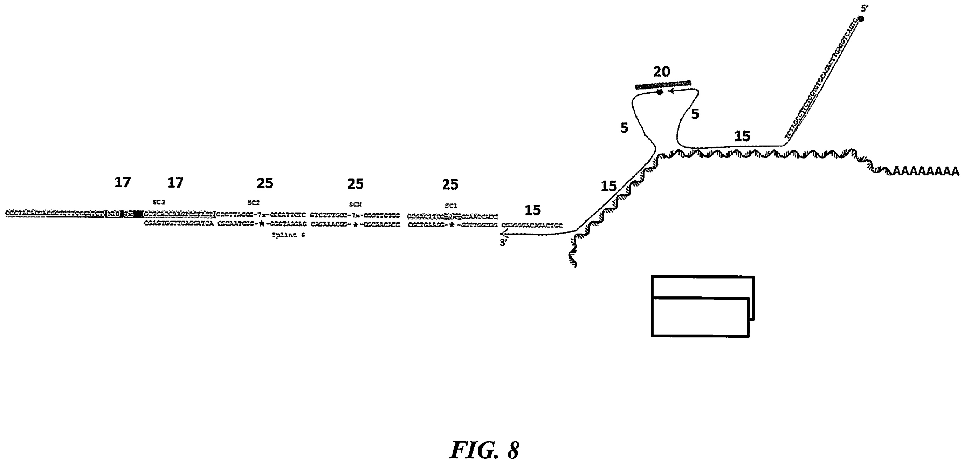

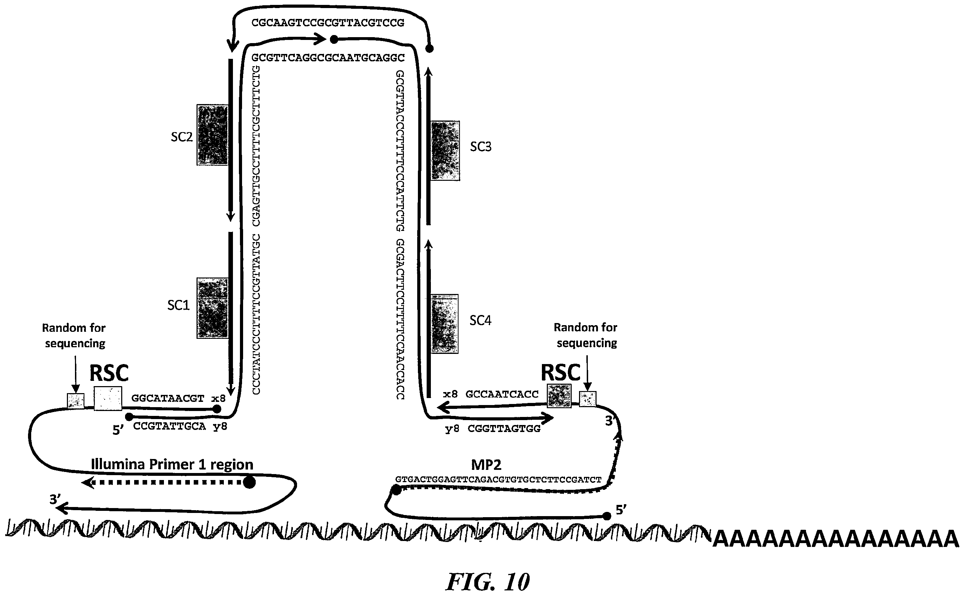

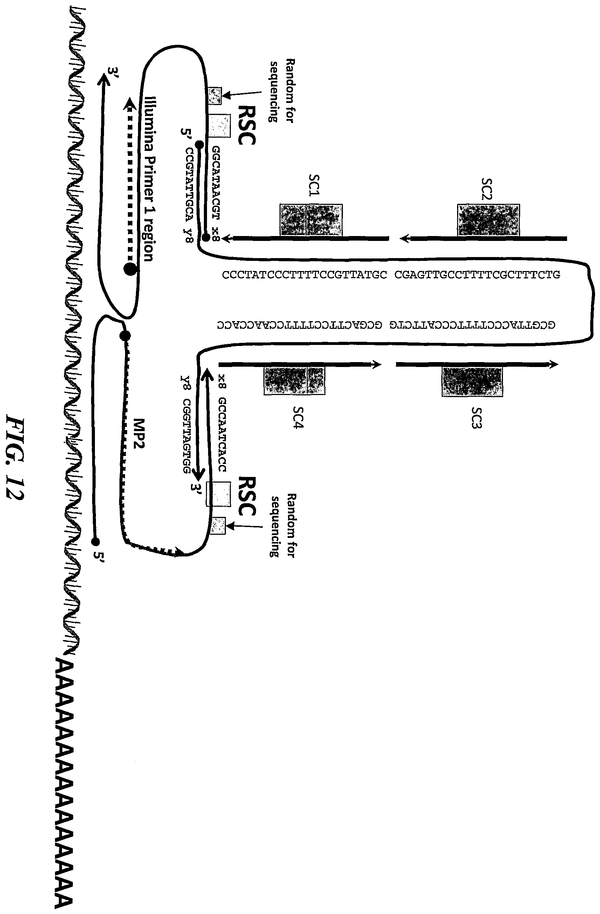

Referring to FIG 7 as illustrating one example, a UBA comprising a proximity probe set of the present disclosure comprises two oligonucleotide sequences, **15** and **19,** each of which are designed to hybridize to a complementary segment of a target mRNA molecule. Typically, the two proximity probes will be designed to hybridize to segments of the target mRNA that are in close proximity to each other, e.g. two target sequence regions that are separated by N nucleotides, where N ranges from 1 to 200 nucleotides. In many embodiments, one or the other of the proximity probes will further comprise an epitope specific barcode sequence. In some embodiments, a bridging oligonucleotide, **20,** is designed to hybridize to complementary sequence regions on each of the individual proximity probes, thereby forming a molecular complex that specifically recognizes the target mRNA, and which may further comprise amplification and sequencing primer sequences and/or common linkers for use in assembling unique cell origination barcodes. In some embodiments, the two proximity probes arc joined by ligation following annealing with the bridging molcculc, thereby forming a covalently linked molecular complex that can be amplified and sequenced. In some embodiments, the common linker used to assemble splint molecules and APS to form the COB is located at the 5' end of the probe complex (FIG. 7). In some embodiments, the common linker is located at the 3' end of the probe complex (FIG. 8). In some embodiments, the target-specific probe set comprises two target-specific proximity probes, two splint molecules for use in assembling APS comprising SC into a unique COB, and a bridging molecule (FIG. 10). In some embodiments, the target-specific probe set comprises two target-specific proximity probes, and a bridging molecule which itself functions as the splint (FIG. 12).

In some embodiments, the UBA may further comprise nucleic acid sequences comprising one or more primers, wherein the primers are used for amplification and/or sequencing of specific UBA probe sequences, ESB code sequences, COB sequences, or combinations thereof. Any suitable primer sequence may be used for amplification and/or sequencing, for example, the Illumina primers may be used for sequencing UBA-ESB-COB assemblies or conjugates, or portions thereof.

In some embodiments, the UBA may comprise a non-specific binding agent for recognition and binding to genomic DNA or chromosomal DNA structures, including but not limited to, for example, an antibody that binds DNA or histones, or a DNA intercalating molecule such as berberine, ethidium bromide, proflavine, daunomycin, dactinomycin, doxorubicin, daunorubicin, or thalidomide, to which an ESB may be attached.

In some embodiments, the UBA may comprise a non-specific binding agent for protein, including but not limited to, for example, an amine-reactive probe selected from the group consisting of succinimidyl esters, sulfosuccinimidyl esters, tetrafluorophenyl esters, sulfodichlorophenol esters, isothiocyanates, and sulfonyl chlorides, to which an ESB may be attached.

### B. Epitope Specific Barcodes (ESB)

The epitope specific barcodes of the present disclosure provide a unique code that is associated with a specific target molecule. ESB are molecules or molecular assemblies that are designed to attach to or bind to a UBA or portions thereof, and can, under appropriate conditions, form a molecular complex comprising the ESB, the UBA, and the target molecule.

In some embodiments, ESB comprise at least one common linker region that allows them to bind to or interact with at least one UBA and/or at least one APS, typically in a sequence-specific manner, a conformation-spccific manner, or a combination of both. Examples of suitable molecular binding interactions between the ESB and their associated UBA and/or APS include, but are not limited to, antibody-antigen binding, receptor-ligand binding, aptamer-target binding, enzyme-substrate interactions, oligonucleotide probe - target sequence hybridization, and the like. The interactions between the ESB and their associated UBA and/or APS are typically driven by ionic bonding, hydrogen bonding, or van der Waals forces. In some embodiments, the attachments between ESB and associated UBA and/or APS may be covalent. In some embodiments, the attachments are non-covalent. In some embodiments, the ESB are attached (either directly or indirectly) to the UBA prior to performing the assay. In other embodiments, the ESB bind to or are attached to the UBA following incubation of the sample with the UBA, i.e. as part of the assay procedure.

In some embodiments of the disclosed methods and compositions, the ESB comprise at least one coding region that encodes the identity of the attached UBA. In some embodiments, the ESB are oligonucleotide sequences, and the coding region comprises an oligonucleotide sequence that is between 5 and 15 nucleotides in length. In some embodiments, the coding region is an oligonucleotide sequence that is 9 nucleotides in length (FIG. 2).

In many embodiments, the ESB are oligonucleotide sequences that further comprise one or more primers, and all or part of the ESB nucleic acid sequence and/or associated COB may be amplified using any nucleic acid amplification method, including, but not limited to, polymerase chain reaction (PCR), branched chain reaction, or rolling circle amplification approaches, as are well known in the art.

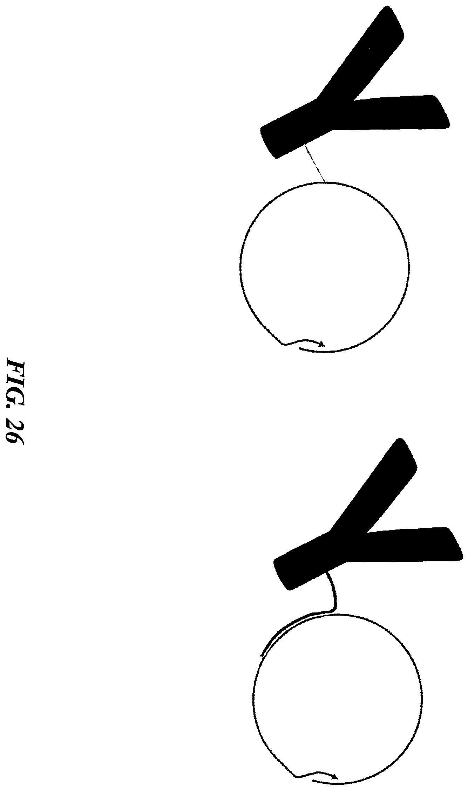

FIG. 26 illustrates non-limiting examples of methods for attaching self-circularizing oligonucleotide barcodes to antibodies or antibody fragments, where the oligonucleotide is attached to the protein molecule via a linker (left) or via annealing to a connection oligonucleotide (right). Oligonucleotide-tagged antibodies enable detection of low abundance proteins through the use of immuno-PCR reactions, wherein the oligonucleotide barcodes corresponding to bound antibodies are amplified and detected by means of quantitative PCR or sequencing.

In some embodiments, the ESB further comprise a capture region which may be used for isolation of UBA-ESB complexes and/or immobilization of the UBA-ESB complexes on a solid surface. In some embodiments, the capture region may be an affinity tag, a bead, a slide, or an array. In some embodiments, the capture region is the ESB, for example, the ESB can be a detectable bead such as a bead with a unique spectral signature (e.g. a bead that incorporates specific fluorophorcs emitting in the visible, ncar-infrarcd, or infrared). In some embodiments, the UBA is directly or indirectly attached to the capture region of the ESB.

### C. Cell Origination Barcodes (COB)

The presently disclosed methods, compositions, and kits further provide means for creating cell origination barcodes, wherein each COB provides a unique code that can be associated to a specific cell of origin. In some embodiments, attachment of a COB to one or more bound UBA-ESB complexes (e.g. using common linker oligonucleotides) identifies the cell of origin for the target molecule(s) to which UBA/ESB complexes have bound. In some embodiments, the COB of the present disclosure are molecular entities (or assemblies, complexes, or conjugates) that may comprise (i) a common linker sequence that is capable of hybridizing to a common linker oligonucleotide associated with a UBA-ESB complex, (ii) a unique code that is associated with a specific cell of origin, and (iii) one or more primer sequences, or combinations thereof.

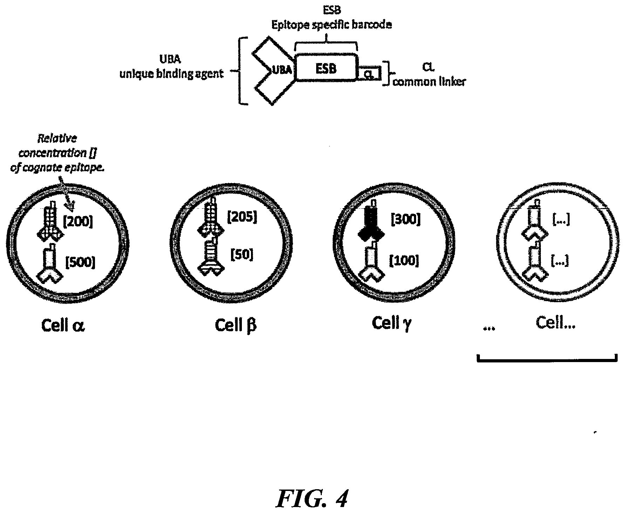

In some embodiments, COB are modular structures comprised of two or more APS. In some embodiments, COB are modular structures comprised of two or more APS attached in linear combination. In some embodiments, the COB comprise a plurality of APS attached in linear combination, wherein the APS comprise small molecules of deterministic weight. In some embodiments, the COB comprise 2, 3, 4, 5, 6, 7, 8, 9, 10, or more unique APS attached in a linear combination. In some embodiments, COBs comprise linear combinations of several APS, for example linear combinations of four APS, which are assembled using a split-pool combinatorial synthesis approach (FIGS. 4-6), as described more fully in the published patent applications referenced previously. Linear attachment of adjacent APS may be accomplished using a variety of techniques, for example, by chemically coupling adjacent APS, or by hybridizing individual APS to a template (splint) nucleic acid molecule that includes two or more sets of sequence regions designed to anneal with complementary sequence regions on individual APS, followed by ligation of the adjacent APS. The template nucleic acid molecule, or splint, may comprise at least one nucleic acid sequence, such as at least part of a linear viral genome or viral genome that can be made linear, e.g. the genomes of adenovirus, hepatitis virus, herpes virus, rotavirus, and the like; bacteriophages such as lambda, M13, ϕX-174, T-series bacteriophages, and the like, including derivatives thereof comprising cloning cassettes, poly-linkers, and the like; plasmids, such as pBR322 and pUC series plasmids, etc., including derivatives thereof comprising cloning cassettes, poly-linkers, and the like; synthetic templates; templates comprising artificial sequences; and the like. Those skilled in the art will understand that virtually any piece of nucleic acid can serve as a template for fabricating a COB provided that it is large enough to include annealing regions for at least two APS, or it can be combined with at least one other nucleic acid sequence so that the combined sequence is large enough to include annealing regions for at least two APS.

In some embodiments, the set of APS recognition sequences of the template or splint molecule are each separated by a linker comprising 1, 2, 3, or more carbon atoms, which acts as a "stop" signal or stop code for polymerase activity thereby preventing unwanted amplification of the full template molecule during nucleic acid amplification steps.

In some embodiments, the plurality of APS may comprise a set of uniquely designed nucleic acid sequences comprising one or more sub-code (SC) regions, wherein the sub-code sequence is unique for each individual APS molecule in the plurality of APS. In some embodiments, the SC regions or sequences are about 3, 4, 5, 6, 7, 8, 9, 10, or more than 10 nucleotides in length. In some embodiments, the sub-codes comprise a unique set of nucleic acid sequences of defined length, e.g. 7 nucleotides (FIG. 2), which are designed to provide error correction capability. In some embodiments, the set of sub-codes comprise 7 nucleotide sequences designed such that any pairwise combination of sequences in the set exhibits a defined "genetic distance", or number of mismatched bases, e.g. a distance of 3. In this case, review of the sub-codes in the set of COB identified in the final step of an assay allows one to detect hybridization or amplification errors prior to performing the final analysis of the assay data.

In some embodiments, the APS further comprise one or more common linker (CL) regions or sequences (e.g. common linker oligonucleotides) for the purpose of facilitating attachment of the APS to each other, or to the ESB, or to a template molecule used for assembly of the COB. Thus, in some embodiments, the common linker regions comprise annealing regions designed to hybridize to complementary sequences on a template molecule. The common linker can be directly or indirectly attached to the rest of the APS molecule, and facilitates either covalent or non-covalent assembly of the APS into a COB. In some embodiments, the common linker sequence may include oligonucleotide sequences or tandem-repeat sequences of about 10 to about 25 nucleotides in length. In some embodiments, the APS comprises two common linker sequences that flank the SC region. In some embodiments, common linker sequences can also be attached at either the 5' end or the 3' end of a COB, and may be utilized for capture and immobilization of a COB on a surface for detection or imaging purposes, e.g. by attaching a sequence that is complementary to the common linker sequence to a solid support or substrate.

In some embodiments, the APS or CL further comprises a random tag region allowing for subsequent quantitation of the number of detected COB. Methods for making and using such random tag regions are known in the art, e.g. see Casbon et al. (2011), Nucleic Acids Research 39(12):e81. The random tag region may function as a molecular counter to estimate the number of template molecules associated with each sequence variant. In some embodiments, a molecular counter is incorporated into an ESB, APS, CL, or an assembled COB prior to performing an amplification reaction, e.g. PCR amplification. A library of molecular counters comprising degenerate base regions (DBR) may be incorporated into the ESB, APS, CL, or assembled COB. The number of unique DBR in a library is generally limited by the length and base composition of the DBR. For example, a DBR comprising a single nucleotide would allow for four different possible counters, one for each base. Larger libraries of unique counter sequences can be achieved by using longer DBR, e.g. an eight nucleotide DBR corresponds to 4⁸ = 65,536 unique sequences. Molecular counters can be used to determine whether a sequence variant is associated with a single template molecule, or alternatively, with multiple template molecules. The number of different DBR sequences associated with one sequence variant can thus serve as a direct measure of the number of initial template molecules. This information can supplement or replace the information provided by read numbers of each sequence variant, including, for example, read numbers obtained after performing a PCR amplification reaction. DBRs can also be used to determine the probability that a sequence variant derives from a polymerase error during an amplification reaction or is a true original variant that was present prior to performing a PCR amplification reaction.

In some embodiments, the elements of a COB can be found in a single molecular entity (a singular COB), or two distinct molecular entities (a dual COB). Each molecular entity may be composed of one molecule, or more than one molecule attached to one another by covalent or non-covalent means. In some embodiments, each component of a dual COB has a target molecule-specific UBA-EBS complex that binds to a different site on the same target molecule. When using a dual COB system, one of the COB may be either labeled as described below or unlabeled. In some embodiments, the unlabeled COB may comprise a capture region.

In some embodiments, complementary oligonucleotide sequences designed to hybridize to an SC serve to attach detectable molecules, e.g. labels or label monomers, to each SC of the COB. The complementary oligonucleotide sequences may be directly labeled, for example, by covalent incorporation of one or more detectable label molecules into the complementary oligonucleotide sequence. Alternatively, the complementary oligonucleotide sequences may be indirectly labeled, for example, by incorporation of biotin or other molecule capable of providing a specific, high affinity ligand interaction, into the complementary oligonucleotide sequence. In such instances, the ligand (e.g. avidin or streptavidin in the case of biotin incorporation) may be covalently attached to the detectable molecule. In cases where the detectable molecules attached to an SC are not directly incorporated into the complementary oligonucleotide sequence, the complementary sequence serves as a bridge between the detectable molecule and the SC, and may be referred to as a bridging molecule, e.g., a bridging nucleic acid.

The COB of the present disclosure, and the APS molecules of which they are composed, can be labeled with any of a variety of labels or label monomers, e.g. radioisotopes, fluorophores, dyes, enzymes, nanoparticles, mass tags, chemiluminescent markers, biotin, or other labels or label monomers known in the art that can be detected directly (e.g. by light emission) or indirectly (e.g. by binding of a fluorescently-labeled antibody). In some embodiments, one or more of the SC in the COB is labeled with one or more label monomers, and the signals provided by the label monomers attached to the SC of a COB constitute a detectable code that identifies the target (or cell) to which the UBA (or the UBA-ESB-COB) binds. In some embodiments, the lack of a given signal from the SB (e.g. a dark spot) may also constitute part of the detectable code. Other examples of label monomers that can be used with the COB described herein, and methods to incorporate the label monomers into the COB are described in US patent 7,473,767.

### D. Primers

In some embodiments of the disclosed methods, compositions, and kits, target-specific primers, generic primers, semi-random primers, or combinations thereof, are used to selectively amplify UBA-ESB-COB complexes for targets of interest in order to optimize the cost efficiency and throughput of the sequencing reactions used for detection and quantitation of target molecules in individual cells.

An example of a target specific primer of the disclosed methods, compositions, and kits is illustrated schematically in FIG. 29, and comprises a sequencing primer region ("Primer 1") located near the 5' end of the molecule as well as the target-specific sequence region located near the 3' end of the molecule. In some embodiments, the sequencing primer region includes the Illumina sequencing primer sequence. Typically, the sequencing primer region will be between about 18 and 30 nucleotides in length. In some embodiments, the sequencing primer region will be between 20 and 25 nucleotides in length. The target-specific sequence region is designed to be complementary to the target sequence of interest. Typically, the target-specific sequence region will be between 6 and 30 nucleotides in length. In some embodiments, the target-specific sequence region will be between 18 and 22 nucleotides in length. In some embodiments the sequencing primer region and the target-specific sequence region will be separated by a linker region of between 0 and 30 nucleotides in length.

An example of a generic primer of the disclosed methods, compositions and kits is illustrated schematically in FIG. 30, and comprises a sequencing primer region ("Primer 1") located near the 5' end of the molecule as well as a poly-C sequence region located near the 3' end of the molecule. In some embodiments, the sequencing primer region includes the Illumina sequencing primer sequence. Typically, the sequencing primer region will be between about 18 and 30 nucleotides in length. In some embodiments, the sequencing primer region will be between 20 and 25 nucleotides in length. The poly-C sequence region is designed to be complementary to a poly-G sequence added to the 3' end of the target-UBA-ESB-COB complex following reverse transcription of the target RNA sequence. In some embodiments, the poly-C sequence region is between 4 and 30 nucleotides in length. In some embodiments, the poly-C sequence region is between 6 and 20 nucleotides in length. In some embodiments, the poly-C sequence region is between 6 and 12 nucleotides in length. In some embodiments the sequencing primer region and the poly-C sequence region will be separated by a linker region of between 0 and 30 nucleotides in length.

An example of a semi-random primer of the disclosed methods, compositions and kits is illustrated schematically in FIG. 31, and comprises a sequencing primer region ("Primer 1") located near the 5' end of the molecule as well as a semi-random sequence region (e.g. "NNNGAG") located near the 3' end of the molecule, where NNN is a random three nucleotide sequence. In some embodiments, the sequencing primer region includes the Illumina sequencing primer sequence. Typically, the sequencing primer region will be between about 18 and 30 nucleotides in length. In some embodiments, the sequencing primer region will be between 20 and 25 nucleotides in length. In some embodiment, the semi-random sequence is of the form of (M)ᵢ(X)ⱼ(N)ₖ, wherein (M)ᵢ and (N)ₖ are any random oligonucleotide nucleotide sequences of length i and k respectively, and wherein (X)ⱼ is a specific oligonucleotide sequence of length j chosen to complement the target oligonucleotide sequence at a specified position relative to the 3' end of a target oligonucleotide sequence. In some embodiment, the value of i and k may range between 0 and 6. In some embodiments, the value of j may range between 3 and 6. In some embodiments, the semi-random primer is designed to be complementary to a target oligonucleotide sequence at a specified position from the 3' end of the target sequence, thereby yielding an amplification product that is approximately Z nucleotides in length, and wherein the value of Z may range from 50 to 1000. In some embodiments, the semi-random sequence is designed to be partially complementary to the cDNA sequence at a position approximately 64 nucleotides from the junction between a cDNA copy of a target RNA sequence and a UBA probe sequence. In some embodiments the semi-random sequence is designed to be partially complementary to the cDNA sequence at a position between 128 and 32 nucleotides from the junction between the cDNA copy of the target RNA sequence and the original UBA probe sequence. In some embodiments, the non-random portion of the semi-random sequence is between 2 and 10 nucleotides in length. In some embodiments, the non-random portion of the semi-random sequence is between 3 and 6 nucleotides in length. In some embodiments, the random portion of the semi-random sequence is between 2 and 10 nucleotides in length. In some embodiments, the random portion of the semi-random sequence is between 3 and 6 nucleotides in length. In some embodiments the sequencing primer region and the semi-random sequence region will be separated by a linker region of between 0 and 30 nucleotides in length.

### IV. Methods

### A. Incubation of Cells with UBA-ESB Complexes

In many embodiments of the disclosed methods, cell suspensions or samples are incubated with one or more UBA-ESB complexes under conditions suitable for binding or hybridization with specific molecular targets on the surfaces of or within the individual cells. In some embodiments, one or more of the targets of interest may be intracellular targets, and the cells may be fixed and permeabilized using any of the methods known in the art, e.g. by adding cold methanol to the cell sample and incubating for a short period of time, followed be aspiration of the methanol, rinsing, and blocking with a bovine serum albumin or casein solution prior to incubation with the UBA-ESB.

### B. Assembly of Cell Origination Barcodes (COB)

Methods for barcoding single cells and assembling the associated cell origination barcodes have been described previously in published patent applications WO2012/106385 and WO2014/026032. COB assembly or synthesis can be performed by any suitable method known in the art, including the ones described briefly herein. In some embodiments, the COB may be assembled by stepwise addition of assayable polymer subunits (APS) comprising oligonucleotides. In some embodiments, a COB is attached to the UBA-ESB complex via a common linker (CL) that may itself be an oligonucleotide, and which may be part of the APS itself or a separate molecular component. In some embodiments, the ESB, APS, and CL may all comprise oligonucleotide sequences. Accordingly, following assembly by means of hybridization between complementary, or substantially complementary, annealing regions on the ESB, APS and CL, the assembled oligonucleotides may be ligated to form covalent bonds between ESB-APS, adjacent APS, or adjacent APS-CL units. Annealing regions may be provided on either or both ends of an oligonucleotide ESB, APS, or CL.

In some embodiments, the APS are added to the bound UBA-ESB by performing one or more rounds of split pool synthesis, wherein each round comprises splitting the cell sample into a plurality of aliquots, incubating each aliquot with a different APS (comprising a different SC) to allow annealing of complementary sequences between the APS and the growing UBA-ESB-APS chain, ligation (in the case of oligonucleotide ESB and APS), rinsing, and pooling of the aliquots. If the APS do not include incorporated CL regions, the cycle may also include an incubation step wherein a CL is allowed to anneal to the growing UBA-ESB-APS chain. In some embodiments, an annealing region that is specific to each step of the stepwise synthesis maybe incorporated into the oligonucleotide components of the reaction. In this case, the use of a step-specific annealing region may stall further assembly of the COB for any cell wherein the previous addition step failed.

The diversity of the COB library (i.e. the number of unique COB that are theoretically possible) that can be achieved by means of performing stepwise split-pool assembly and synthesis is dependent on the number of unique APS available for use in each round, and the total number of rounds used to assemble the COB. For example, for a COB created using two rounds of assembly/synthesis (i.e. for a COB having two APS positions) and 10 unique APS, the total number of unique COB sequences that are possible is 2¹⁰ = 1,024. Alternatively, for a COB created using four rounds of assembly/synthesis (i.e. for a COB having four APS positions) and 10 unique APS, the total number of unique COB sequences that are possible is 4¹⁰ = 1,048,576. In general, it is desirable to design the COB library such that the total number of unique barcodes available is significantly larger than the number of individual cells to be labeled, thereby ensuring that the probability that any two cells are labeled with the same cell origination barcode is extremely low.

In some embodiments, the APS are stitched together and/or to a CL using an annealing primer (i.e. a template molecule or "splint"). The annealing primer may comprise a first complementary region to the CL or an APS added during the previous round of stepwise synthesis. The annealing primer may also comprise a second complementary region to the APS being added during a current round. Thus, the annealing primer can hybridize to two oligonucleotide subunits of successive rounds, thereby stitching them together. In some embodiments, the first complementary regions of annealing primers of each round are different from the first complementary regions of annealing primers of other rounds. In some embodiments, the second complementary regions of annealing primers of each round are different from the second complementary regions of annealing primers of other rounds. In some embodiments, the first or second complementary regions of annealing primers of different rounds are shared between rounds. In some embodiments, a template or "splint" (i.e. an extended CL molecule) is used for assembly of APS, wherein the splint includes multiple sets of annealing regions designed to permit the stepwise hybridization and ligation of individual APS to create the completed COB.

In some embodiments, a CL or "splint" oligonucleotide comprises one or more pairs of loop annealing regions. Accordingly, the APS can be designed to hybridize to the CL or splint to create loop geometries, i.e. by hybridizing to the loop annealing regions at each end of a CL. In some embodiments, the loop annealing regions may be designed to be specific to the round of split-pool synthesis such that successive rounds of addition and hybridization populate the APS positions along the splint. The APS can then be linked together using any of the methods known in the art, for example, by ligation. In some embodiments, the APS may be designed to ensure that they do not hybridize efficiently to the splint at the loop annealing regions specific to other synthesis rounds. Consequently, if an APS from a particular round is missing for some reason, APS that are added in subsequent rounds are less likely to be ligated properly, thus reducing the likelihood of downstream analysis errors. Alternatively, COB may occasionally be synthesized even with a missing APS, the location of which is flanked by a pair of loop annealing regions. The resulting COB can then be analyzed accordingly, and can either be discarded or the retrieved information can be alternatively processed.

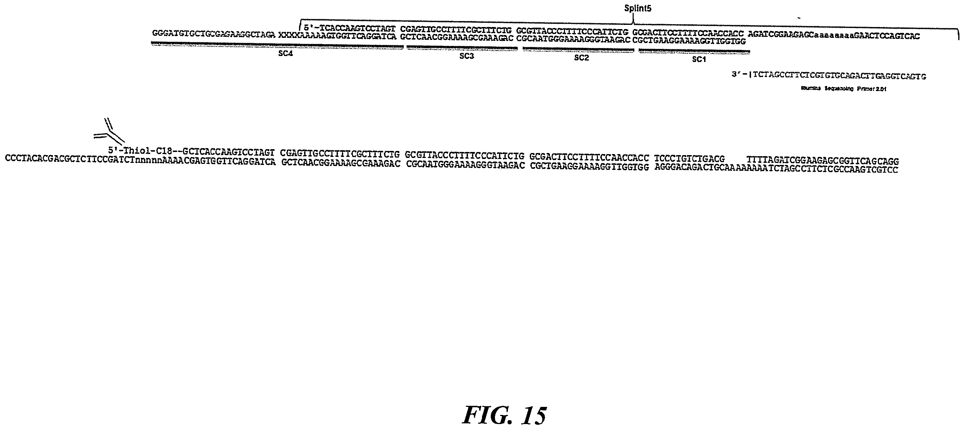

FIG. 15 illustrates one example of a splint oligonucleotide molecule used for assembly of APS into a unique cell origination barcode comprising coding regions SC1 - SC4. The lower figure indicates one example of an oligonucleotide for barcoding an antibody or antibody fragment, but which is equally applicable to use with UBA comprising oligonucleotide probes.

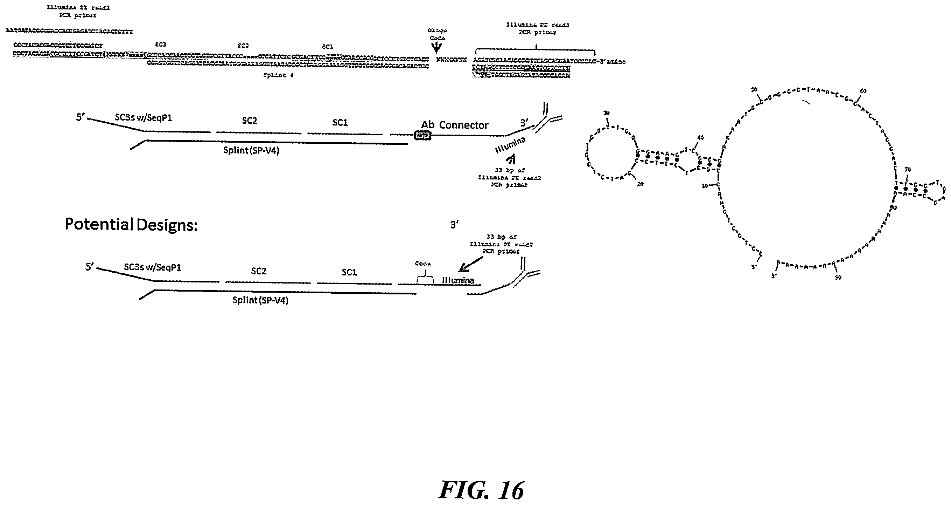

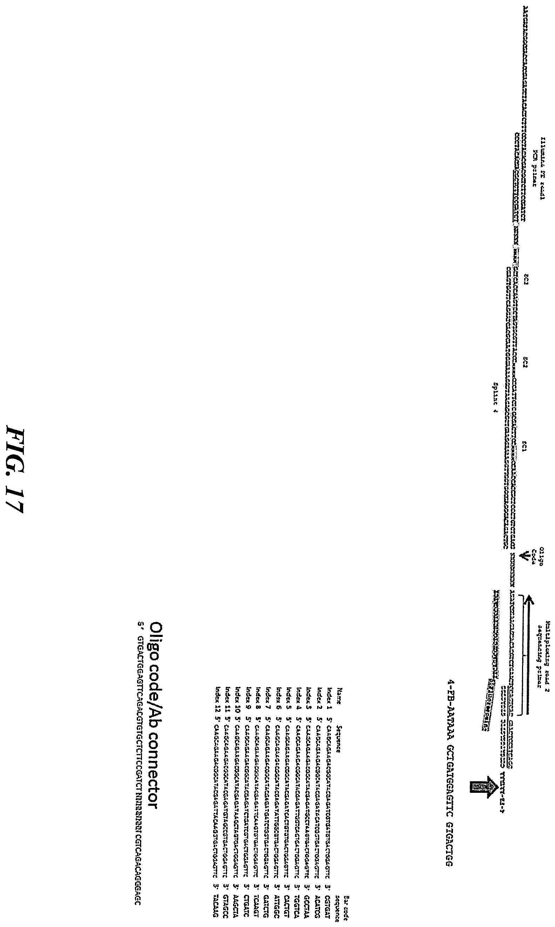

FIGS. 16 - 19 illustrate an example of a splint oligonucleotide molecule used for assembly of APS into a unique cell origination barcode comprising coding regions SC1 - SC3, and which further comprise amplification and/or sequencing primers. In some embodiments, the sequencing primers may comprise Illumina sequencing primers. An example of an oligonucleotide linker sequence for attaching oligonucleotides to antibodies, and which further comprises a 9 nucleotide epitope specific barcode region, is also illustrated (FIG. 17). In some embodiments, all or a portion of a cell origination barcode sequence may be detected by hybridization to an oligonucleotide detection probe comprising a fluorophore (FIG. 17, upper figure).

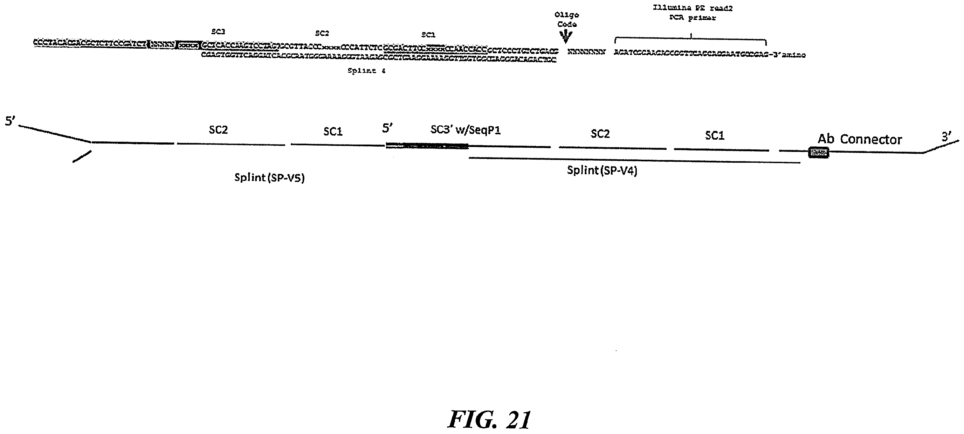

FIG. 21 illustrates an example of extending the length (i.e. the number of sub-code regions) of a COB by hybridizing a second splint molecule (Splint SP-V5) to the 5' end of the growing COB assembled using a first splint molecule (Splint SP-V4). In this example, a modified APS comprising both an SC region and a sequencing primer (SeqP1) was used to create the third coding region (SC3'), thereby providing the 5' sequence to which the second splint (Splint SP-V5) is hybridized. The use of a larger number of sub-code regions enables creation of a much larger number of unique cell origination barcodes for use in tagging mRNA or protein target molecules from individual cells. In some embodiments, a single splint oligonucleotide of greater length is used to assemble a larger number of APS to create the COB.

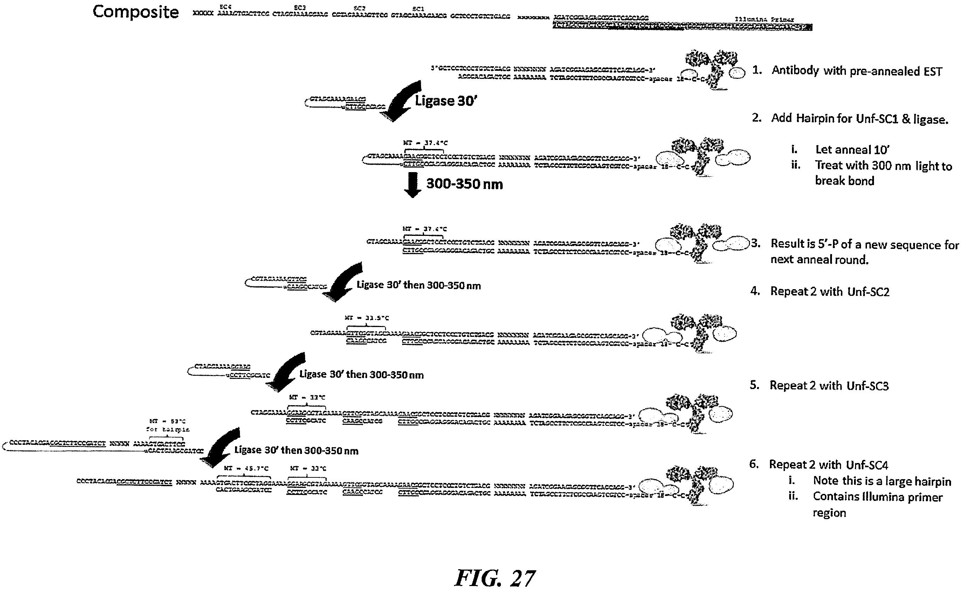

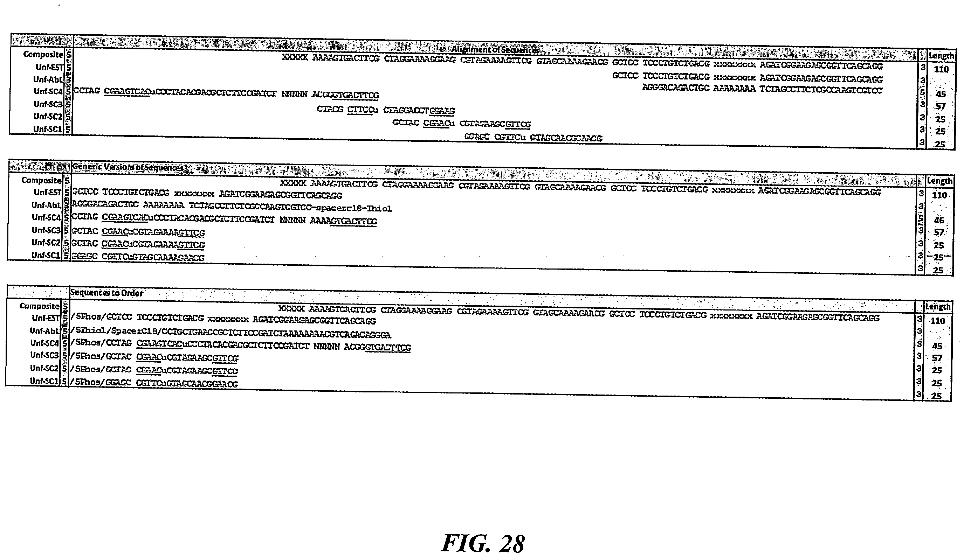

FIG. 27 illustrates a non-limiting example of a process for barcoding each occurrence of a bound antibody-EST (epitope specific tag, or epitope specific barcode) complex with a unique cell origination barcode using APS that comprise a hairpin oligonucleotide structure containing a photo-cleavable bond. The APS comprising the hairpin structure containing the first coding region, SC1, is annealed and ligated to the EST attached to bound antibodies. Following annealing and ligation, the sample is exposed to UV (300 nm) light to break the photocleavable bond, thereby creating a free 5'-phosphate terminal sequence that is available for hybridization with the next APS hairpin. Repeated rounds of annealing, ligation, and exposure to UV light are used to create a set of unique COB using the split-pool synthesis approach described above. In the non-limiting example illustrated in FIG. 27, the final APS hairpin structure includes an Illumina primer sequence. A non-limiting example of the set of oligonucleotide sequences used to create the hairpin structures of the method illustrated in FIG. 27 are shown in FIG. 28.

### C. Methods for Detection of Barcodes

Methods for amplification and detection of epitope specific barcodes and cell origination barcodes have been described more fully in published patent applications WO2012/106385 and WO2014/026032. In some embodiments, the assembled UBA-ESB-COB or ESB-COB products are amplified and, optionally, the results are compared with amplification of similar target nucleic acids from a reference sample. Nucleic acid amplification can be performed by any means known in the art. In some cases, the ligated products are amplified by polymerase chain reaction (PCR). Examples of PCR techniques that can be used include, but are not limited to, quantitative PCR, quantitative fluorescent PCR (QF-PCR), multiplex fluorescent PCR (MF-PCR), real time PCR (RT-PCR), single cell PCR, restriction fragment length polymorphism PCR (PCR-RFLP), real-time restriction fragment length polymorphism PCR (RT-PCR-RFLP), hot start PCR, nested PCR, in situ polonony PCR, in situ rolling circle amplification (RCA), bridge PCR, picotiter PCR and emulsion PCR. Other suitable amplification methods include the ligase chain reaction (LCR), transcription amplification, self-sustained sequence replication, selective amplification of target polynucleotide sequences, consensus sequence primed polymerase chain reaction (CP-PCR), arbitrarily primed polymerase chain reaction (AP-PCR), degenerate oligonucleotide-primed PCR (DOP-PCR) and nucleic acid based sequence amplification (NABSA). Other amplification methods that can be used herein include those described in U.S. Patent Nos. 5,242,794; 5,494,810; 4,988,617; and 6,582,938. In some embodiments, the amplification is performed inside a cell.

In some embodiments of the disclosed methods, compositions, and kits, target-specific or semi-random primers are used to selectively amplify UBA-ESB-COB complexes for targets of interest in order to optimize throughput and minimize costs for performing the sequencing reactions used for detection and quantitation of target molecules in individual cells.

In some embodiments, a target-specific primer, as illustrated schematically in FIG. 29, is used to selectively amplify those cell origination barcodes associated with the target or set of targets of interest, thereby reducing the amount of sequencing capacity expended on sequencing barcoded material associated with, for example, housekeeping gene transcripts and other common transcripts.

In some embodiments, a generic primer, as illustrated schematically in FIG. 30, is used to pre-amplify all barcoded material, followed by selective amplification using one or more target-specific primers to amplify those cell origination barcodes associated with the target or set of targets of interest, thereby reducing the amount of sequencing capacity expended on sequencing barcoded material associated with, for example, housekeeping gene transcripts and other common transcripts.

In some embodiments, a semi-random primer, as illustrated schematically in FIG. 31, is used to selectively amplify those cell origination barcodes associated with the target or set of targets of interest, thereby reducing the amount of sequencing capacity expended on sequencing barcoded material associated with, for example, housekeeping gene transcripts and other common transcripts. The semi-random sequence is designed to be partially complementary (via the non-random portion of the sequence) to the cDNA sequence at a position approximately 64 nucleotides from the junction between the cDNA copy of the target RNA sequence and the original UBA probe sequence, thereby ensuring that the amplified product is of approximately the same length as the read length of commercial high-throughput sequencing systems to ensure optimal use of sequencing capacity and throughput. In some embodiments the semi-random sequence is designed to be partially complementary to the cDNA sequence at a position between 128 and 32 nucleotides from the junction between the cDNA copy of the target RNA sequence and the original UBA probe sequence.

In any of the embodiments, the detection or quantitative analysis of the UBA-ESB-COB, ESB-COB, or COB library can be accomplished by sequencing. The APS subunits or entire COB can be detected via full sequencing of all oligonucleotide tags by any suitable methods or systems known in the art, e.g. by using the Illumina HiSeq 2000 sequencing system. Sequencing can be accomplished through classic Sanger sequencing methods which are well known in the art. Sequencing can also be accomplished using high-throughput and/or next-generation sequencing systems, some of which allow detection of a sequenced nucleotide immediately after or upon its incorporation into a growing strand, i.e., detection of sequence in red time or substantially real time. In some cases, high throughput sequencing generates at least 1,000, at least 5,000, at least 10,000, at least 20,000, at least 30,000, at least 40,000, at least 50,000, at least 100,000 or at least 500,000 sequence reads per hour; with each read being at least 50, at least 60, at least 70, at least 80, at least 90, at least 100, at least 120, at least 150, at least 200, or at least 250 bases per read.

### D. Multiplexed Testing

In certain embodiments, the methods of detection are performed in multiplex assays, wherein a plurality of target molecules is detected in the same assay (i.e. in a single reaction mixture). In some embodiments, the assay is a hybridization assay or an affinity binding assay in which the plurality of target molecules is detected simultaneously. In some embodiments, the assay is a hybridization assay or an affinity binding assay in which the plurality of target molecules is detected simultaneously in single cells. In certain embodiments, the plurality of target molecules detected in the same assay is, at least 2 different target molecules, at least 5 different target molecules, at least 10 different target molecules, at least 20 different target molecules, at least 50 different target molecules, at least 75 different target molecules, at least 100 different target molecules, at least 200 different target molecules, at least 500 different target molecules, at least 750 different target molecules, or at least 1,000 different target molecules. In other embodiments, the plurality of target molecules detected in the same assay is up to 5 different target molecules, up to 10 different target molecules, up to 20 different target molecules, up to 50 different target molecules, up to 100 different target molecules, up to 150 different target molecules, up to 200 different target molecules, up to 500 different target molecules, up to 750 different target molecules, up to 1,000 different target molecules, up to 2,000 target molecules, or up to 5,000 target molecules. In yet other embodiments, the plurality of target molecules detected is any range in between the foregoing numbers of different target molecules, such as, but not limited to, from 20 to 50 different target molecules, from 50 to 200 different target molecules, from 100 to 1000 different target molecules, from 500 to 5000 different target molecules, and so on and so forth.

### E. Quantitative Detection

In addition to the qualitative analytical capabilities provided by the UBA-ESB-COB complexes of the present disclosure and analytical techniques based thereon, in some embodiments the UBA-ESB-COB can be uniquely suitable for conducting quantitative analyses. By providing a one-to-one binding stoichiometry between the UBA-ESB-COB and their associated target molecules, e.g. in embodiments in which the UBA-ESB complex comprises a short random sequence (FIG. 10) to uniquely tag each instance of a target molecule, all or a representative portion of the target molecules present in the sample can be identified and counted. This individual counting of the various molecular species provides an accurate and direct method for determining the absolute or relative concentration of the target molecules in the biological sample. Moreover, the ability to address and count single molecules allows one to leverage the benefits of assay miniaturization, including high sensitivity, minimal sample quantity requirements, fast reaction rates which are afforded by solution phase kinetics in a small volume, and ultimately, very low reagent costs.

### F. Detection of mRNA Target Molecules

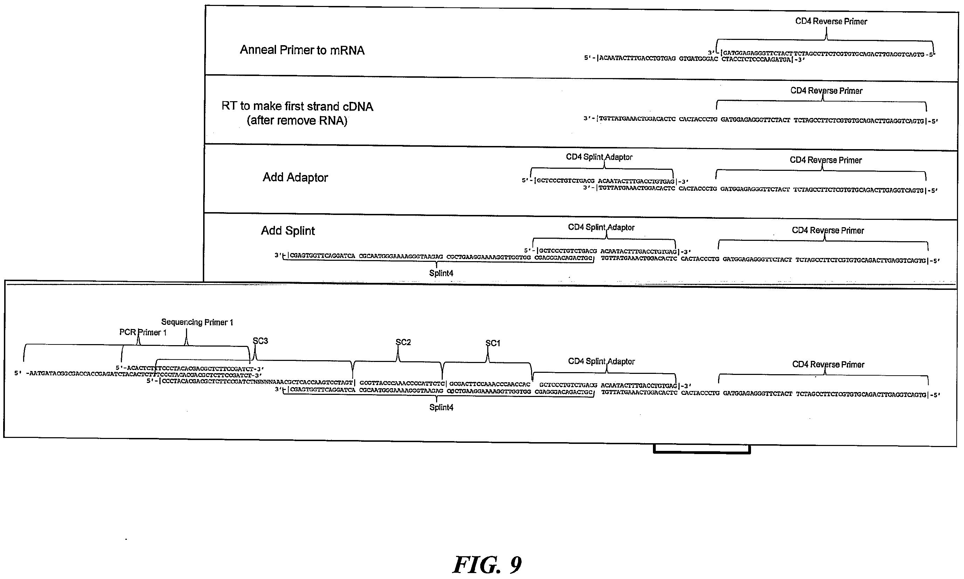

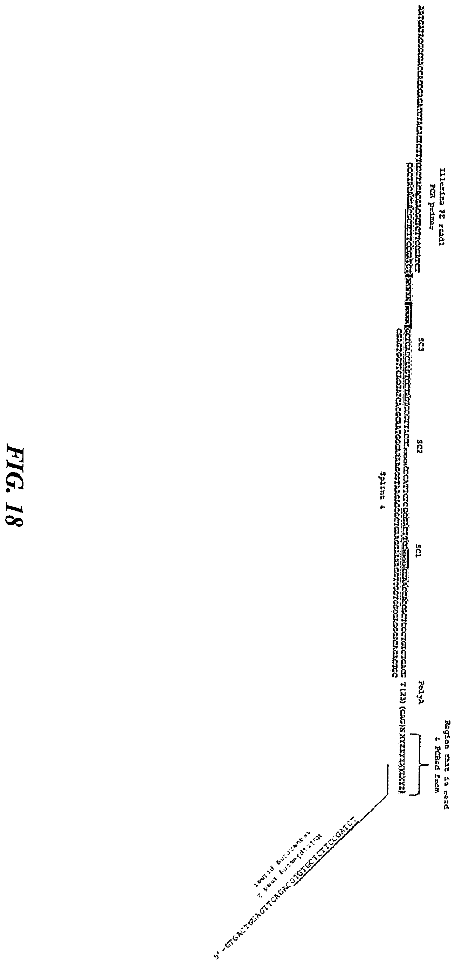

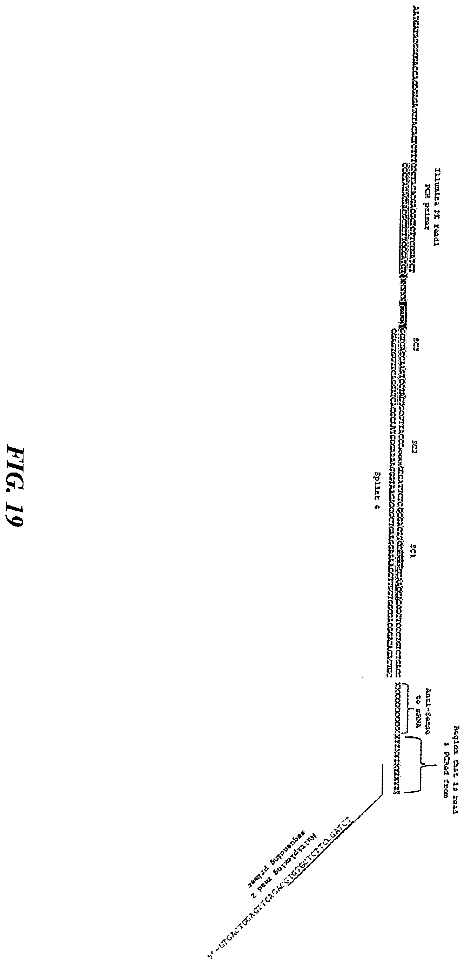

A non-limiting example of the process used to detect specific mRNA target molecules and label each occurrence with a unique cell origination barcode is illustrated in FIG. 9 for detection of CD4 mRNA. A CD4 reverse primer is added to a cell sample that has been fixed and permeabilized and allowed to anneal, following which a reverse transcription (RT) reaction is performed to create a cDNA copy of a portion of the CD4 mRNA molecules. After removal of the mRNA molecule (e.g. by treating with RNase H), a splint adaptor is annealed to the cDNA. The splint adaptor is used to anneal a splint molecule, which is then used to assemble two or more APS comprising SC regions (three APS comprising codes SC1 - SC3 are illustrated in FIG. 9) in a combinatorial fashion to create a unique COB. In some embodiments, the reverse primer used to hybridize with the target molecule includes a sequence recognition region that is specific for the target nucleic acid molecule (FIG. 18). In some embodiments, the sequence recognition region ranges from 10 to 20 nucleotides in length. In some embodiments, the sequence recognition region is a hexamer (FIG. 19). In some embodiments, the oligonucleotide probe is designed to hybridize non-specifically with mRNA molecules in general, e.g. by using a poly-T sequence recognition region (FIG. 17). In some embodiments, the splint molecule is also used for the addition of one or more amplification and/or sequencing primers. In some embodiments, the annealed molecular complex is subjected to ligation to create covalent molecular assemblies that can be amplified and sequenced.

FIG. 18 illustrates a non-limiting example of a method for barcoding mRNA molecules using a generic poly-T (or poly-dT) primer sequence. Following addition of the poly-T primer sequence to the cell sample, a reverse transcription reaction is performed, after which a "splint" oligonucleotide is annealed and used for assembly of APS comprising coding regions SC1 - SC3 into a unique cell origination barcode that may be amplified and sequenced using Illumina primers.

FIG. 19 illustrates a non-limiting example of a method for barcoding mRNA molecules using a target mRNA sequence-specific primer, e.g.
GCTCCCTGTCTGACG XXXXXXXXXXX
Following addition of the sequence-specific primer to the cell sample, a reverse transcription reaction is performed, after which a "splint" oligonucleotide is annealed and used for assembly of APS comprising coding regions SC1 - SC3 into a unique cell origination barcode that may be amplified and sequenced using Illumina primers. In some embodiments, one or more rounds of nested PCR amplification may be performed using an internal primer, prior to amplification and sequencing using the Illumina primers. In some embodiments, a hexamer primer, e.g.
GCTCCCTGTCTGACG NNNNNN
is used to hybridize with target mRNA molecules.

In some embodiments, target mRNA molecules are detected using a proximity probe set, the compositions for which are described above. The use of a pair of proximity oligonucleotide probes, each comprising a target recognition sequence that is complementary to non-overlapping but closely spaced sequence regions of the same target mRNA, provides for reduced non-specific probe hybridization and increased target detection specificity by creating a requirement that two sequence recognition events occur simultaneously and in close proximity to one another.

FIG. 7 illustrates one embodiment of a proximity probe set (i.e. a UBA) for detection and barcoding of target mRNA molecules (or RNA molecules in general), the probe set comprising a pair of oligonucleotide proximity probes, **15** and **19,** each of which includes a sequence region that is complementary to the target mRNA sequence, and which may be subsequently joined using a bridge oligonucleotide **(20).** The proximity probes may further comprise one or more primer sequences, epitope specific barcode regions, and/or common linker regions for use in creating unique cell origination barcodes using the compositions and methods of the present disclosure. The proximity probe set is added to a cell sample that has been fixed and permeabilized, the probes are allowed to anneal to target mRNA molecules, and then ligated to create a molecular complex that contains the epitope specific barcode (i.e. the target specific barcode) and primers that allow for amplification of the entire complex. Non-bound probe molecules may be rinsed away, and individual cells of the sample may be barcoded using the split-pool synthesis methods described above. Following the cell barcoding procedure, the molecular complexes comprising the UBA-ESB-COP are amplified using PCR amplification or any other suitable nucleic acid amplification technique and sequences to identify and quantify which mRNA molecules were present in the sample, on an individual cell basis.

FIG. 8 illustrates another embodiment of the use of a proximity probe set to barcode specific target mRNA molecules with a unique COB. In this embodiment, the positioning of primers and common linkers is arranged such that the COB is attached to the 3' end of the probe complex rather than the 5' end, as was illustrated in the example of FIG. 7.

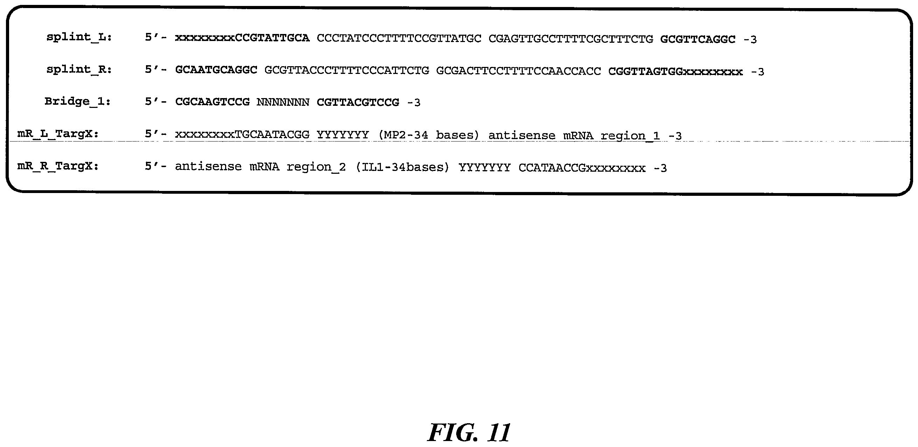

FIG. 10 illustrates another embodiment of a proximity probe set for detecting and barcoding target mRNA molecules that utilizes two splint molecules and a bridge oligonucleotide in addition to the two proximity probe sequences. Each splint molecule comprises a sequence region that is complementary to one of the proximity probes, and a sequence region that is complementary to part of the bridge oligonucleotide. In this example, the epitope specific barcode is replaced by an RNA specificity code (RSC) region, one on each of the two proximity probes, which comprises a 7 nucleotide code to identify the mRNA sequence recognized by the proximity probes. The proximity probes may further comprise primer sequences for use in amplification and sequencing. Each proximity probe may also comprise a short random sequence region for use in sequencing and amplification bias correction. An example of the oligonucleotide sequences used in creating the proximity probe set of FIG. 10 is illustrated in FIG. 11.

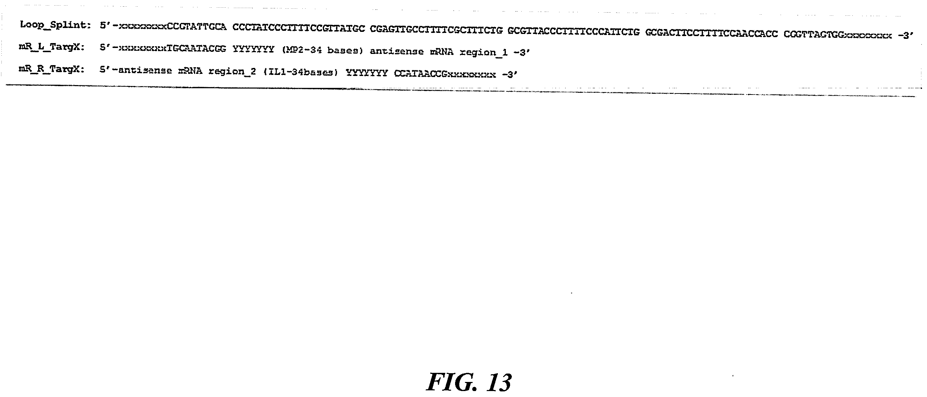

FIG. 12 illustrates another embodiment of a proximity probe set for detecting and barcoding target mRNA molecules, which utilizes a single, combined splint-bridge oligonucleotide to join the two proximity probes. A non-limiting example of the oligonucleotide sequences used in creating the proximity probe set of FIG. 12 is illustrated in FIG. 13.

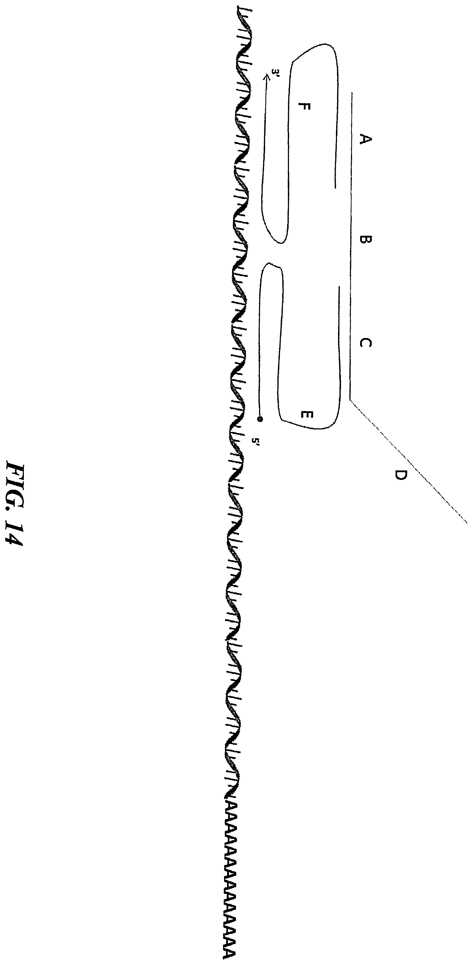

FIG. 14 illustrates another embodiment of a proximity probe set for detecting and barcoding target mRNA molecules, which utilizes a single, combined splint-bridge oligonucleotide to join the two proximity probes.

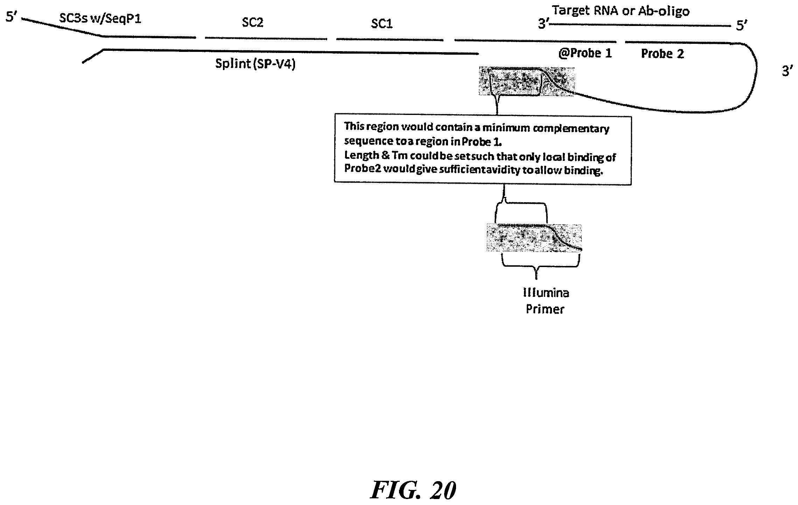

FIG. 20 illustrates another example of a proximity probe set and splint oligonucleotide molecule used for assembly of APS comprising coding regions SC1 - SC3 into a unique cell origination barcode for specific mRNA target molecules (or oligonucleotide-tagged antibodies). In this example, one of the proximity probe molecules (probe 2) is extended to further comprise an internal "bridge" oligonucleotide sequence capable of hybridization to a short sequence region of probe 1, thereby reducing the length of the target mRNA sequence region that is included in subsequent amplification and sequencing steps.

FIGS. 22 and 23 illustrate examples of proximity probe sets (including pairs of target specific probes which may further comprise bridge sequences, and one or more splint oligonucleotide molecules) used for assembly of APS to create unique cell origination barcodes for target mRNA molecules, where the number of complementary sequence recognition events and the proximity requirements thereof combine to provide for increased target detection specificity.

In some embodiments, the proximity probe sets disclosed herein may be used for detection of specific mRNA sequences in the absence of performing additional cell origination barcoding steps. For example, in some embodiments, a cell sample may be lysed to release mRNA, following which the sample is contacted with a plurality of beads, wherein a bead comprises a plurality of tethered oligonucleotide sequences capable of hybridizing to the released mRNA molecules, e.g. through the use of a poly-T sequence recognition region. Following hybridization of the released mRNA from the cell sample, a first oligonucleotide proximity probe is annealed with the hybridized mRNA molecules on the plurality of beads, wherein the first oligonucleotide proximity probe comprises an epitope specific barcode sequence and a first target recognition sequence that is capable of hybridizing to a first segment of the target nucleic acid sequence. Simultaneously, or subsequently, a second oligonucleotide proximity probe is annealed with the hybridized mRNA molecules on the beads, wherein the second oligonucleotide proximity probe comprises a second target recognition sequence that is capable of hybridizing to a second segment of the target nucleic acid sequence, and wherein the first and second segments of the target nucleic acid sequence are different and are separated from each other by a specified number of nucleotides, N. A bridge oligonucleotide is then, simultaneously or subsequently, annealed with the hybridized oligonucleotide proximity probes on the plurality of beads, wherein the bridge oligonucleotide comprises two probe recognition sequences, wherein the first probe recognition sequence is capable of hybridizing to a segment of the first oligonucleotide proximity probe, and the second probe recognition sequence is capable of hybridizing to a segment of the second oligonucleotide proximity probe, thereby creating a target specific probe complex that includes the epitope specific barcode. In some embodiments, the annealed components (i.e. the pair of oligonucleotide proximity probes and the bridge oligonucleotide) are ligated to create covalently joined target specific probe complexes. In many embodiments, the plurality of tethered oligonucleotide sequences further comprise one or more primer sequences, e.g. amplification primers or sequencing primers. In some embodiments, the target specific probe complexes are amplified using a PCR reaction and one or more target specific primers. In some embodiments, the PCR amplification products are sequenced to detect or quantify the presence of one or more mRNA sequences in the sample.

### F. Discrimination between Whole Cells and Dead Cells, Cell Fragments, or Cell Clusters

When performing assays to identify a plurality of target molecules in individual cells in a sample comprising a complex mixture of cells, it may be desirable to discriminate between whole cells and dead cclls, cell fragments, or clusters of cells so that data for the latter may be excluded from subsequent analysis, thereby improving the quality of the data. In studies involving samples comprising millions of cells, where each cell is individually barcoded, the presence of cell fragments, cell doublets, or larger clusters of cells can contribute "noise" in the form of erroneous data indicating the presence of cells that have markers that they shouldn't have. Accordingly, the methods, compositions, and kits of the present disclosure provide means for discriminating between the single cells of interest and dead cells, cell fragments, or clusters of cells present in samples.

In some embodiments, discrimination between the single cells of interest and dead cells, cell fragments, or clusters of cells present in samples is achieved by analyzing the ratio of DNA to protein for each "cell". In some embodiments, discrimination between the single cells of interest and dead cells, cell fragments, or clusters of cells is achieved by analyzing the amount of DNA detected per "cell". In yet other embodiments, discrimination is achieved by analyzing the amount of protein detected per "cell".

In some embodiments, the amount of DNA per "cell" may be determined by choosing to include one or more UBA that are directed towards genomic DNA or chromosomal DNA structures, for example, binding agents including, but not limited to, antibodies that bind DNA or histones, or DNA intercalating molecules (such as berberine, ethidium bromide, proflavine, daunomycin, dactinomycin, doxorubicin, daunorubicin, or thalidomide) in the set of UBAs chosen to identify a specific set of target molecules. Following completion of the assay, the amount of DNA per "cell" is determined from the number of DNA-specific UBA-ESB complexes detected for each cell, as identified by the cell origination barcode (COB). In some embodiments, it may be useful to compare the number of DNA-specific UBA-ESB complexes recovered to a calibration curve generated using the same set of DNA-directed UBAs and known concentrations of genomic or chromosomal DNA, under similar incubation conditions to correct for binding stoichiometry in cases where the binding stoichiometry between the DNA-specific UBA and genomic DNA or chromosomal DNA structures is not 1-to-1. In some embodiments, the same approach is used to discriminate between whole cells and "dead" cells by performing the incubation with one or more UBAs directed towards genomic DNA or chromosomal DNA structures prior to fixing and permeabilizing the cell sample.

In some embodiments, the amount of protein per "cell" may be determined by choosing to include one or more UBA that are directed non-specifically towards protein, for example, including but not limited to amine-reactive moieties such as succinimidyl esters, sulfosuccinimidyl esters, tctrafluorophcnyl esters, sulfodichlorophcnol esters, isothiocyanates, or sulfonyl chlorides, or one or more UBA that are directed specifically towards a common protein, e.g. antibodies directed towards actin or other housekeeping proteins, in the set of UBA chosen to identify a specific set of target molecules. Following completion of the assay, the amount of protein per "cell" is determined from the number of non-specific protein UBA-ESB complexes detected for each cell, as identified by the cell origination barcode (COB). In some embodiments, it may be useful to compare the number of non-specific protein UBA-ESB complexes recovered (or specific protein UBA-ESB complexes in the case that antibodies to actin or other housekeeping proteins are used) to a calibration curve generated using the same set of protein-directed UBA and known concentrations of protein, under similar incubation conditions to correct for binding stoichiometry in cases where the binding stoichiometry between the non-specific protein UBA and protein is not 1-to-1. Alternatively, in some embodiments, the average number of accessible amine groups on the surface of a given protein or set of proteins is calculated on the basis of protein structural data, and is subsequently used to determine the amount of protein per cell based on the number of non-specific protein UBA-ESB complexes recovered for each cell.

### G. Methods for Identification of Rare Cells

When performing assays to identify a plurality of target molecules in individual cells in a sample comprising a complex mixture of cells, it is often desirable to identify a specific sub-population of cells within the complex mixture and focus the subsequent analysis on that sub-population, thereby improving the specificity of the data. In studies involving samples comprising millions of cells, each individually barcoded, the presence of rare cells may constitute as little as 0.01% of the total cell population. Accordingly, the methods, compositions, and kits of the present disclosure provide for means for discriminating between the subset of cells of interest and the majority of cells present in the sample.

In some embodiments, a specific subset of cells may be identified by including one or more UBA that are directed towards specific intracellular or cell surface markers, for example, including, but not limited to, oligonucleotide probe sequences that are designed to hybridizes to viral genomic sequences, e.g. HIV viral sequences, or antibodies directed against CD1, CD3, CD8, or CD4, in the set of UBA chosen to identify a specific set of target molecules. Subsequent analysis is restricted to the selected sub-population of cells by selectively amplifying and sequencing those COB that are attached to the UBA-ESB complexes used to identify the sub-population of cells, thereby generating a list of all cells (as identified by their respective COB) which meet the selection criteria used to define the sub-population.

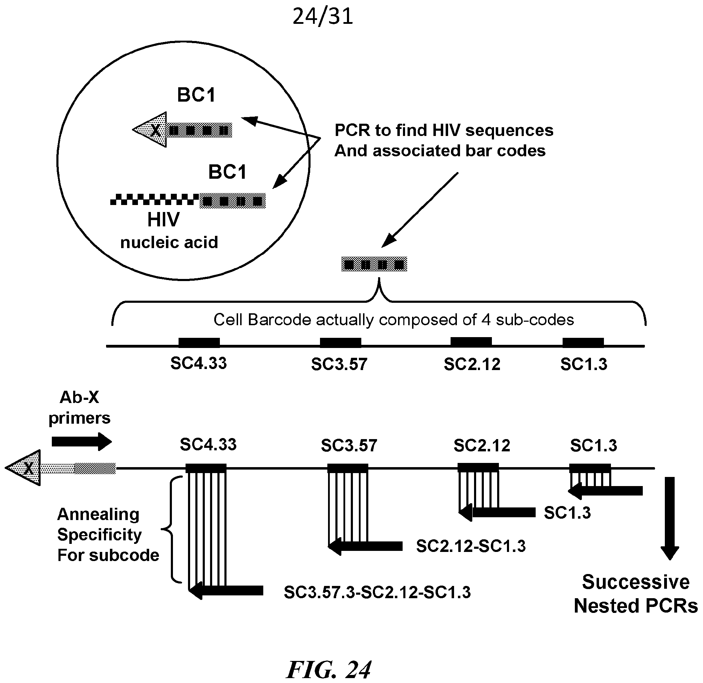

A complete listing of additional UBA-ESB associated with the selected sub-population of cells may be determined using the list of COB for the sub-population. In some embodiments, the list of COB is used to design a set of primers, for example 4 sets of primers in the case that 4 APS (each comprising an SC) are used to construct the COB (see FIG. 24), for performing a nested set of multiplexed PCR rounds. Starting from the APS position farthest from the UBA (or the end of the ESB-COB conjugate where the UBA would have been attached), the first set of primers is designed to hybridize to the annealing region(s) flanking the SC of the first (outermost) set of APS, i.e. flanking the set of SC₁ sequences at the APS₁ position, the second set of primers is complementary to the APS₂ - APS₁ set of sequences, the third set of primers is complementary to the APS₃ - APS₂ - APS₁ set of sequences, and the fourth set of primers is complementary to the APS₄ - APS₃ - APS₂ - APS₁ set of sequences. In each step, several rounds of PCR are used to selectively amplify a subset of the collection of COB, using a second primer that is complementary to a primer sequence, for example, the Illumina primer sequence, that flanks the far side of the ESB. Thus, performing several rounds of PCR amplification with each of the primer sets in succession will selectively amplify only the epitope specific barcode - cell origination barcode conjugates of interest, i.e. the epitope specific barcodes associated with the selected sub-population of cells. In some embodiments, the annealing step comprises performing a slow lowering of temperature from 98 °C to allow the "best" primer to find the correct complementary strand. In some embodiments, the polymerase and ligase used are chosen to maximize homoduplex formation. In some embodiment, the annealing step is followed by treatment with a nuclease, e.g. EcoR1, which cleaves heteroduplex DNA under the appropriate assay conditions prior to performing the next amplification cycle, thereby removing adventitious annealing events driven by the template. Sequencing of the resulting PCR products using any of the sequencing methods or systems known in the art, or subsequently performing sequence-specific quantitative PCR, allows one to identify which target molecules are present within individual cells of the selected sub-population of cells.

### H. Methods for Filtering Out Selected Cell Sub-Populations from Further Analysis

When performing assays to identify a plurality of target molecules in individual cells in a sample comprising a complex mixture of cclls, it is often desirable to filter out specific sub-populations of cells within the complex mixture, and focus the subsequent analysis on the remaining cells, thereby improving the specificity of the data. For example, in some applications it may be desirable to identify mature B cells in a population of cells (using antibodies directed towards cell surface markers such as CD19, CD38, BCMA, and the like) and eliminate them from further consideration, so that subsequent analysis may be focused on any stem cells that are present. Accordingly, the methods, compositions, and kits of the present disclosure also provide means for eliminating specified populations of cells from further analysis. In some embodiments this is accomplished by labeling multiple UBA (e.g. a set of antibodies) with the same ESB, so that following the binding step of the assay, selective amplification and sequencing of ESB-COB conjugates for the specified set of UBA provides a list of cells to be excluded from further analysis. Selective amplification and sequencing may be performed as described above.

### I. Resampling to Detect Additional Target Molecules in Selected Sub-Populations of Cells

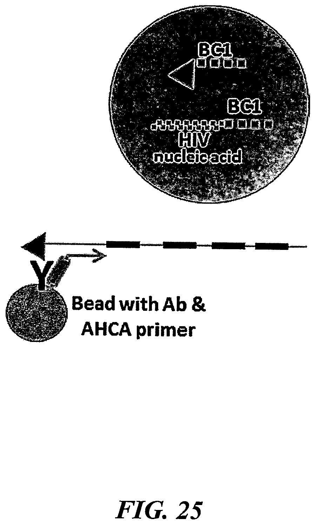

When performing assays to identify a plurality of target molecules in individual cells in a sample comprising a complex mixture of cells, it is often desirable to resample a barcoded cell suspension to determine if additional target molecules are also present in a selected sub-population. Accordingly, the methods, compositions, and kits of the present disclosure also provide means for resampling to detect one or more target molecules of interest at a point in time that is subsequent to that at which the initial cell barcoding procedure was performed. In some embodiments, detection of one or more target molecules of interest in individual cells of the barcoded cell suspension is enabled by including one or more UBA that are directed non-specifically towards protein, for example, including but not limited to amine-reactive moieties such as succinimidyl esters, sulfosuccinimidyl esters, tetrafluorophenyl esters, sulfodichlorophenol esters, isothiocyanates, or sulfonyl chlorides, in the original set of UBA used to perform the initial cell barcoding. Following the selective amplification and sequencing performed as described above to obtain a list of cell origination barcodes associated with cells of a selected sub-population, an aliquot of the barcoded cell suspension is lysed and incubated with beads comprising, for example, an immobilized antibody directed against one of the additional target molecules of interest and a tethered secondary primer (FIG. 25) that includes a code sequence for identification of the antibody immobilized on a given bead downstream from the primer sequence. A plurality of beads may be used, wherein the plurality of beads comprises, for example, immobilized antibodies directed against a plurality of target molecules along with their corresponding secondary primers, and wherein any single bead comprises a single type of immobilized antibody. Following immuno-precipitation of the target molecule(s) (e.g., a target protein), a secondary primer extension reaction is performed using an appropriate polymerase, e.g. Taq DNA Polymerase, Klenow DNA Polymerase I, and the like, followed by amplification to generate amplification product that includes the antibody code sequence and the cell origination barcode sequence. Comparison of the cell origination barcodes to the list of COB identified for the selected sub-population of interest then allows one to identify and quantify the presence of the additional target molecules of interest in the selected sub-population on an individual cell basis.

In some embodiments, a similar approach is utilized to detect mRNA molecules of interest in a selected sub-population of cells by using a non-specific UBA directed towards mRNA molecules in general, e.g. a UBA comprising a poly-T (or poly-dT) sequence, in the cell barcoding step, and a set of beads comprising immobilized oligonucleotide probes that are specific for the mRNA molecules of interest, along with immobilized secondary primers.

### V. Kits

The present disclosure also describes kits for barcoding molecules and cells, wherein the kits comprise one or more of the compositions described above. In some embodiments, the kits may comprise one or more target specific UBA-ESB complexes, or reagents for attaching pre-synthesized ESB to user-supplied UBA. In some embodiments, the UBA of the presently disclosed kits comprise one or more antibodies, which may further comprise attached ESB that encode the identity of the associated antibody. In some embodiments, the UBA of the presently disclosed kits comprise one or more oligonucleotide probes that are designed to hybridize to selected nucleic acid target, and which may further comprise attached ESB that encode for the identity of the associated target probe. In some embodiments, the disclosed kits may comprise, additionally or as a stand-alone product, sets of APS and any additional enzymes or reagents that may be required for their assembly into cell origination barcodes. In some embodiments, the sets of APS comprise sets of sub-code regions that are designed to provide error detection and correction capability at the sequencing step of the analysis. In some embodiments, the disclosed kits may comprise, additionally or as a stand-alone product, sets of primers for the selective amplification of epitope specific barcodes for a selected sub-population of cells.

### VI. Applications

The compositions, methods, and kits disclosed herein can be used for diagnostic, prognostic, thcrapcutic, patient stratification, drug development, treatment sclcction, and screening purposes. The disclosed compositions, methods, and kits provide the advantage that many different target molecules can be analyzed at one time, at the single cell level, from a single biological sample. This enables, for example, several diagnostic tests to be performed on one sample.

Examples of applications include, but are not limited, to biomarker discovery, target validation for drug discovery, gene expression profiling, protein expression profiling, proteome analyses, metabolomic studies, post-translation modification studies (e.g. for monitoring glycosylation, phosphorylation, acetylation, and other amino acid modifications), pharmacokinetic studies (e.g. drug metabolism, ADME profiling, and toxicity studies), analyses of specific serum or mucosal antibody levels; evaluation of non-nucleic acid diagnostic indicators, pathogen detection, foreign antigen detection, and the like.

### VII. Computer Software

Also disclosed herein are computer software packages stored on non-transitory computer readable media that provide analysis capabilities for decoding and grouping the sequencing data obtained for sets of epitope specific barcode - cell origination barcode conjugates. Examples of the capabilities provided by such software packages include sequence alignment and comparison tools, hierarchical clustering tools, amplification and/or sequencing error detection and correction tools, data visualization tools, and the like.

## Claims

1. A method for detecting one or more target molecules in a sub-population of cells, the method comprising:
a) contacting a sample comprising a complex mixture of cells with two unique binding agents, wherein the two unique binding agents are proximity oligonucleotide probes designed to hybridize to complementary segments of a target mRNA that are in close proximity to each other, and wherein at least one of the two oligonucleotide probes is attached to an oligonucleotide epitope specific barcode that represents the identities of the target molecules;
b) linking the proximity oligonucleotide probes together using bridge oligonucleotides;
c) sequentially attaching two or more oligonucleotide assayable polymer subunits to the epitope specific barcodes using a split-pool combinatorial synthesis approach to create unique cell origination barcodes that represent the identities of individual cells to which the unique proximity oligonucleotide probes have bound;
d) selectively amplifying and sequencing the epitope specific barcode and cell origination barcodes associated with the proximity oligonucleotide probes to identify a sub-population of cells expressing the target RNA.

2. The method of claim 1, wherein the unique binding agent comprises a nucleic acid sequence that is capable of hybridizing to a viral genome nucleic acid sequence.

3. The method of claim 2, wherein the viral genome is the HIV viral genome.

4. The method of claim 1, wherein the selective amplification of step (d) comprises performing two or more successive rounds of multiplexed, nested amplification reactions.

5. The method of claim 4, wherein each successive round of amplification utilizes a set of primers designed to hybridize to the assayable polymer subunits located at a different position in the sequence of two or more assayable polymer subunit positions that constitute the cell origination barcodes identified in step (d).

## Patentansprüche

1. Verfahren zum Erfassen eines oder mehrerer Zielmoleküle in einer Subpopulation von Zellen, wobei das Verfahren umfasst:
a) Inberührungbringen einer Probe, die eine komplexe Mischung von Zellen umfasst, mit zwei eindeutigen Bindemitteln, wobei die zwei eindeutigen Bindemittel Nähe-Oligonukleotid-Sonden sind, die dazu konzipiert sind, komplementäre Segmente einer Ziel-mRNA, die in unmittelbarer Nähe zueinander sind, zu hybridisieren, und wobei mindestens eine der zwei Oligonukleotid-Sonden an einem Oligonukleotid-Epitop-spezifischen Barcode, der die Identitäten der Zielmoleküle darstellt, angebracht ist;
b) Verbinden der Nähe-Oligonukleotid-Sonden miteinander unter Verwenden von Brücken-Oligonukleotiden;
c) sequenzielles Anbringen zweier oder mehrerer Oligonukleotid-bestimmbarer Polymer-Subeinheiten an den Epitop-spezifischen Barcodes unter Verwenden eines kombinatorischen Split-Pool-Syntheseansatzes, um eindeutige Zellherkunfts-Barcodes zu schaffen, die die Identitäten individueller Zellen darstellen, mit welchen sich die eindeutigen Nähe-Oligonukleotid-Sonden gebunden haben;
d) selektives Amplifizieren und Sequenzieren des Epitop-spezifischen Barcodes und der Zellherkunft-Barcodes, die mit den Nähe-Oligonukleotid-Sonden assoziiert sind, um eine Subpopulation von Zellen, die die Ziel-RNA exprimieren, zu identifizieren.

2. Verfahren nach Anspruch 1, wobei das eindeutige Bindemittel eine Nukleinsäuresequenz umfasst, die zum Hybridisieren zu einer Nukleinsäuresequenz eines viralen Genoms fähig ist.

3. Verfahren nach Anspruch 2, wobei das virale Genom ein HIV-Virus-Genom ist.

4. Verfahren nach Anspruch 1, wobei die selektive Amplifikation von Schritt (d) das Ausführen zweier oder mehrerer aufeinanderfolgender Durchgänge gemultiplexter, verschachtelter Amplifikationsreaktionen ist.

5. Verfahren nach Anspruch 4, wobei jeder aufeinanderfolgende Amplifikationsdurchgang einen Satz von Primern einsetzt, der konzipiert ist, um die bestimmbaren Polymersubeinheiten, die an einer unterschiedlichen Position in der Sequenz zweier oder mehrerer bestimmbarer Polymer-Subeinheitspositionen liegen, die die Zellherkunft-Barcodes bilden, die bei Schritt (d) identifiziert werden, zu hybridisieren.

## Revendications

1. Procédé destiné à détecter une ou plusieurs molécules cibles dans une sous-population de cellules, le procédé comprenant :
a) la mise en contact d'un échantillon comprenant un mélange complexe de cellules comportant deux agents de liaison uniques, dans lequel les deux agents de liaison uniques sont des sondes oligonucléotidiques de proximité conçues pour s'hybrider à des segments complémentaires d'un ARNm cible qui sont très proches l'un de l'autre et dans lequel au moins une des deux sondes oligonucléotidiques est fixée à un code à barres spécifique à l'épitope oligonucléotide qui représente les identités des molécules cibles ;
b) la liaison des sondes oligonucléotidiques de proximité entre-elles à l'aide des oligonucléotides de pontage ;
c) la fixation de manière séquentielle de deux sous-unités polymères dosables oligonucléotidiques ou plus aux codes à barres spécifiques à l'épitope à l'aide d'une approche de synthèse combinatoire de partage/mélange pour créer des codes à barres d'origine cellulaire uniques qui représentent les identités des cellules individuelles auxquelles les sondes oligonucléotidiques de proximité uniques se sont liées ;
d) l'amplification et le séquençage de manière sélective du code à barres spécifique à l'épitope et les codes à barres d'origine cellulaire associés aux sondes oligonucléotidiques de proximité pour identifier une sous-population de cellules exprimant l'ARN cible.

2. Procédé selon la revendication 1, dans lequel l'agent de liaison unique comprend une séquence d'acides nucléiques qui est capable de s'hybrider à une séquence d'acides nucléiques du génome viral.

3. Procédé selon la revendication 2, dans lequel le génome viral est le génome viral du VIH.

4. Procédé selon la revendication 1, dans lequel l'amplification sélective de l'étape (d) comprend la réalisation de deux ou plusieurs cycles successifs de réactions d'amplification multiplexées imbriquées.

5. Procédé selon la revendication 4, dans lequel chaque cycle successif d'amplification utilise un ensemble d'amorces conçues pour s'hybrider aux sous-unités polymères dosables situées à une position différente dans la séquence de deux positions ou plus de la sous-unité polymère dosable qui constituent les codes à barres d'origine cellulaire identifiés à l'étape (d).
